(19) Europäisches Patentamt
European Patent Office
Office européen des brevets

(11) **EP 4 748 842 A1**

(12) **EUROPEAN PATENT APPLICATION**
published in accordance with Art. 153(4) EPC

(43) Date of publication:
**27.05.2026 Bulletin 2026/22**

(21) Application number: **24842452.5**

(22) Date of filing: **22.07.2024**

(51) International Patent Classification (IPC):
***C07D 491/22*** *(2006.01)* ***C07D 498/22*** *(2006.01)*
***A61K 31/4375*** *(2006.01)* ***A61P 35/00*** *(2006.01)*

(52) Cooperative Patent Classification (CPC):
**A61K 31/4375; A61K 31/4745; A61K 47/54; A61K 47/64; A61K 47/68; A61P 35/00; A61P 35/02; C07D 231/12; C07D 491/22; C07D 498/22**

(86) International application number:
**PCT/CN2024/106693**

(87) International publication number:
**WO 2025/016467 (23.01.2025 Gazette 2025/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **20.07.2023 CN 202310902042**
**30.11.2023 CN 202311631907**

(71) Applicant: **Shanghai Pharmaceuticals Holding Co., Ltd.**
**Shanghai 201203 (CN)**

(72) Inventors:
- **ZHOU, Wei**
  **Shanghai 201203 (CN)**
- **XIA, Guangxin**
  **Shanghai 201203 (CN)**
- **WANG, Lei**
  **Shanghai 201203 (CN)**
- **ZHU, Yang**
  **Shanghai 201203 (CN)**
- **LV, Wei**
  **Shanghai 201203 (CN)**

(74) Representative: **Ipsilon Belgium**
**Bellevue 5/501**
**9050 Gent-Ledeberg (BE)**

(54) **CAMPTOTHECIN COMPOUND, ANTIBODY DRUG CONJUGATE, AND PREPARATION METHOD THEREFOR AND USE THEREOF**

(57) Provided are a camptothecin compound, an antibody drug conjugate, and a preparation method therefor and the use thereof. Specifically provided is a camptothecin compound as represented by formula (K) or a pharmaceutically acceptable salt thereof. The provided camptothecin compound and the antibody drug conjugate thereof have a good cytotoxic activity and can inhibit the proliferation of tumor cells.

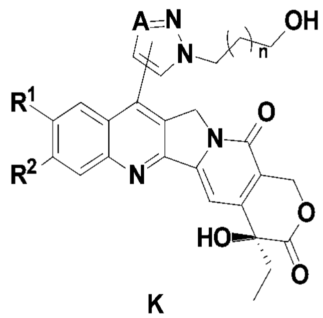

K

EP 4 748 842 A1

## Description

This application claims the priority of the Chinese patent application 2023109020423 with an application date of July 20, 2023 and the Chinese patent application 2023116319073 with an application date of November 30, 2023. The entire text of the aforementioned Chinese patent applications is incorporated by reference in this application.

### TECHNICAL FIELD

The present invention relates to the field of biomedicine, and specifically to a camptothecin compound, an antibody-drug conjugate, and a preparation method therefor and a use thereof.

### BACKGROUND

Camptothecin (Camptothecin, CPT) and its derivatives are one of the important traditional anti-tumor drug families, which have attracted widespread attention owing to their unique mechanism of action (selectively inhibiting DNA topoisomerase I). Three camptothecin derivatives have been approved for marketing for tumor treatment (Irinotecan, Topotecan, and Belotecan). In addition, many other camptothecin derivatives, such as Gimatecan, Karenitecin, Rubitecan, Silatecan, Exatecan, Lurtotecan, and Simmitecan, are in different phases of clinical trials.

Antibody-drug conjugates (antibody-drug conjugate, ADC) combine the high specificity of monoclonal antibody drugs with the high activity of compound cytotoxic drugs, so as to improve the targeting ability of anti-tumor drugs and reduce toxic side effects. The emergence of ADC drugs has filled the gap between antibody drugs and traditional chemotherapeutic drugs, improved drug specificity, and ameliorated the therapeutic window. Enhertu (approved in 2019) and Trodelvy (approved in 2020) are both ADC drugs that employ camptothecin derivatives as their effector molecule. However, there is still a need for further development of camptothecin derivatives and ADC drugs with improved therapeutic efficacy and/or safety.

### SUMMARY

The technical problem to be solved by the present invention is to overcome the defect of single structure of existing camptothecin compounds and antibody-drug conjugates, and to provide a camptothecin compound, an antibody-drug conjugate, and a preparation method therefor and a use thereof. The camptothecin compound and antibody-drug conjugate provided by the present invention exhibit excellent cytotoxic activity and are capable of inhibiting the proliferation of tumor cells.

The present invention resolves the technical problem by the following technical solutions.

The present invention provides a camptothecin compound of formula K or a pharmaceutically acceptable salt thereof:

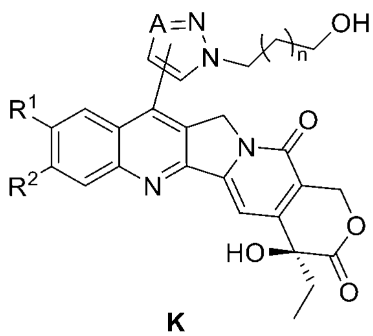

**K**

wherein $R^1$ is $C_{1-6}$ alkyl;
$R^2$ is halogen;
A is CH or N;
n is 0, 1, 2 or 3.

In one embodiment, the camptothecin compound of formula K or a pharmaceutically acceptable salt thereof satisfies one or more of the following conditions:

(1) in $R^1$, the $C_{1-6}$ alkyl is methyl, ethyl, n-propyl, isopropyl, n-butyl, isobutyl, sec-butyl or tert-butyl; for example,

methyl;

(2) in $R^2$, the halogen is fluorine, chlorine, bromine or iodine; for example, fluorine;

(3)

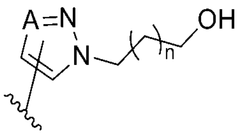

is

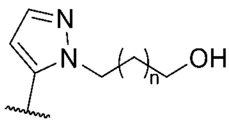, 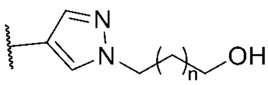, 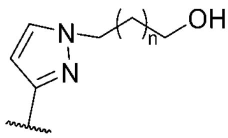

or

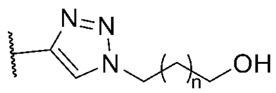.

In one embodiment, $R^1$ is methyl; and $R^2$ is fluorine, chlorine, bromine or iodine, for example, fluorine.

In one embodiment,

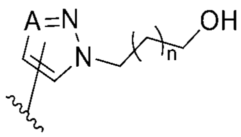

is

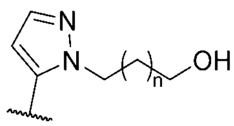, 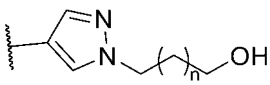,

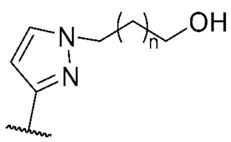 or 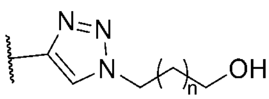,

preferably

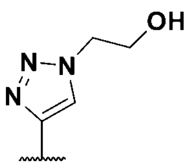, 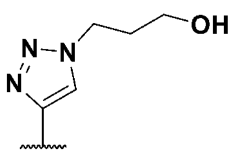,

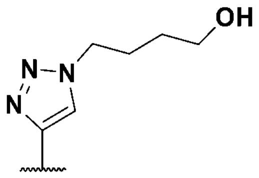, 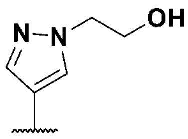, 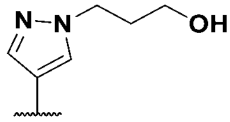, 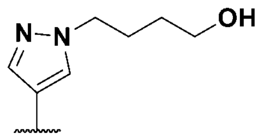, 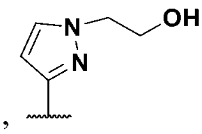,

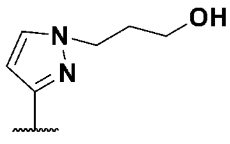 or 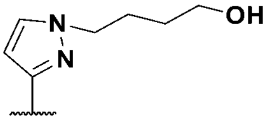.

In one embodiment, n is 0, 1 or 2.

In one embodiment, the camptothecin compound of formula K is the compound of formula K-1 or the compound of formula K-2;

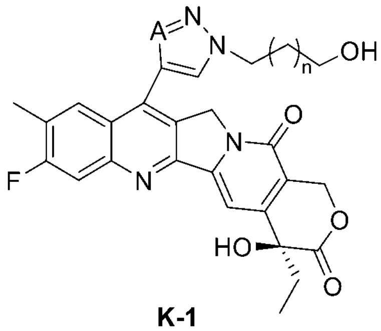

**K-1**

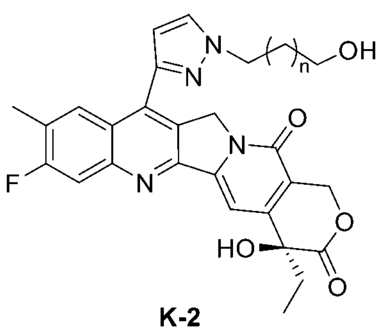

**K-2**

wherein, A and n are each independently as described in any one of the embodiments of the present invention.

In one embodiment, for a camptothecin compound of formula K or a pharmaceutically acceptable salt thereof, the camptothecin compound of formula K is selected from any one of the following structures:

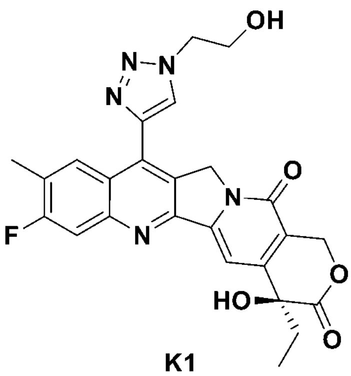

**K1**,

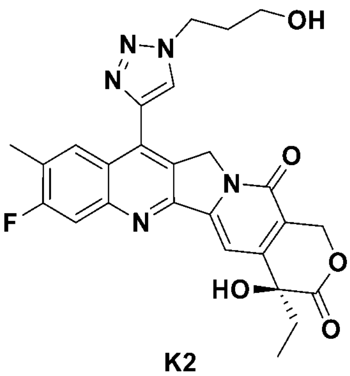

**K2**,

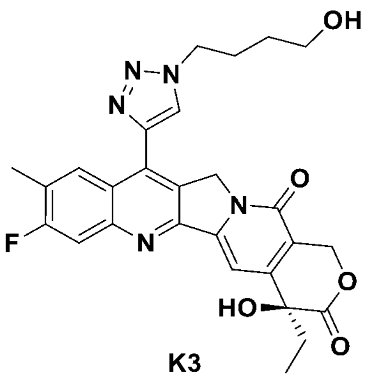

**K3**,

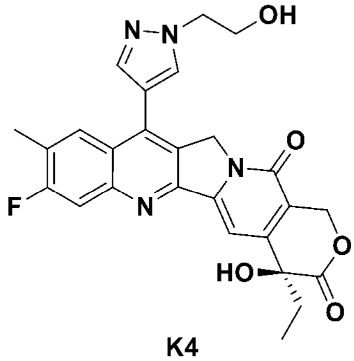

K4 ,

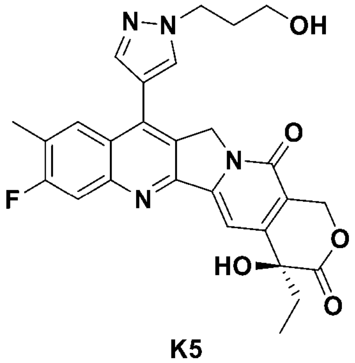

K5 ,

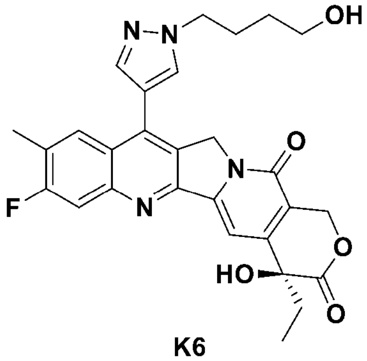

K6 ,

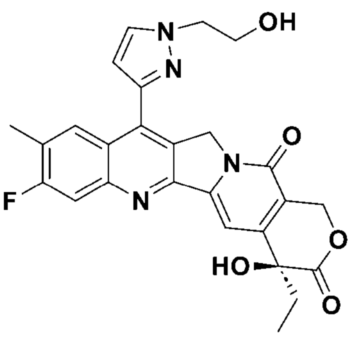

K7 ,

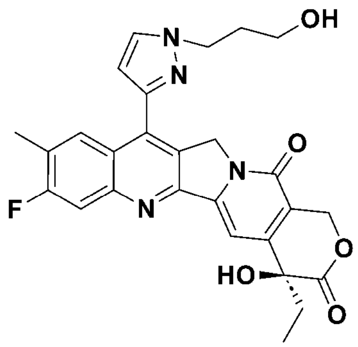

K8 ,

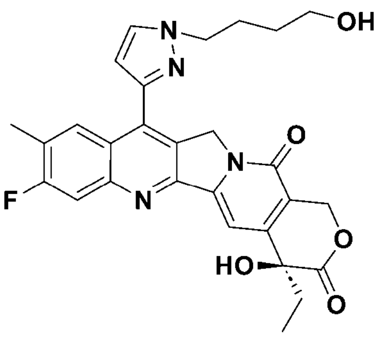

K9 .

The present invention further provides a linker-drug conjugate of formula I or a pharmaceutically acceptable salt thereof:

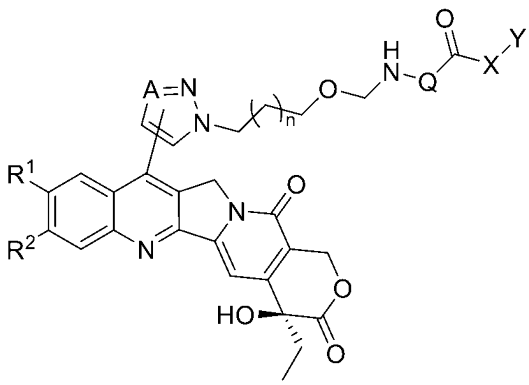

I

wherein, Q is a single amino acid residue, a dipeptide residue, a tripeptide residue, a tetrapeptide residue or a pentapeptide residue;

X is $-(CR^aR^b)_r-CO-NH-(CR^aR^b)_q-(OCH_2CH_2)_p-$, $-(OCH_2CH_2)_m-$, $-(CR^aR^b)_m-$, $-O(CR^aR^b)_m-$, $-NH-(CR^aR^b)_m-CR^cR^d-$ or $-S(CR^aR^b)_m-CR^cR^d-$;

$R^a$ and $R^b$ are each independently H, D, halogen, $C_{1-6}$ alkyl, halogenated $C_{1-6}$ alkyl, deuterated $C_{1-6}$ alkyl, $C_{1-6}$ alkoxy, hydroxyl, amino, cyano, nitro, 3-10-membered cycloalkyl or 3-10-membered heterocycloalkyl;

or, $R^a$ and $R^b$, together with the carbon atom to which they are attached, form a 3-10-membered cycloalkyl or a 3-10-membered heterocycloalkyl;

$R^c$ and $R^d$ are each independently H, $C_{1-6}$ alkyl, halogen, halogenated $C_{1-6}$ alkyl, deuterated $C_{1-6}$ alkyl, 3-10-membered cycloalkyl, 3-10-membered heterocycloalkyl, 6-14-membered aryl or 5-10-membered heteroaryl;

or, $R^c$ and $R^d$, together with the carbon atom to which they are attached, form a 3-10-membered cycloalkyl or a 3-10-membered heterocycloalkyl;

the heteroatoms in each of the 3-10-membered heterocycloalkyl and 5-10-membered heteroaryl are each independently selected from one, two or three of N, O or S, and the number of heteroatoms is each independently 1, 2 or 3;
Y is

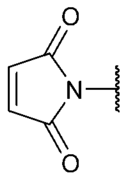 or 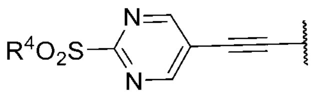 ;

$R^4$ is $C_{1-6}$ alkyl or a phenyl group substituted with one or more $C_{1-6}$ alkyl groups;
m, p, q and r are independently an integer from 0 to 14;
the definitions of

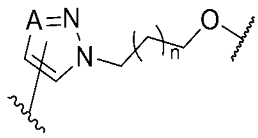 ,

$R^1$, $R^2$, A and n are as described in any one of the embodiments of the present invention.

In one embodiment, in X, $-(CR^aR^b)_r$-CO-NH-$(CR^aR^b)_q$-$(OCH_2CH_2)_p$-is-$(CR^aR^b)_r$-CO-NH-$(CR^aR^b)_q$-$(OCH_2CH_2)_p$-*, wherein "-*" terminal is linked to a carbonyl group (the carbonyl group in

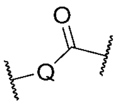

);

In one embodiment, in the linker-drug conjugate of formula I or a pharmaceutically acceptable salt thereof:

Q is a single amino acid residue, a dipeptide residue, a tripeptide residue, a tetrapeptide residue or a pentapeptide residue;
X is $-(CR^aR^b)_r$-CO-NH-$(CR^aR^b)_q$-$(OCH_2CH_2)_p$-*,*-$(OCH_2CH_2)_m$-,-$(CR^aR^b)_m$-,*-O$(CR^aR^b)_m$-,*-NH-$(CR^aR^b)_m$-$CR^cR^d$- or *-S$(CR^aR^b)_m$-$CR^cR^d$-, wherein "*" terminal (either "-*" or "*-") is linked to a carbonyl group (the carbonyl group in

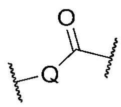

);
$R^a$ and $R^b$ are independently H, D, halogen, $C_{1-6}$ alkyl, halogenated $C_{1-6}$ alkyl, deuterated $C_{1-6}$ alkyl, $C_{1-6}$ alkoxy, hydroxy, amino, cyano, nitro, 3-10-membered cycloalkyl or 3-10-membered heterocycloalkyl;
or, $R^a$ and $R^b$, together with the carbon atom to which they are attached, form a 3-10-membered cycloalkyl or a 3-10-membered heterocycloalkyl;
$R^c$ and $R^d$ are independently H, $C_{1-6}$ alkyl, halogen, halogenated $C_{1-6}$ alkyl, deuterated $C_{1-6}$ alkyl, 3-10-membered cycloalkyl, 3-10-membered heterocycloalkyl, 6-14-membered aryl or 5-10-membered heteroaryl;
or, $R^c$ and $R^d$, together with the carbon atom to which they are attached, form a 3-10-membered cycloalkyl or a 3-10-membered heterocycloalkyl;
the heteroatoms in each of the 3-10-membered heterocycloalkyl and 5-10-membered heteroaryl are independently selected from one, two or three of N, O or S, and the number of the heteroatoms is independently 1, 2 or 3;
Y is

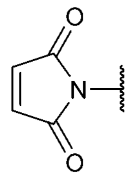 or 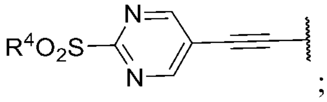 ;

$R^4$ is $C_{1-6}$ alkyl or a phenyl group substituted with one or more $C_{1-6}$ alkyl groups;
m, p1, q1, r1, p, q and r are independently an integer from 0 to 14;
the definitions of

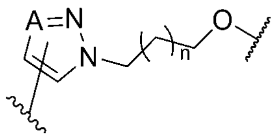 ,

$R^1$, $R^2$, A and n are as described in any one of the embodiments of the present invention.

In one embodiment, the linker-drug conjugate of formula I or a pharmaceutically acceptable salt thereof:

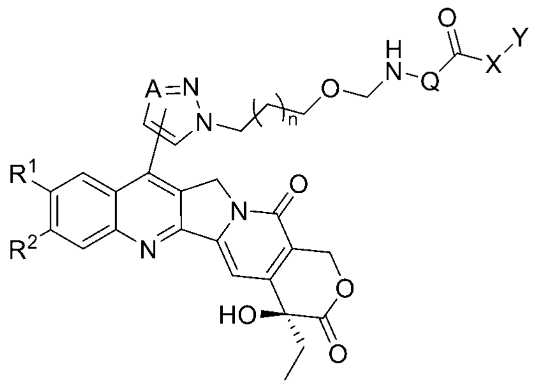

**I**

wherein, Q is a single amino acid residue, a dipeptide residue, a tripeptide residue or a tetrapeptide residue;
X is $-(CR^aR^b)_r-CO-NH-(CR^aR^b)_q-(OCH_2CH_2)_p-$, $-(OCH_2CH_2)_m-$, $-(CR^aR^b)_m-$, $-O(CR^aR^b)_m-$, $-NH-(CR^aR^b)_m-CR^cR^d-$ or $-S(CR^aR^b)_m-CR^cR^d-$;
$R^a$ and $R^b$ are independently H, D, halogen, $C_{1-6}$ alkyl, halogenated $C_{1-6}$ alkyl, deuterated $C_{1-6}$ alkyl, $C_{1-6}$ alkoxy, hydroxy, amino, cyano, nitro, 3-10-membered cycloalkyl or 3-10-membered heterocycloalkyl;
or, $R^a$ and $R^b$, together with the carbon atom to which they are attached, form a 3-10-membered cycloalkyl or a 3-10-membered heterocycloalkyl;
$R^c$ and $R^d$ are independently H, $C_{1-6}$ alkyl, halogen, halogenated $C_{1-6}$ alkyl, deuterated $C_{1-6}$ alkyl, 3-10-membered cycloalkyl, 3-10-membered heterocycloalkyl, 6-14-membered aryl or 5-10-membered heteroaryl;
or, $R^c$ and $R^d$, together with the carbon atom to which they are attached, form a 3-10-membered cycloalkyl or a 3-10-membered heterocycloalkyl;
the heteroatoms in each of the 3-10-membered heterocycloalkyl and 5-10-membered heteroaryl are independently selected from one, two or three of N, O or S, and the number of heteroatoms is independently 1, 2 or 3;
Y is

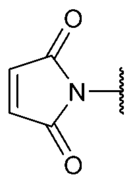 ;

m, p, q and r are independently an integer from 0 to 14;
the definitions of $R^1$, $R^2$, A and n are as described in any one of the embodiments of the present invention.

In one embodiment, the amino acid residues (the amino acid residues being the single amino acid residues or the amino acid residues constituting the dipeptide residues, tripeptide residues, tetrapeptide residues or pentapeptide residues) are independently -Phe-, -Lys-, -Val-, -Ala-, -Cit-, -Leu-, -Glu-, -Ile-, -Arg-, -Trp- or -Gly-.
In one embodiment, the dipeptide residues are -Lys-Phe-, -Ala-Val-, -Lys-Val-, -Lys- Ala-, -Cit-Val-, -Cit-Phe-, -Cit-Leu-, Cit-Ile-, -Arg-Phe-, -Cit-Trp- or -Val-Gly-, for example -Ala-Val-.
In one embodiment, the tripeptide residues are -Ala-Val-Glu-, -Cit-Val-Glu-, -Ala-Val-αGlu- or -Cit-Val-αGlu-.
In one embodiment, the tetrapeptide residues are -Gly-Phe-$(Gly)_2$- or -$(Gly)_2$-Phe-Gly-, for example -Gly-Phe-$(Gly)_2$-, preferably

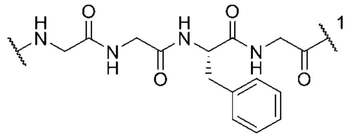

,

wherein 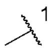 is linked to the -NH- group.
In one embodiment, in $R^a$, $R^b$, $R^c$ and $R^d$, the halogen is independently fluorine, chlorine, bromine or iodine.
In one embodiment, in $R^a$, $R^b$, $R^c$ and $R^d$, any of the $C_{1-6}$ alkyl groups in the $C_{1-6}$ alkyl, halogenated $C_{1-6}$ alkyl and deuterated $C_{1-6}$ alkyl is independently methyl, ethyl, n-propyl, isopropyl, n-butyl, isobutyl, sec-butyl or tert-butyl.
In one embodiment, in $R^a$, $R^b$, $R^c$ and $R^d$, the halogenated $C_{1-6}$ alkyl is independently a $C_{1-6}$ alkyl substituted with 1, 2 or 3 halogens, and the halogen is preferably independently fluorine, chlorine, bromine or iodine.
In one embodiment, in $R^a$, $R^b$, $R^c$ and $R^d$, the deuterated $C_{1-6}$ alkyl is independently a $C_{1-6}$ alkyl substituted with 1, 2 or 3 deuteriums (D).
In one embodiment, in $R^a$, $R^b$, $R^c$ and $R^d$, the 3-10-membered cycloalkyl is independently cyclopropyl, cyclobutyl, cyclopentyl or cyclohexyl.
In one embodiment, in $R^a$, $R^b$, $R^c$ and $R^d$, the 3-10-membered heterocycloalkyl is independently a 3-6-membered heterocycloalkyl, the heteroatoms of the 3-6-membered heterocycloalkyl are preferably independently selected from one, two or three of N, O or S, and the number of the heteroatoms is preferably independently 1, 2 or 3.
In one embodiment, when $R^a$ and $R^b$, together with the carbon atom to which they are attached, form a 3-10-membered cycloalkyl, the 3-10-membered cycloalkyl is independently cyclopropyl, cyclobutyl, cyclopentyl or cyclohexyl.
In one embodiment, when $R^a$ and $R^b$, together with the carbon atom to which they are attached, form a 3-10-membered heterocycloalkyl, the 3-10-membered heterocycloalkyl is independently a 3-6-membered heterocycloalkyl, the heteroatoms of the 3-6-membered heterocycloalkyl are preferably independently selected from one, two or three of N, O or S, and the number of the heteroatoms is preferably independently 1, 2 or 3.
In one embodiment, in $R^c$ and $R^d$, the 6-14-membered aryl is independently phenyl or naphthyl.
In one embodiment, in $R^c$ and $R^d$, the 5-10-membered heteroaryl is independently a 5-6-membered heteroaryl, the heteroatoms of the 5-6-membered heteroaryl are preferably independently selected from one, two or three of N, O or S, and the number of the heteroatoms is preferably independently 1, 2 or 3.
In one embodiment, in $R^4$, any of the $C_{1-6}$ alkyl groups in the $C_{1-6}$ alkyl and the phenyl substituted with one or more $C_{1-6}$ alkyl groups is independently methyl, ethyl, n-propyl, isopropyl, n-butyl, isobutyl, sec-butyl or tert-butyl, for example methyl.
In one embodiment, X is -$(CR^aR^b)_r$-CO-NH-$(CR^aR^b)_q$-$(OCH_2CH_2)_p$-*, *-$(OCH_2CH_2)_m$-, - $(CR^aR^b)_m$-, *-O$(CR^aR^b)_m$-, *-NH-$(CR^aR^b)_m$-$CR^cR^d$- or *-S$(CR^aR^b)_m$-$CR^cR^d$-, wherein "*" terminal (either "*-" or "-*") is linked to a carbonyl group (the carbonyl group in

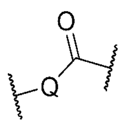

).
In one embodiment, Y is

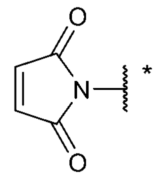 or 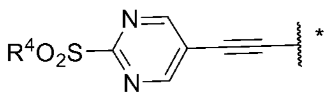 ,

wherein "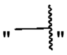" terminal is linked to X.

In one embodiment, the linker-drug conjugate of formula I or a pharmaceutically acceptable salt thereof satisfies one or more of the following conditions:

(1) each halogen is independently fluorine, chlorine, bromine or iodine;
(2) each $C_{1-6}$ alkyl is independently methyl, ethyl, n-propyl, isopropyl, n-butyl, isobutyl, sec-butyl or tert-butyl;
(3) each $C_{1-6}$ alkoxy is independently methoxy, ethoxy, n-propoxy, isopropoxy, n-butoxy, isobutoxy, sec-butoxy or tert-butoxy;
(4) each 3-10-membered cycloalkyl is independently cyclopropyl, cyclobutyl, cyclopentyl or cyclohexyl;
(5) each 3-10-membered heterocycloalkyl is independently a 3-6-membered heterocycloalkyl, and the heteroatoms of the 3-6-membered heterocycloalkyl are independently selected from one, two or three of N, O or S, with the number of heteroatoms independently being 1, 2 or 3;
(6) each 6-14-membered aryl is independently phenyl or naphthyl;
(7) each 5-10-membered heteroaryl is independently a 5-6-membered heteroaryl, and the heteroatoms of the 5-6-membered heteroaryl are independently selected from one, two or three of N, O or S, with the number of heteroatoms independently being 1, 2 or 3;
(8) in Q, the single amino acid residue is -Phe-, -Lys-, -Val-, -Ala-, -Cit-, -Leu-, -Ile-, -Arg-, -Trp- or -Gly-;
(9) in Q, the dipeptide residue is $^{C=O}$-Lys-Phe-$^{NH}$, $^{C=O}$-Ala-Val-$^{NH}$, $^{C=O}$-Lys-Val-$^{NH}$, $^{C=O}$- Lys- Ala-$^{NH}$, $^{C=O}$-Cit-Val-$^{NH}$, $^{C=O}$ Cit-Phe-$^{NH}$, $^{C=O}$ -Cit-Leu-$^{NH}$, $^{C=O}$ Cit-Ile-$^{NH}$, $^{C=O}$ -Arg-Phe-$^{NH}$, $^{C=O}$ -Cit-Trp-$^{NH}$ or $^{C=O}$ -Val-Gly-$^{NH}$; for example, $^{C=O}$ -Ala-Val-$^{NH}$;
(10) in Q, the tripeptide residue is $^{C=O}$ -Ala-Val-Glu-$^{NH}$, $^{C=O}$ -Cit-Val-Glu-$^{NH}$, $^{C=O}$ - Ala-Val-$\alpha$Glu-$^{NH}$ or $^{C=O}$ -Cit-Val-$\alpha$Glu-$^{NH}$; and
(11) in Q, the tetrapeptide residue is $^{C=O}$ -Gly-Phe-$(Gly)_2$-$^{NH}$ or $^{C=O}$ -$(Gly)_2$-Phe-Gly-$^{NH}$, for example, $^{C=O}$ -Gly-Phe-$(Gly)_2$-$^{NH}$.

In one embodiment, the linker-drug conjugate of formula I or a pharmaceutically acceptable salt thereof satisfies one or more of the following conditions:

(1) $R^a$ and $R^b$ are H;
(2) m is 1, 2, 3, 4 or 5;
(3) p is 8, 9, 10, 11, 12, 13 or 14;
(4) q is 1, 2, 3, 4 or 5;
(5) r is 1, 2, 3, 4 or 5;
(6) Q is a dipeptide residue or a tetrapeptide residue; for example, $^{C=O}$ -Ala-Val-$^{NH}$ or $^{C=O}$ - Gly-Phe-$(Gly)_2$-$^{NH}$; and
(7) X is $-(CR^aR^b)_r-CO-NH-(CR^aR^b)_q-(OCH_2CH_2)_p-$ or $-(CR^aR^b)_m-$; for example,

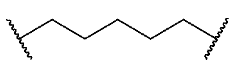 or 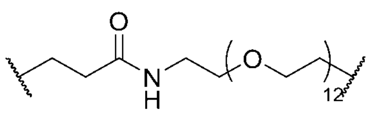 ,

further for example,

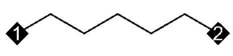

or

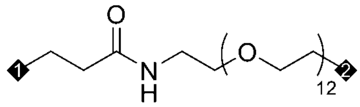,

wherein the position "1" is linked to Y and the position "2" is linked to

"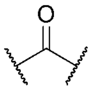".

In one embodiment, the linker-drug conjugate of formula I or a pharmaceutically acceptable salt thereof satisfies one or more of the following conditions:

(1) $R^a$ and $R^b$ are each independently H;
(2) m is 5;
(3) p is 8;
(4) q is 2;
(5) r is 2;
(6) n is 0 or 1;
(7) the single amino acid residue is -Phe-, -Lys-, -Val-, -Ala-, -Cit-, -Leu-, -Ile-, -Arg- or - Trp-;
(8) the dipeptide residue is $^{C=O}$-Lys-Phe-$^{NH}$, $^{C=O}$-Ala-Val-$^{NH}$, $^{C=O}$-Lys-Val-$^{NH}$, $^{C=O}$-Lys-Ala-$^{NH}$, $^{C=O}$-Cit-Val-$^{NH}$, $^{C=O}$-Cit-Phe-$^{NH}$ , $^{C=O}$-Cit-Leu-$^{NH}$, $^{C=O}$-Cit-Ile$^{NH}$, $^{C=O}$-Arg-Phe-$^{NH}$, $^{C=O}$-Cit-Ttp-$^{NH}$ or $^{C=O}$-Val-Gly-$^{NH}$;
(9) the tripeptide residue is $^{C=O}$-Ala-Val-Glu-$^{NH}$, $^{C=O}$-Cit-Val-Glu-$^{NH}$, $^{C=O}$-Ala-Val-$\alpha$Glu-$^{NH}$ or $^{C=O}$-Cit-Val-$\alpha$Glu-$^{NH}$;
(10) the tetrapeptide residue is $^{C=O}$-Gly-Phe-$(Gly)_2$-$^{NH}$ or $^{C=O}$-$(Gly)_2$-Phe-Gly-$^{NH}$.

In one embodiment, Q is a tetrapeptide residue, preferably -Gly-Phe-$(Gly)_2$- (e.g., $^{C=O}$ - Gly-Phe-$(Gly)_2$- $^{NH}$, wherein "$^{C=O}$-" refers to the terminal linked to the carbonyl group).

In one embodiment, X is $-(CR^aR^b)_r-CO-NH-(CR^aRb)_q-(OCH_2CH_2)_p-*$or$-(CR^aR^b)_m-$, preferably, X is $-(CR^aR^b)_m-$, wherein "-*" terminal is linked to the carbonyl group (the carbonyl group in

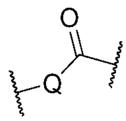

).

In one embodiment, $R^a$ and $R^b$ are H.

In one embodiment, m is an integer from 0 to 8, for example, 5, 6, 7 or 8, preferably 5.

In one embodiment, p is an integer from 0 to 10, for example, 5, 6, 7 or 8, preferably 8.

In one embodiment, q and r are independently an integer from 0 to 8, for example, 2 or 3.

In one embodiment, Y is

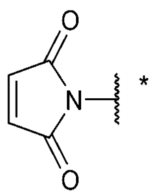,

wherein "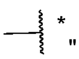" terminal is linked to X.

In one embodiment,

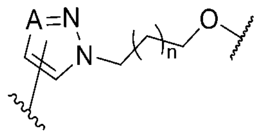

is

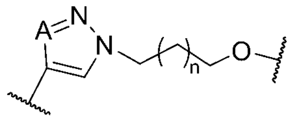

.

In one embodiment, the linker-drug conjugate of formula I or a pharmaceutically acceptable salt thereof:

wherein, Q is a tetrapeptide residue;
X is $-(CR^aR^b)_r$-CO-NH-$(CR^aR^b)_q$-$(OCH_2CH_2)_p$-*or $-(CR^aR^b)_m$-*, wherein "-*" terminal is linked to the carbonyl group;
$R^a$ and $R^b$ are H;
Y is

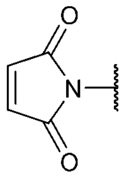

or

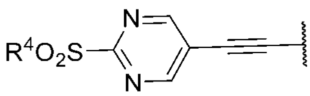

;

$R^4$ is $C_{1-6}$ alkyl;
m is 5;
P is 8;
q and r are each independently 2 or 3;
the definitions of

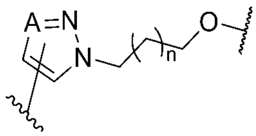

,

$R^1$, $R^2$, A and n are as described in any one of the embodiments of the present invention.

In one embodiment, the linker-drug conjugate of formula I or a pharmaceutically acceptable salt thereof:

wherein, Q is a tetrapeptide residue;
X is-$(CR^aR^b)_m$-;
$R^a$ and $R^b$ are each independently H;
Y is

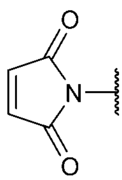

;

m is 5;

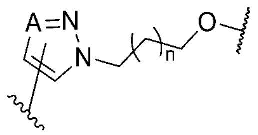

is

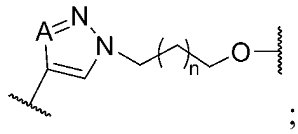

;

the definitions of $R^1$, $R^2$, A and n are as described in any one of the embodiments of the present invention.

In one embodiment, the linker-drug conjugate of formula I or a pharmaceutically acceptable salt thereof,

$R^1$ is $C_{1-6}$ alkyl;
$R^2$ is halogen;
A is CH or N;
n is 0, 1, 2 or 3;

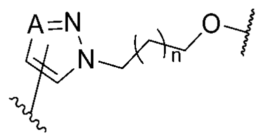

is

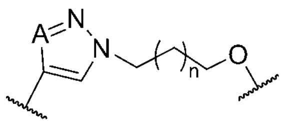

or

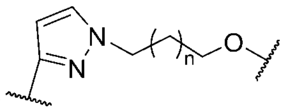

;

Q is a single amino acid residue, a dipeptide residue, a tripeptide residue or a tetrapeptide residue;
X is $-(CR^aR^b)_r-CO-NH-(CR^aR^b)_q-(OCH_2CH_2)_p-*$ or $-(CR^aR^b)_m-*$, wherein "-*" terminal group is linked to the carbonyl group;
$R^a$ and $R^b$ are H;
Y is

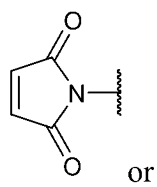

or

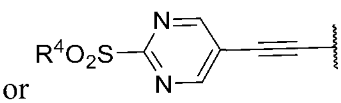

;

$R^4$ is $C_{1-6}$ alkyl;
m is an integer from 0 to 8;
p is an integer from 0 to 10;
q and r are each independently an integer from 0 to 8.

In one embodiment,

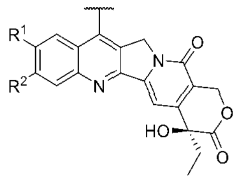

is

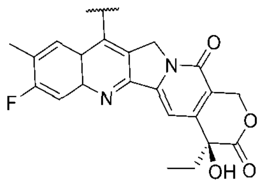.

In one embodiment,

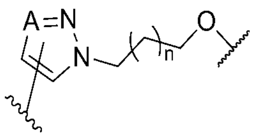

is

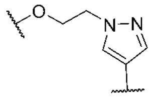 or 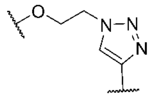.

In one embodiment,

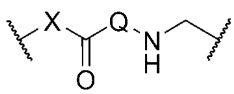

is

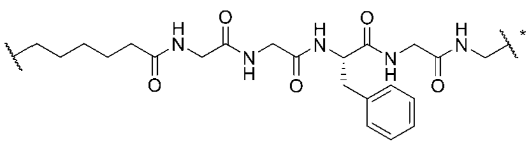,

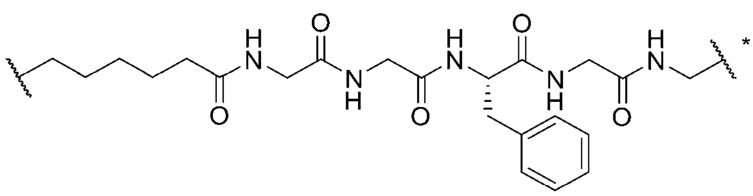,

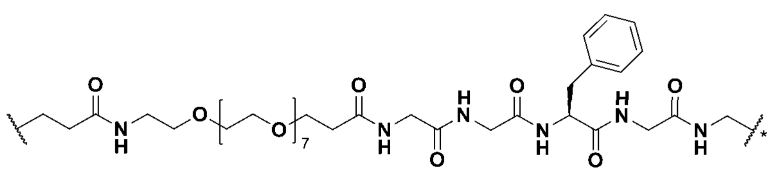

or

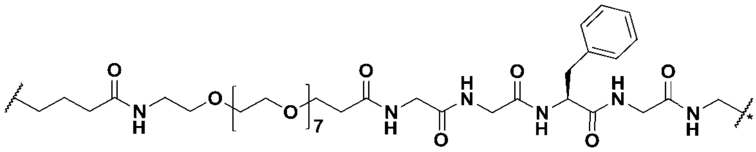

,

wherein "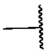" terminal is linked to -O-.

In one embodiment, the present invention provides the linker-drug conjugate of formula I or a pharmaceutically acceptable salt thereof, and the linker-drug conjugate of formula I is any one of the following structures:

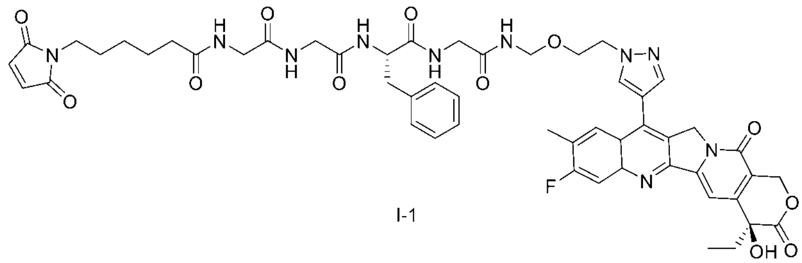

I-1

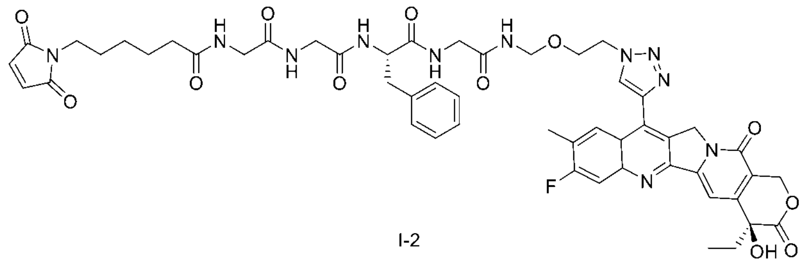

I-2

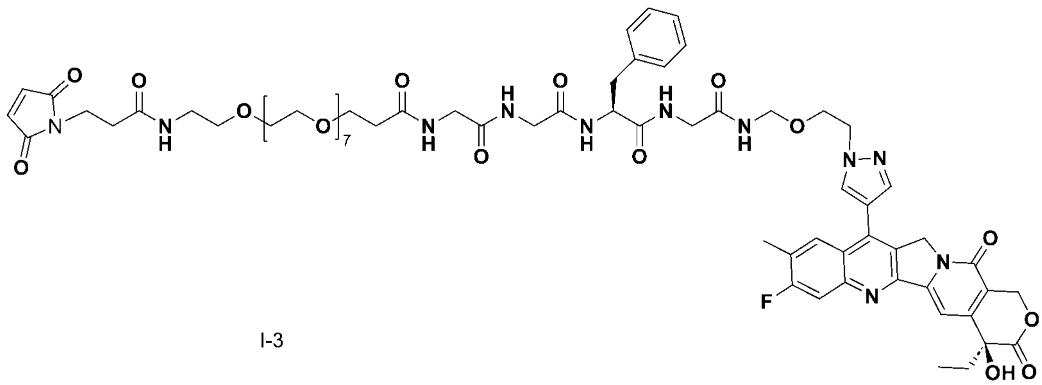

I-3

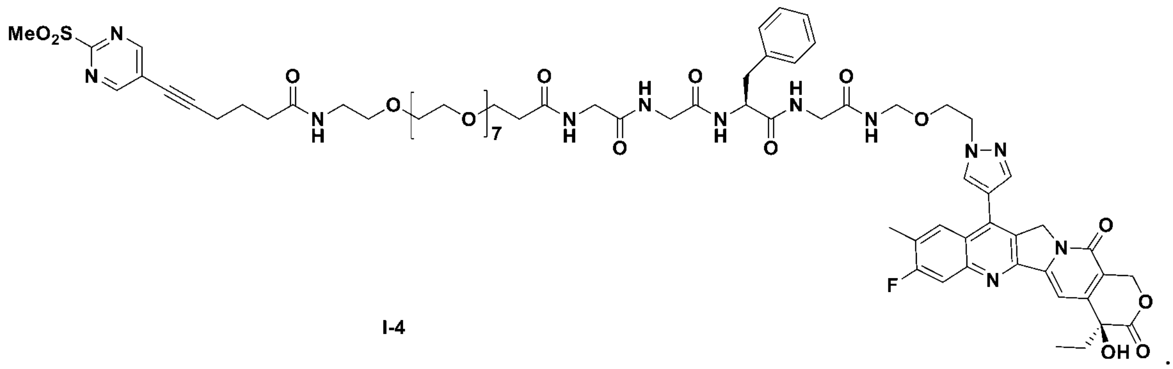

I-4

The present invention further provides an antibody-drug conjugate of formula II or a pharmaceutically acceptable salt thereof:

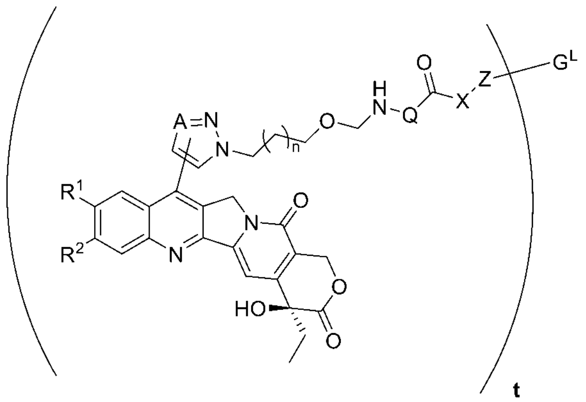

II

wherein, $G^L$ is an antibody;
t is a drug-to-antibody ratio;
Z is

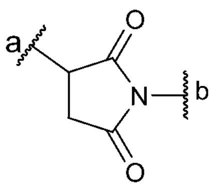
or
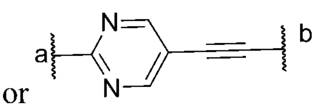
,

wherein "b" terminal is linked to X and "a" terminal is linked to $G^L$;
the definitions of

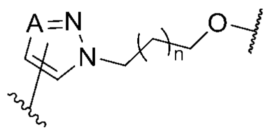
,

$R^1$, $R^2$, A and n are as described in any one of the embodiments of the present invention;
the definitions of Q and X are as described in any one of the embodiments of the present invention.

In one embodiment, $G^L$ is an anti-HER2 antibody, such as trastuzumab, and preferably, the $G^L$ is linked to Z via a thioether (-S-) bond.

In the antibody-drug conjugate of formula II or a pharmaceutically acceptable salt thereof, t is a conventional

conjugation ratio for such antibody-drug conjugates in the art; for example, t is 1-8 (natural number and/or decimal), such as 7, 7.7 or 7.8.

In one embodiment, the antibody-drug conjugate of formula II or a pharmaceutically acceptable salt thereof:

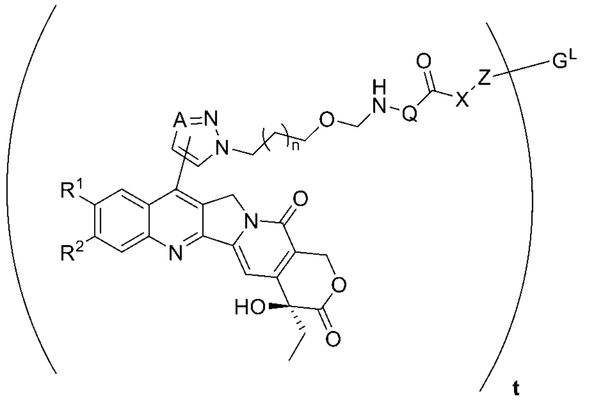

**II**

wherein, $G^L$ is an antibody;
t is a drug-to-antibody ratio;
Z is

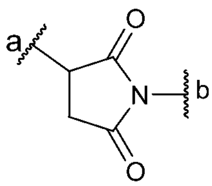

,

wherein "b" terminal is linked to X and "a" terminal is linked to $G^L$;
the definitions of $R^1$, $R^2$, A and n are as described in any one of the embodiments of the present invention;
the definitions of Q and X are as described in any one of the embodiments of the present invention.

In one embodiment, the antibody-drug conjugate of formula II or a pharmaceutically acceptable salt thereof satisfies one or more of the following conditions:

(1) $G^L$ is an anti-HER2 antibody, such as trastuzumab;
(2) t is a natural number and/or decimal from 1 to 8; for example, a natural number and/or decimal from 7 to 8; further for example, 7.8; or t is a natural number from 1 to 8; for example, a natural number from 7 to 8; and
(3) the "a" terminal of Z is linked to the $G^L$ via a thioether bond.

In one embodiment, in the antibody-drug conjugate of formula II or a pharmaceutically acceptable salt thereof:

Z is

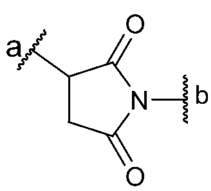

or

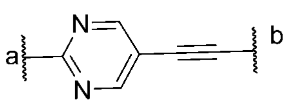

,

wherein "b" terminal is linked to X and "a" terminal is linked to $G^L$; $G^L$ is an antibody (e.g., an anti-HER2 antibody);
t is 1-8;
$R^1$, $R^2$, A, n,

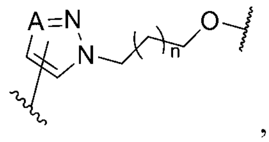

,

Q, X are as described in any one of the embodiments of the present invention.

In one embodiment, the antibody-drug conjugate of formula II or a pharmaceutically acceptable salt thereof, and the antibody-drug conjugate of formula II is any one of the following structures:

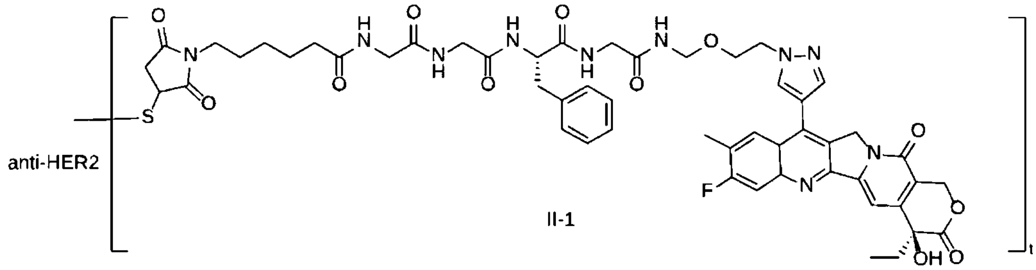

II-1

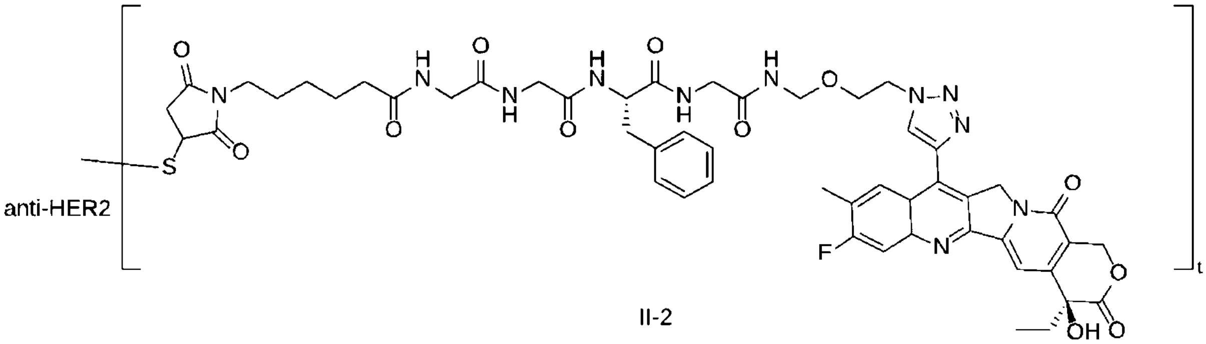

II-2

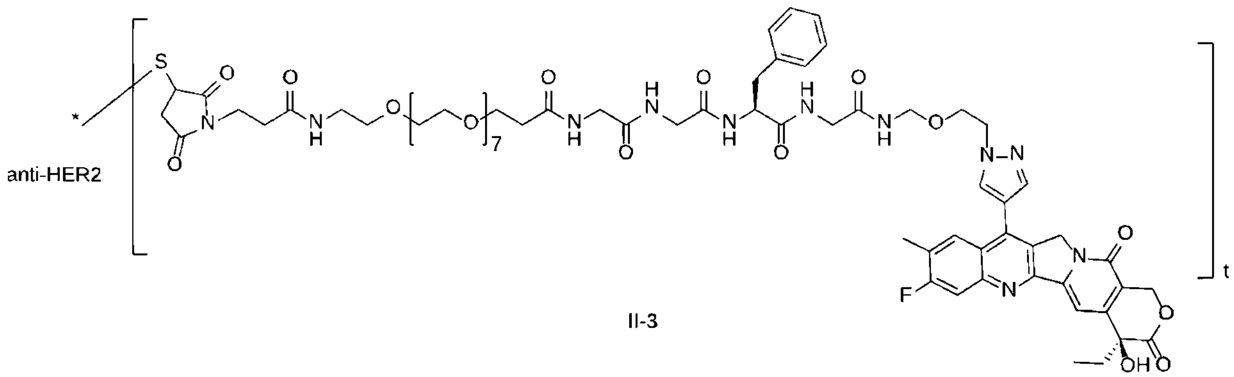

II-3

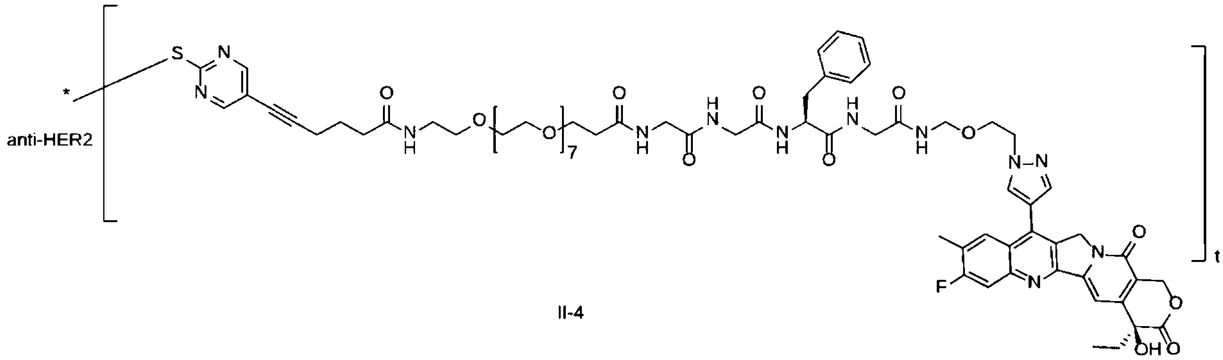

II-4

wherein the anti-HER2 antibody is trastuzumab.

In one embodiment, the antibody-drug conjugate of formula II is any one of the following structures:

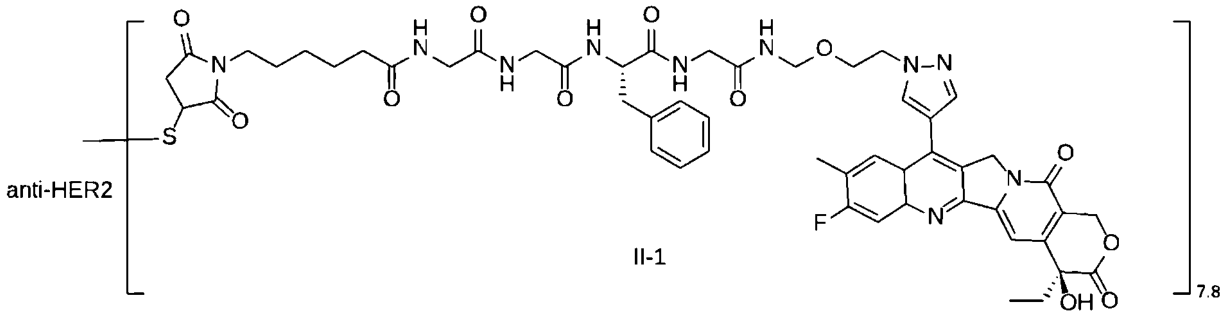

II-1

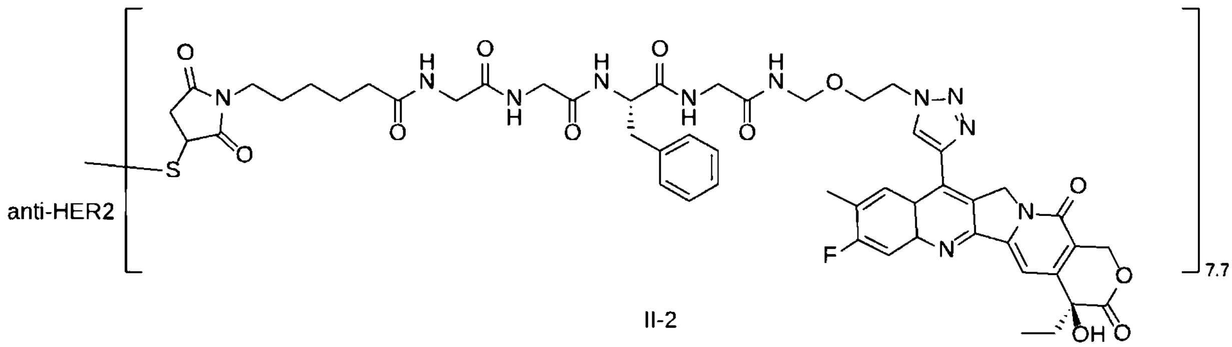

II-2

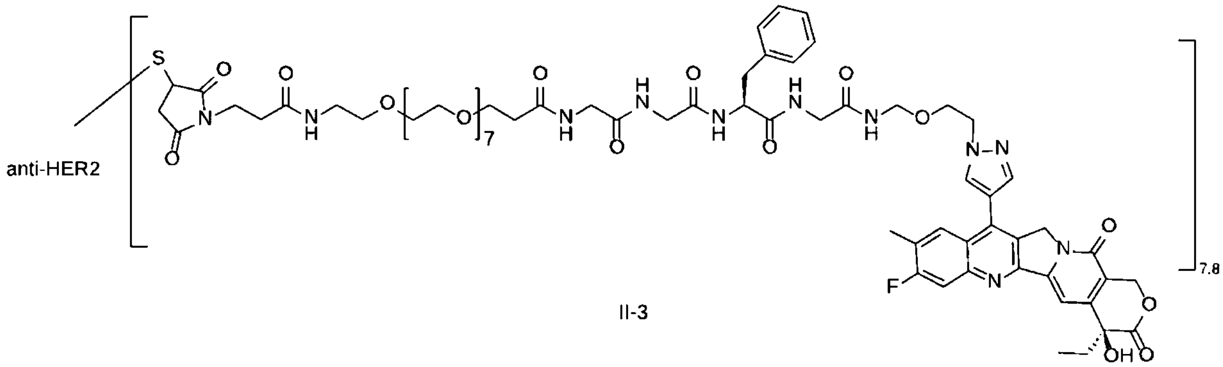

II-3

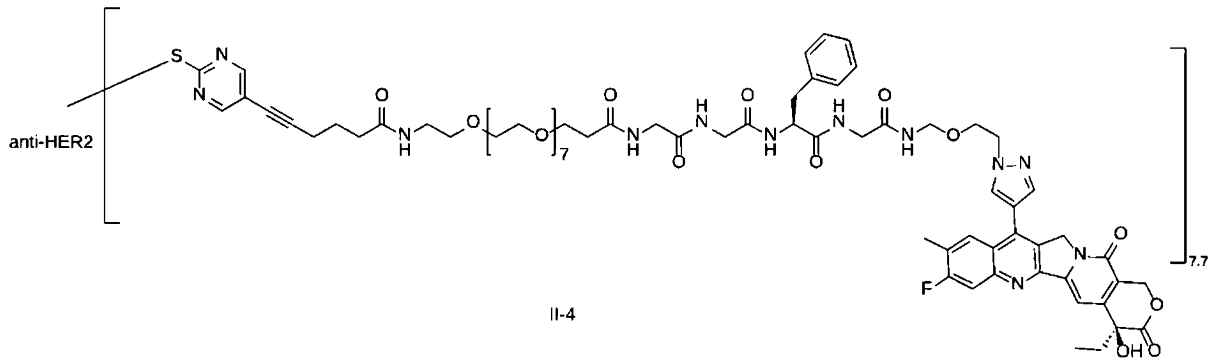

II-4

wherein the anti-HER2 antibody is trastuzumab.

The present invention provides a preparation method of the antibody-drug conjugate of formula II or a pharmaceutically acceptable salt thereof, which comprises the following steps: subjecting the linker-drug conjugate of formula I or a pharmaceutically acceptable salt thereof and an antibody to a conjugation reaction, to obtain the antibody-drug conjugate of formula II or a pharmaceutically acceptable salt thereof;

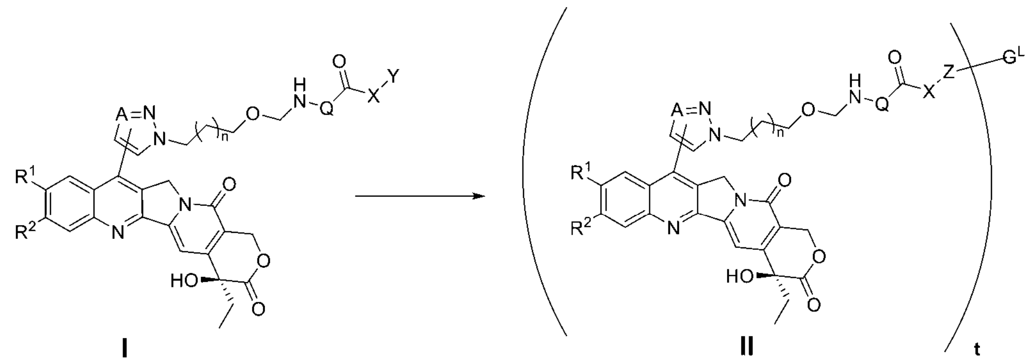

I II

wherein $R^1$, $R^2$, A, n,

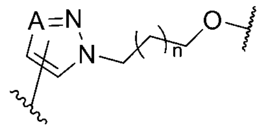

,

Q, X, $R^a$, $R^b$, Z, $G^L$, t, m, p, q and r are independently as described in any one of the embodiments of the present invention.

The present invention provides a preparation method of the camptothecin compound of formula K or a pharmaceutically acceptable salt thereof, which is scheme 1 or scheme 2;

scheme 1 comprises the following steps: in a haloalkane solvent (e.g., dichloromethane) and under the action of a catalyst (e.g., tetrakis(acetonitrle)copper(I) hexafluorophosphate), subjecting the compound of formula Ka and the azide compound of formula Kb to a cyclization reaction to obtain the compound of formula K;

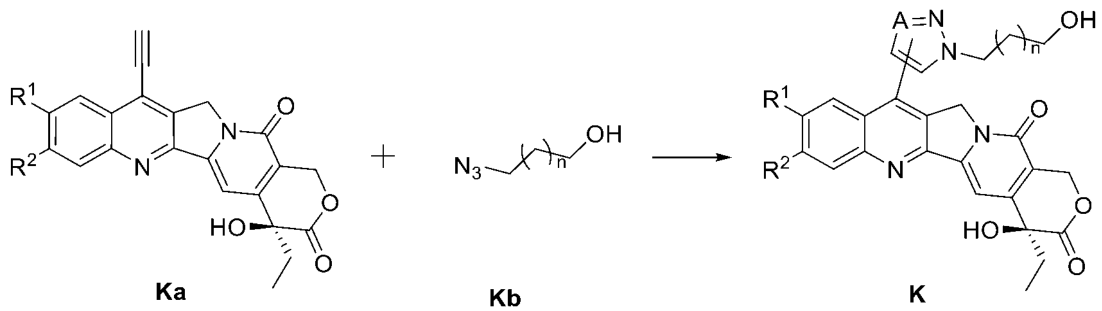

Ka Kb K

wherein A is N;

$R^1$, $R^2$ and n are as described in any one of the embodiments of the present invention;

scheme 2 comprises the following steps: in an aromatic hydrocarbon solvent (e.g., toluene) and under the presence of an organic acid (e.g., acetic acid), subjecting the compound of formula Kc and the compound of formula Kd to a cyclization reaction to obtain the compound of formula K;

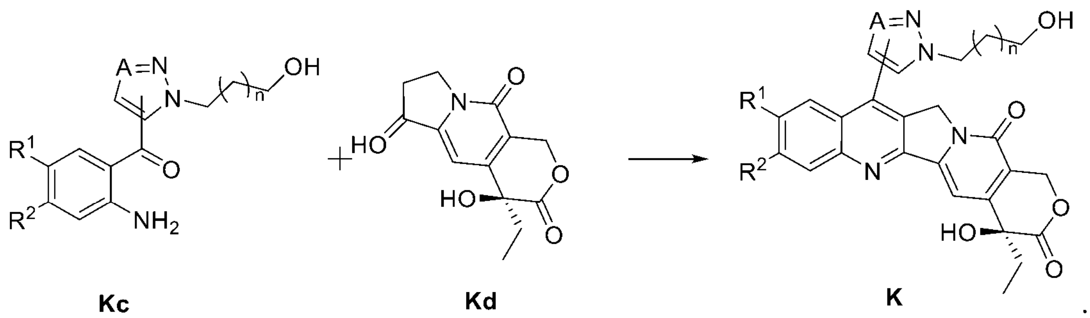

Kc Kd K

;

wherein A is CH;
$R^1$, $R^2$ and n are as described in any one of the embodiments of the present invention.

The present invention further provides a compound of formula Ka or a pharmaceutically acceptable salt thereof;

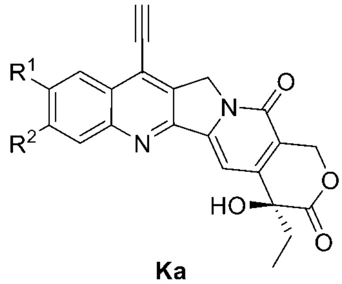

**Ka**

wherein $R^1$ and $R^2$ are as described in any one of the embodiments of the present invention.

Preferably, the compound of formula Ka is

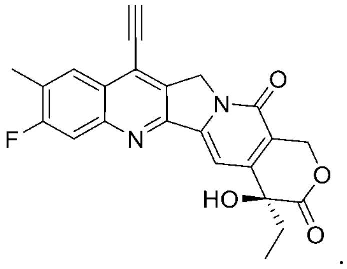

The present invention further provides the compound of formula Kc or a pharmaceutically acceptable salt thereof;

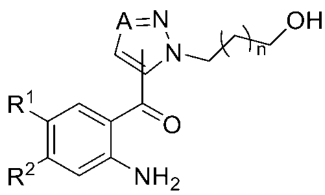

**Kc**

wherein, A is CH, and $R^1$, $R^2$ and n are as described in any one of the embodiments of the present invention.

Preferably, the compound of formula Kc is any one of the following compounds:

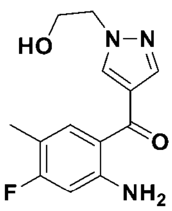 , 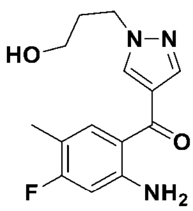 , 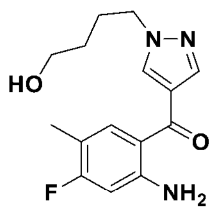 , 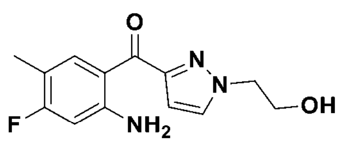 ,

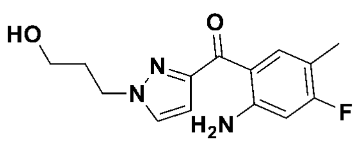 or 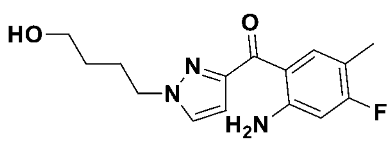 .

The present invention further provides a pharmaceutical composition, comprising the camptothecin compound of formula K or a pharmaceutically acceptable salt thereof, the linker-drug conjugate of formula I or a pharmaceutically

acceptable salt thereof, or the antibody-drug conjugate of formula II or a pharmaceutically acceptable salt thereof as described in any one of the embodiments of the present invention, and pharmaceutical excipients.

The present invention further provides a use of a (therapeutically effective amount of) camptothecin compound of formula K or a pharmaceutically acceptable salt thereof, linker-drug conjugate of formula I or a pharmaceutically acceptable salt thereof, or antibody-drug conjugate of formula II or a pharmaceutically acceptable salt thereof as described in any one of the embodiments of the present invention, or the aforementioned pharmaceutical composition, in the manufacture of a medicament for preventing and/or treating cancer.

The cancer is preferably breast cancer, lung cancer (e.g., lung adenocarcinoma), renal cancer, liver cancer, ovarian cancer, urethral cancer, prostate cancer, glioblastoma multiforme, pancreatic cancer, colorectal cancer, gastrointestinal stromal tumor, cervical cancer, squamous cell carcinoma, peritoneal cancer, colon cancer, rectal cancer, uterine cancer, salivary gland cancer, renal cancer, vulvar cancer, thyroid cancer, penile cancer, leukemia, malignant lymphoma, plasmacytoma, myeloma, sarcoma, melanoma, bladder cancer, gastric cancer or esophageal cancer; preferably breast cancer, or preferably lung adenocarcinoma, gastric cancer or colon cancer, such as gastric cancer, lung cancer, colon cancer or breast cancer.

Definition of Terms

The stereochemical definitions and rules used in the present invention generally follow S. P. Parker, Ed., McGraw-Hill Dictionary of Chemical Terms (1984), McGraw-Hill Book Company, New York; and Eliel, E. and Wilen, S., "Stereochemistry of Organic Compounds", John Wiley & Sons, Inc., New York, 1994.

Depending on the choice of starting materials and methods, the compounds of the present invention may exist in the form of one of the possible isomers or a mixture thereof, such as racemates and mixtures of diastereoisomers (depending on the number of asymmetric carbon atoms). Optically active (R)- or (S)-isomers can be prepared using chiral synthons or chiral reagents, or resolved by conventional techniques.

Any resulting mixture of stereoisomers can be separated into pure or substantially pure geometric isomers, enantiomers, or diastereoisomers based on differences in the physicochemical properties of the components, for example, by chromatography and/or fractional crystallization.

In the present invention, the groups and their substituents may be selected by those skilled in the art to provide stable moieties and compounds. When a substituent is described by a conventional chemical formula written from left to right, the substituent also comprises chemically equivalent substituents obtained when the structural formula is written from right to left.

Those skilled in the art will understand that, according to conventions used in the art, the " 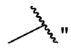 " used in the structural formulae of the groups described in the present invention means that the corresponding group is linked to other fragments or groups in the compound through this site.

In the present invention, a single dash "-" may be added before the substituent, indicating that the named substituent is linked to the parent moiety via a single bond. For example, $CH_3$-C(=O)- means that the C(=O) moiety in the acetyl group is linked to the parent moiety via a single bond.

The term "halogen" refers to fluorine, chlorine, bromine or iodine.

The term "alkyl" refers to a straight-chain or branched-chain alkyl group having a specified number of carbon atoms (e.g., $C_1$~$C_6$). Alkyl groups include but are not limited to methyl, ethyl, n-propyl, isopropyl, n-butyl, tert-butyl, isobutyl, sec-butyl, n-pentyl, n-hexyl, n-heptyl, n-octyl and the like.

In the present application, as a group or part of other groups, the term "alkoxy" refers to - O-alkyl, where the definition of alkyl is as described above.

The term "cycloalkyl" refers to a saturated monocyclic cyclic group composed solely of carbon atoms and having a specified number of carbon atoms (e.g., $C_{3-6}$, $C_{3-10}$). Cycloalkyl groups include but are not limited to cyclopropyl, cyclobutyl, cyclopentyl, cyclohexyl and the like.

The term "heterocyclic group" refers to a cyclic group having a specified number of ring atoms (e.g., 5- to 10-membered, 3- to 10-membered), a specified number of heteroatoms (e.g., 1, 2 or 3), and specified types of heteroatoms (one or more of N, O and S), which is monocyclic, bridged or spirocyclic, and each ring is saturated. Heterocycloalkyl groups include but are not limited to azetidinyl, pyrrolidinyl, tetrahydrofuranyl, morpholinyl, piperidinyl and the like.

The term "aryl" refers to a cyclic group having a specified number of carbon atoms (e.g., $C_{6-10}$, $C_{6-14}$) and consisting solely of carbon atoms, which is monocyclic or polycyclic, and at least one ring has aromaticity (satisfying Hückel's rule). The aryl group is linked to other fragments in the molecule via an aromatic ring or a non-aromatic ring. Aryl groups include but are not limited to phenyl, naphthyl and the like.

The term "heteroaryl" refers to a cyclic group having a specified number of ring atoms (e.g., 5- to 10-membered), a specified number of heteroatoms (e.g., 1, 2 or 3), and specified types of heteroatoms (one or more of N, O and S), which is monocyclic or polycyclic, and at least one ring has aromaticity (complying with Hückel's rule). The heteroaryl group is

linked to other fragments in the molecule via an aromatic ring or a non-aromatic ring. Heteroaryl groups include but are not limited to furanyl, pyrrolyl, thienyl, pyrazolyl, imidazolyl, oxazolyl, thiazolyl, pyridinyl, pyrimidinyl, indolyl and the like.

The term "pharmaceutically acceptable salt" refers to a salt obtained by reacting the compound with a pharmaceutically acceptable (relatively non-toxic, safe and suitable for use in patients) acid or base. When the compound comprises a relatively acidic functional group, a base addition salt can be obtained by contacting the free form of the compound with a sufficient amount of a pharmaceutically acceptable base in a suitable inert solvent. When the compound comprises a relatively basic functional group, an acid addition salt can be obtained by contacting the free form of the compound with a sufficient amount of a pharmaceutically acceptable acid in a suitable inert solvent.

The term "pharmaceutical excipients" refers to the excipients and additives used in the production of pharmaceuticals and the preparation of prescriptions, and are all substances comprised in pharmaceutical preparations except for active ingredients. For details, refer to the Pharmacopoeia of the People's Republic of China (2020 Edition) or Handbook of Pharmaceutical Excipients (Raymond C Rowe, 2009).

The term "treatment" refers to a therapeutic treatment. When referring to a specific disorder, treatment means: (1) alleviating one or more biological manifestations of the disease or disorder; (2) interfering with (a) one or more points in the biological cascade that causes or induces the disorder, or (b) one or more biological manifestations of the disorder; (3) improving one or more symptoms, effects or side effects associated with the disorder, or one or more symptoms, effects or side effects associated with the disorder or its treatment; or (4) slowing the progression of the disorder or one or more biological manifestations of the disorder.

The term "prevention" refers to a reduction in the risk of the acquisition or occurrence of a disease or disorder.

The term "therapeutically effective amount" refers to the amount of a compound that is sufficient to effectively treat the diseases or disorders described herein when administered to a patient. The "therapeutically effective amount" will vary depending on the compound, the disorder and its severity, and the age of the patient to be treated, but can be adjusted as needed by those skilled in the art.

The term "patient" refers to any animal that is to receive or has received administration of the compound or composition according to the embodiments of the present invention, preferably a mammal, and most preferably a human. The term "mammal" includes any mammal. Examples of mammals include but are not limited to cattle, horses, sheep, pigs, cats, dogs, mice, rats, rabbits, guinea pigs, monkeys, humans and the like, with humans being most preferred.

Without departing from the common general knowledge in the art, the above preferred conditions may be combined arbitrarily to obtain various preferred embodiments of the present invention.

All reagents and raw materials used in the present invention are commercially available.

The positive and progressive effects of the present invention are as follows: the present invention provides a novel-structured camptothecin compounds and antibody-drug conjugates thereof. The camptothecin compounds and antibody-drug conjugates thereof exhibit excellent cytotoxic activity and are capable of inhibiting the proliferation of tumor cells.

## DESCRIPTION OF DRAWINGS

Figure 1 shows the volume changes of gastric cancer tumor cell under the treatment of the antibody-drug conjugate.
Figure 2 shows the body weight changes of mouse under the treatment of the antibody-drug conjugate.
Figure 3 shows the volume changes of breast cancer tumor cell under the treatment of the antibody-drug conjugate.
Figure 4 shows the body weight changes of mouse under the treatment of the antibody-drug conjugate.

## DETAILED DESCRIPTION

The present invention is further illustrated below by examples, but the present invention is not thereby limited to the scope of the examples. For the experimental methods without specifying specific conditions in the following examples, they are selected according to conventional methods and conditions or according to commercial product specifications.

In the following examples, DMAP represents 4-dimethylaminopyridine; rac-BINAP represents rac-1,1'-binaphthalene-2,2'-bis(diphenylphosphine); PBS represents phosphate- buffered saline.

Preparation Example 1: Synthesis of Compound 10

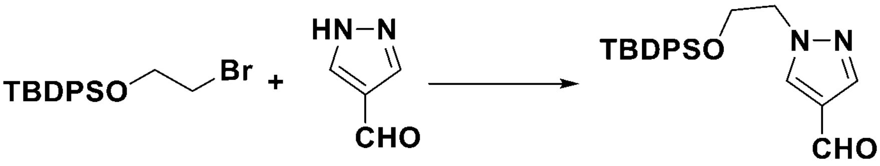

10

Under nitrogen, (2-bromoethoxy) (tert-butyl) diphenylsilane (22.5 g, synthesized with reference to the literature Synlett 2014; 25(19): 2802-2805) and 1H-pyrazole-4-carbaldehyde (5.0 g, commercially available) were dissolved in 100 mL of anhydrous N, N-dimethylformamide. Potassium carbonate (8.6 g) was added at room temperature, and the mixture was heated to 85 °C for reaction for 4 hours. The reaction was monitored by TLC. After TLC indicated that the starting material had been completely reacted, the reaction mixture was cooled to room temperature. 200 mL water was added to quench the reaction, followed by extraction with ethyl acetate, dried over anhydrous sodium sulfate, filtered, and concentrated under reduced pressure, then purified by column chromatography to obtain Compound 10 as a colorless oily substance, 12.8 g, with a yield of 65%.$^1$H NMR (400 MHz, $CDCl_3$) δ 9.86 (s, 1H), 8.02 (s, 1H), 7.98 (s, 1H), 7.54 - 7.48 (m, 4H), 7.45 - 7.33 (m, 6H), 4.27 (t, J = 5.1 Hz, 2H), 4.00 (t, J = 5.1 Hz, 2H), 1.00 (s, 9H).

Preparation Example 2: Synthesis of Compounds 10-1, 10-2, 10-3, 10-4 and 10-5

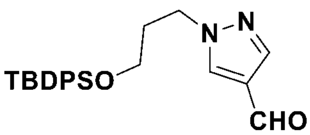

10-1

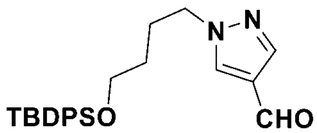

10-2

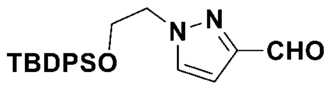

10-3

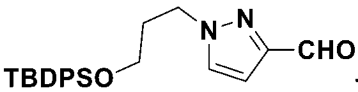

10-4

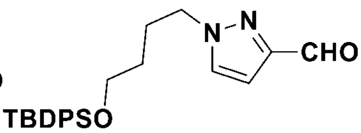

10-5

Referring to the synthesis method of Compound 10 in Preparation Example 1, Compound 10-1 was synthesized using (3-bromopropoxy)(tert-butyl) diphenylsilane (prepared with reference to the synthesis method of Compound 67 in Patent WO2022063278) and 1H-pyrazole-4-carbaldehyde: $^1$H NMR (400 MHz, $CDCl_3$) δ 9.80 (s, 1H), 7.94 (s, 1H), 7.79 (s, 1H), 7.69 - 7.61 (m, 4H), 7.45 - 7.36 (m, 6H), 4.33 (t, J = 6.8 Hz, 2H), 3.65 (t, J = 5.7 Hz, 2H), 2.16 - 2.06 (m, 2H), 1.09 (s, 9H).

Referring to the synthesis method of Compound 10 in Preparation Example 1, Compound 10-2 is synthesized using (4-bromobutoxy)(tert-butyl)diphenylsilane (prepared with reference to the synthesis method of compound 70 in Patent WO2022063278) and 1H-pyrazole-4-carbaldehyde: $^1$H NMR (400 MHz, $CDCl_3$) δ 9.83 (s, 1H), 7.95 (s, 1H), 7.85 (s, 1H), 7.70 - 7.59 (m, 4H), 7.43 - 7.36 (m, 6H), 4.15 (t, J = 7.1 Hz, 2H), 3.70 (t, J = 6.1 Hz, 2H), 2.08 - 1.93 (m, 2H), 1.60 - 1.49 (m, 2H), 1.07 (s, 9H).

Referring to the synthesis method of Compound 10 in Preparation Example 1, Compound 10-3 is synthesized using (2-bromoethoxy)(tert-butyl)diphenylsilane and 1H-pyrazole-3-carbaldehyde (commercially available): $^1$H NMR (400 MHz, $CDCl_3$) δ 9.85 (s, 1H), 7.59 (d, J = 2.0 Hz, 1H), 7.52 - 7.48 (m, 4H), 7.41 - 7.32 (m, 6H), 6.90 (d, J = 2.0 Hz, 1H), 4.74 (t, J = 5.5 Hz, 2H), 3.98 (t, J = 5.5 Hz, 2H), 0.95 (s, 9H).

Referring to the synthesis method of Compound 10 in Preparation Example 1, Compound 10-4 is synthesized using (3-bromopropoxy)(tert-butyl)diphenylsilane and 1H-pyrazole-3-carbaldehyde: $^1$H NMR (400 MHz, $CDCl_3$) δ 9.86 (s, 1H), 7.69 - 7.65 (m, 4H), 7.53 (d, J = 2.1 Hz, 1H), 7.43 - 7.35 (m, 6H), 6.88 (d, J = 2.1 Hz, 1H), 4.82 - 4.60 (m, 2H), 3.71 (t, J = 6.0 Hz, 2H), 2.13 - 2.03 (m, 2H), 1.07 (s, 9H).

Referring to the synthesis method of Compound 10 in Preparation Example 1, Compound 10-5 is synthesized using (4-bromobutoxy)(tert-butyl)diphenylsilane and 1H-pyrazole-3-carbaldehyde: $^1$H NMR (400 MHz, $CDCl_3$) δ 9.85 (s, 1H), 7.68 - 7.65 (m, 4H), 7.55 (d, J = 2.0 Hz, 1H), 7.43 - 7.37 (m, 6H), 6.88 (d, J = 2.0 Hz, 1H), 4.57 (t, J = 7.1 Hz, 2H), 3.68 (t, J = 6.2 Hz, 2H), 2.01 - 1.89 (m, 2H), 1.61 - 1.51 (m, 2H), 1.06 (s, 9H).

Example 1: Preparation of Compounds K1, K2 and K3

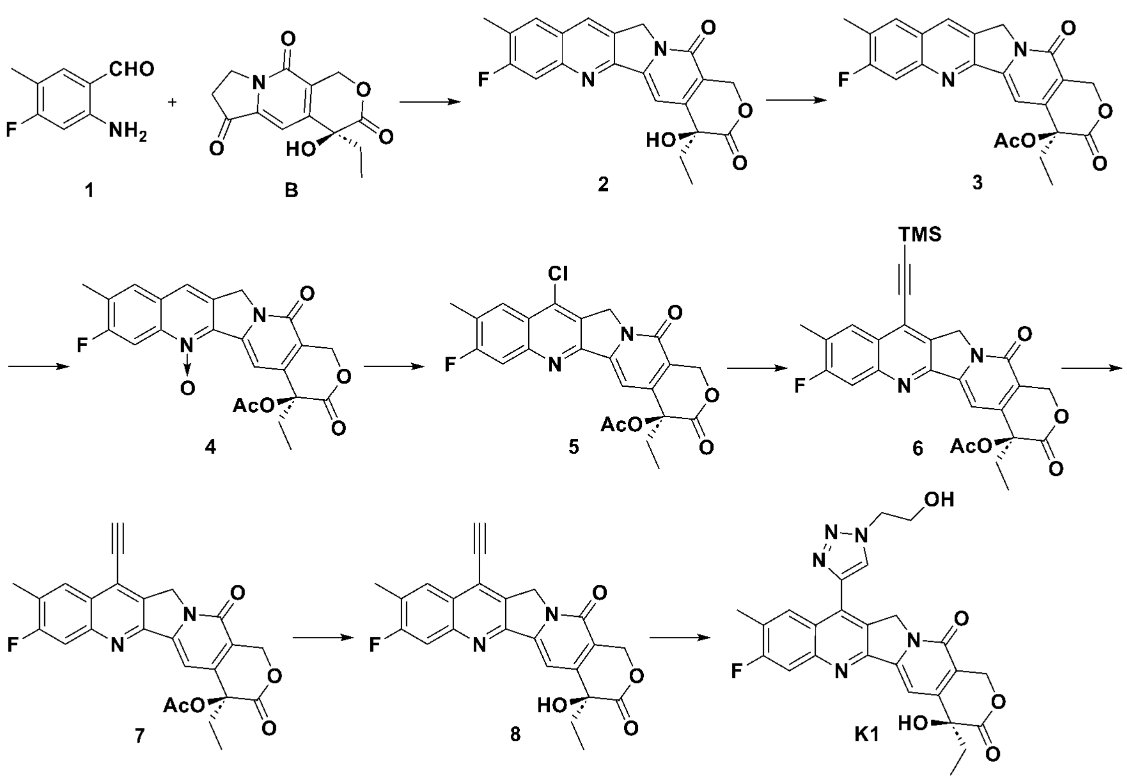

1 B 2 3 4 5 6 7 8 K1

Step 1: Synthesis of Compound 2

Anhydrous N, N-dimethylformamide (100 mL), Compound 1 (7.0 g, commercially available), Compound B (10.0 g, CAS: 110351-94-5, purchased from Shanghai Xiyao Pharmaceutical Technology Co., Ltd.), and iodine (0.96 g) were added to a 250 mL three-necked flask. The mixture was heated to 80°C and stirred for 8 h under nitrogen. The reaction was monitored by TLC. After TLC indicated that the starting material had been completely reacted, the reaction mixture was slowly poured into an ice-water mixture. A solid precipitated, which was filtered and dried to obtain Compound 2 as a brown solid (10 g, yield 69%). $^1$H NMR (400 MHz, DMSO) δ 8.59 (s, 1H), 8.02 (d, J = 8.3 Hz, 1H), 7.85 (d, J = 11.0 Hz, 1H), 7.30 (s, 1H), 6.52 (s, 1H), 5.42 (s, 2H), 5.21 (s, 2H), 2.46 (s, 3H), 1.95 - 1.79 (m, 2H), 0.88 (t, J = 7.1 Hz, 3H). MS (ESI) m/z =381.1 (M + $H^+$).

Step 2: Synthesis of Compound 3

Anhydrous dichloromethane (250 mL), Compound 2 (2.50 g), DMAP (0.12 g), anhydrous pyridine (5.31 mL), and acetic anhydride (3.75 mL) were added to a 500 mL three-necked flask. The mixture was stirred at room temperature for reaction for 8 h under nitrogen. The reaction was monitored by TLC. After TLC indicated that the starting material had been completely reacted, the reaction mixture was sequentially washed with dilute hydrochloric acid, saturated sodium bicarbonate solution, and saturated sodium chloride solution, then separated. The organic phase was dried and concentrated under reduced pressure to obtain 2.2 g of crude Compound 3. The crude product was used directly in the next reaction without further purification.

Step 3: Synthesis of Compound 4

Acetic acid (50 mL) and Compound 3 (2.0 g) were added to a 100 mL single-necked flask. The mixture was stirred at room temperature to dissolve, then 30% hydrogen peroxide solution (5.0 mL) was added. The mixture was heated to 70°C and stirred for reaction for 3 h. The reaction was monitored by TLC. After TLC indicated that the starting material had been completely reacted, the reaction mixture was cooled to room temperature. Acetic acid was evaporated under reduced pressure, and the residue was dissolved in chloroform. The solution was sequentially washed with saturated

sodium bicarbonate solution, purified water, and saturated sodium chloride solution, then separated. The organic phase was dried and concentrated under reduced pressure to obtain 1.5 g of crude Compound 4. The crude product was used directly in the next reaction without further purification.

Step 4: Synthesis of Compound 5

Anhydrous N,N-dimethylformamide (100 mL) and Compound 4 (1.00 g) were added to a 100 mL single-necked flask.After complete dissolution, the mixture was cooled to 0°C, and oxalyl chloride (0.45 mL) was slowly added dropwise. After the dropwise addition, the mixture was heated to room temperature and stirred for reaction for 1 h. The reaction was monitored by TLC. After TLC indicated that the starting material had been completely reacted, part of the N,N-dimethylformamide was evaporated under reduced pressure. Then the remaining reaction solution was slowly poured into an ice-water mixture, and a solid precipitated, which was filtered. The solid was dried and purified by column chromatography to obtain Compound 5 as a yellow solid (0.8 g, yield 80%). $^1$H NMR (400 MHz, $CDCl_3$) δ 8.14 (d, J = 7.8 Hz, 1H), 7.82 (d, J = 10.3 Hz, 1H), 7.16 (s, 1H), 5.68 (d, J = 17.3 Hz, 1H), 5.40 (d, J = 17.4 Hz, 1H), 5.29 (s, 2H), 2.58 (s, 3H), 2.33 - 2.24 (m, 1H), 2.22 (s, 3H), 2.14 (dq, J = 14.4, 7.1 Hz, 1H), 0.98 (t, J = 7.4 Hz, 3H). MS (ESI) m/z =457.1 (M + $H^+$).

Step 5: Synthesis of Compound 6

Anhydrous toluene (10 mL), Compound 5 (3.0 g), anhydrous potassium carbonate (1.8 g), palladium acetate (0.14 g), rac-BINAP (0.81 g), and trimethylsilylacetylene (3.71 mL) were added to a 100 mL sealed tube. After purging with nitrogen, the tube was sealed, heated to 100°C and stirred for reaction for 15 h. The reaction was monitored by TLC. After TLC indicated that the starting material had been completely reacted, the reaction mixture was cooled to room temperature. The solvent was evaporated under reduced pressure, and the residue was purified by column chromatography to obtain Compound 6 as a yellow solid (3.0 g, yield 88%). $^1$H NMR (400 MHz, $CDCl_3$) δ 8.12 (d, J = 7.9 Hz, 1H), 7.78 (d, J = 10.4 Hz, 1H), 7.15 (s, 1H), 5.68 (d, J = 17.2 Hz, 1H), 5.41 (d, J = 17.2 Hz, 1H), 5.28 (s, 2H), 2.57 (s, 3H), 2.33 - 2.25 (m, 1H), 2.22 (s, 3H), 2.14 (td, J = 15.0, 7.6 Hz, 1H), 0.98 (t, J = 7.4 Hz, 3H), 0.40 (s, 9H). MS (ESI) m/z =518.2 (M + $H^+$).

Step 6: Synthesis of Compound 7

Methanol (100 mL), Compound 6 (3.0 g), potassium fluoride (1.34 g), and acetic acid (1.0 mL) were added to a 250 mL single-necked flask. The mixture was stirred at room temperature for reaction for 2 h under nitrogen. The reaction was monitored by TLC. After TLC indicated that the starting material had been completely reacted, the solvent was evaporated under reduced pressure. The residue was dissolved in chloroform, sequentially washed with purified water and saturated sodium chloride solution, then separated. The organic phase was dried and concentrated under reduced pressure to obtain 2.8 g of crude product. The crude product was recrystallized from ethanol to obtain 2.3 g of Compound 7 (yield 90%). $^1$H NMR (400 MHz, $CDCl_3$) δ 8.17 (d, J = 8.2 Hz, 1H), 7.81 (d, J = 10.1 Hz, 1H), 7.16 (s, 1H), 5.68 (d, J = 17.1 Hz, 1H), 5.41 (d, J = 17.3 Hz, 1H), 5.31 (s, 2H), 4.03 (s, 1H), 2.57 (s, 3H), 2.34 - 2.24 (m, 1H), 2.22 (s, 3H), 2.19 - 2.10 (m, 1H), 0.98 (t, J = 7.4 Hz, 3H). MS (ESI) m/z =447.1 (M + $H^+$).

Step 7: Synthesis of Compound 8

Concentrated sulfuric acid (10 mL) was added to a 10 mL single-necked flask. After cooling to 0°C, Compound 7 (1.0 g) was added. The mixture was warmed to room temperature and stirred for reaction for 1 h under nitrogen. The reaction was monitored by TLC. After TLC indicated that the starting material had been completely reacted, the reaction solution was slowly poured into an ice-water mixture. The pH was adjusted to neutral with saturated sodium bicarbonate solution, and a solid precipitated. The solid was filtered and dried to obtain 0.7 g of Compound 8 (yield 80%). $^1$H NMR (400 MHz, DMSO) δ 8.18 (d, J = 8.2 Hz, 1H), 7.96 (d, J = 10.7 Hz, 1H), 7.31 (s, 1H), 6.55 (s, 1H), 5.51 (s, 1H), 5.44 (s, 2H), 5.26 (s, 2H), 2.53 (s, 3H), 1.93 - 1.80 (m, 2H), 0.88 (t, J = 7.3 Hz, 3H). MS (ESI) m/z =404.1 (M + $H^+$).

Step 8: Synthesis of Compound K1

Dichloromethane (10 mL), Compound 8 (0.1 g), 2-azidoethanol (0.03 g), and tetrakis(acetonitrle)copper(I) hexafluorophosphate (0.02 g) were added to a 10 mL single-necked flask. The mixture was stirred at room temperature for reaction for 1.5 h under nitrogen. The reaction was monitored by TLC. After TLC indicated that the starting material had been completely reacted, the reaction mixture was sequentially washed with purified water and saturated sodium chloride solution, then separated. The organic phase was dried and concentrated under reduced pressure to obtain a crude product.The crude product was purified by column chromatography to obtain 0.08 g of Compound K1 (yield 65%). $^1$H NMR (400 MHz, DMSO) δ 9.00 (s, 1H), 8.49 (d, J = 8.0 Hz, 1H), 7.94 (d, J = 10.6 Hz, 1H), 7.34 (s, 1H), 6.54 (s, 1H), 5.44 (s, 2H),

5.36 (s, 2H), 5.18 (s, 1H), 4.64 (s, 2H), 3.96 (s, 2H), 2.50 (s, 3H), 1.88 (tt, J = 14.4, 7.1 Hz, 2H), 0.89 (t, J = 7.0 Hz, 3H). MS (ESI) m/z =492.2 (M + $H^+$).

Referring to the above synthesis method, 2-azidoethanol is replaced with 3-azidopropanol and 4-azidobutanol respectivelyto synthesize Compound K2 and Compound K3.

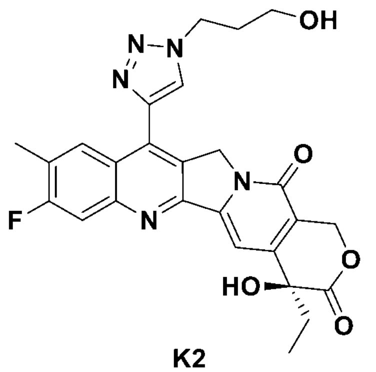

**K2**

**K2:** $^1$H NMR (400 MHz, DMSO) δ 9.01 (s, 1H), 8.49 (d, *J* = 8.0 Hz, 1H), 7.94 (d, *J* = 10.5 Hz, 1H), 7.33 (s, 1H), 6.54 (s, 1H), 5.44 (s, 2H), 5.36 (s, 2H), 4.60 (t, *J* = 7.0 Hz, 2H), 4.53 (t, *J* = 5.0 Hz, 1H), 3.48 (dd, *J* = 11.1, 5.7 Hz, 2H), 2.50 (s, 3H), 2.00 (m, 2H), 1.93 - 1.81 (m, 2H), 0.89 (t, *J* = 7.0 Hz, 3H). MS (ESI) m/z =506.2 (M + $H^+$).

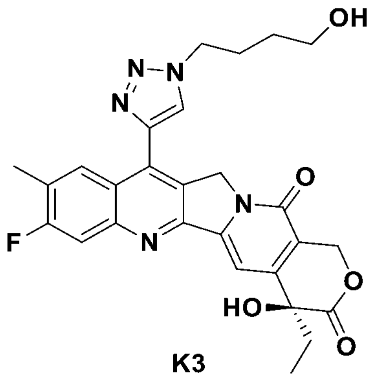

**K3**

K3: $^1$H NMR (400 MHz, DMSO) δ 9.08 (s, 1H), 8.49 (d, *J* = 8.0 Hz, 1H), 7.94 (d, *J* = 10.5 Hz, 1H), 7.33 (s, 1H), 6.54 (s, 1H), 5.44 (s, 2H), 5.36 (s, 2H), 4.60 (t, *J*= 7.0 Hz, 2H), 4.53 (t, *J* = 5.0 Hz, 1H), 3.48 (dd, *J*= 11.1, 5.7 Hz, 2H), 2.50 (s, 3H), 2.09 - 2.00 (m, 2H), 1.93 - 1.81 (m, 2H), 1.56 - 1.47 (m, 2H), 0.89 (t, *J*= 7.0 Hz, 3H). MS (ESI) m/z =520.2 (M + $H^+$).

Example 2: Preparation of Compounds K4, K5, K6, K7, K8 and K9

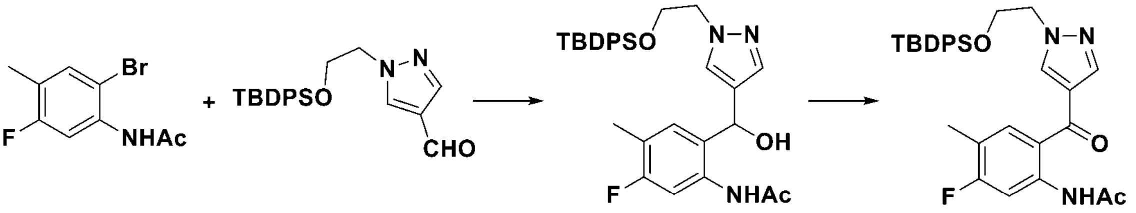

9 10 11 12

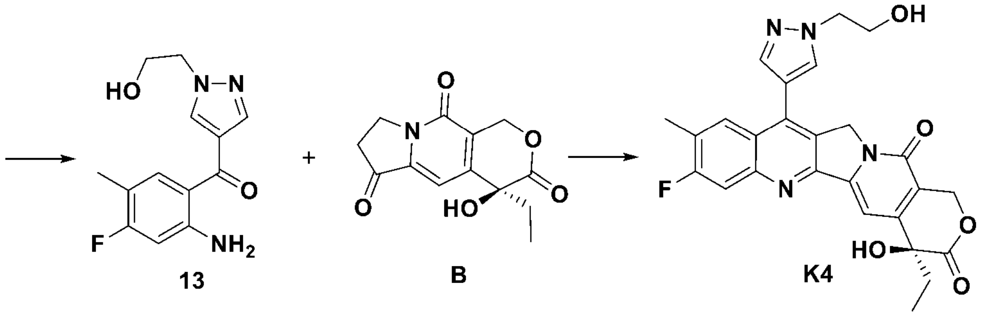

13 B K4

Step 1: Synthesis of Compound 11

Under nitrogen, Compound 9 (1.0 g, synthetic method refers to Example 1 of Patent CN111470998A) was dissolved in 15 mL anhydrous tetrahydrofuran, and the solution was cooled to -78°C for half an hour. Under low temperature, a hexane solution of n-butyllithium (2.5 M, 3.5 mL) was added dropwise, and after the dropwise addition, an anhydrous tetrahydrofuran solution (5 mL) of Compound 10 (1.5 g) was quickly added, followed by continuing the reaction at -78°C for one hour. The reaction was monitored by TLC. After TLC indicated that the starting material had been completely reacted, the reaction was quenched with saturated ammonium chloride solution, extracted with ethyl acetate, dried over anhydrous sodium sulfate, concentrated under reduced pressure, and the residue was purified by column chromatography to obtain Compound 11 as a light yellow oil (0.6 g, yield 25%). $^1$H NMR (400 MHz, $CDCl_3$) δ 8.69 (s, 1H), 7.95 (d, J = 11.6 Hz, 1H), 7.52 - 7.28 (m, 14H), 6.91 (d, J = 8.2 Hz, 1H), 5.86 (s, 1H), 4.24 - 4.13 (m, 2H), 3.99 - 3.87 (m, 2H), 2.15 (s, 3H), 2.00 (s, 3H), 0.96 (s, 9H). MS (ESI) m/z =546.3 (M + $H^+$).

Step 2: Synthesis of Compound 12

Under nitrogen, Compound 11 (0.6 g) was dissolved in 15 mL acetonitrile, manganese dioxide (0.86 g) was added, and the mixture was heated under reflux for 3 h. The reaction was monitored by TLC. After TLC indicated that the starting material had been completely reacted, the mixture was filtered through diatomaceous earth, the filtrate was concentrated under reduced pressure, and the residue was purified by column chromatography to obtain Compound 12 as a colorless oil (0.55 g, yield 92%).$^1$H NMR (400 MHz, $CDCl_3$) δ 10.82 (s, 1H), 8.39 (d, J = 12.4 Hz, 1H), 7.99 (s, 1H), 7.91 (s, 1H), 7.62 (d, J = 8.3 Hz, 1H), 7.55 - 7.33 (m, 10H), 4.30 (t, J = 5.0 Hz, 2H), 4.03 (t, J = 5.0 Hz, 2H), 2.27 - 2.15 (m, 6H), 1.00 (s, 9H). MS (ESI) m/z =544.2 (M + $H^+$).

Step 3: Synthesis of Compound 13

Under nitrogen, Compound 12 (0.55 g) was dissolved in 10 mL ethanol, 3 mL concentrated hydrochloric acid was added, and the mixture was heated under reflux for 3 h with a white solid precipitated. The reaction was monitored by TLC. After TLC indicated that the starting material had been completely reacted, the solid was filtered and dried to obtain 0.19 g Compound 13 (yield 80%), which was used directly in the next reaction without further purification.

Step 4: Synthesis of Compound K4

Under nitrogen, Compound 13 (0.19 g) and Compound B (0.22 g) were dissolved in 10 mL toluene and 5 mL acetic acid, and the mixture was heated to 100°C and reacted overnight. The reaction was monitored by TLC. After TLC indicated that the starting material had been completely reacted, the reaction solution was directly concentrated under reduced pressure, and the residue was purified by column chromatography to obtain 0.28 g Compound K4 as a white solid (yield 80%). $^1$H NMR (400 MHz, DMSO) δ 8.43 (s, 1H), 8.16 (d, J = 8.2 Hz, 1H), 8.08 (s, 1H), 7.91 (d, J = 10.8 Hz, 1H), 7.33 (s, 1H), 5.42 (s, 2H), 5.38 - 5.12 (m, 2H), 4.34 (t, J = 5.5 Hz, 2H), 3.88 (t, J = 5.6 Hz, 2H), 2.47 (s, 3H), 2.00 - 1.73 (m, 2H), 0.89

(t, J = 7.3 Hz, 3H). MS (ESI) m/z =491.2 (M + $H^+$).

Referring to the above synthetic method, Compound 10 was replaced with Compound 10-1, Compound 10-2, Compound 10-3, Compound 10-4 and Compound 10-5 respectively to synthesize Compound K5, Compound K6, Compound K7, Compound K8 and Compound K9.

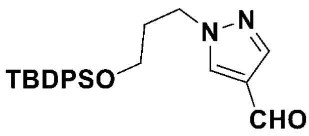

10-1

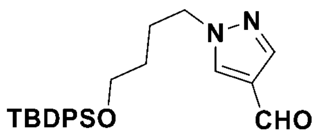

10-2

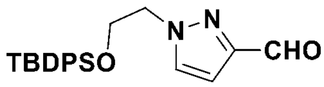

10-3

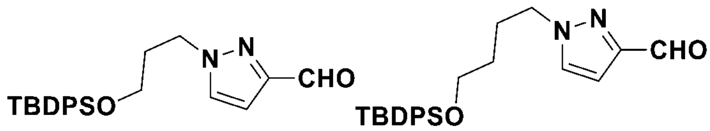

10-4 10-5

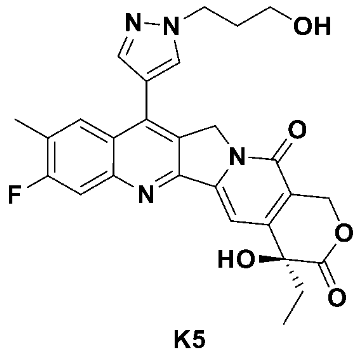

K5

**K5:** $^1H$ NMR (400 MHz, DMSO) δ 8.46 (s, 1H), 8.11 (d, J = 8.3 Hz, 1H), 8.06 (s, 1H), 7.88 (d, J = 10.8 Hz, 1H), 7.31 (s, 1H), 6.50 (s, 1H), 5.42 (s, 2H), 5.33 - 5.12 (m, 2H), 4.37 (t, J = 7.1 Hz, 2H), 3.51 (t, J = 6.1 Hz, 2H), 2.46 (s, 3H), 2.17 - 1.99 (m, 2H), 1.96 - 1.72 (m, 2H), 0.89 (t, J = 7.3 Hz, 3H). MS (ESI) m/z =505.2 (M + $H^+$).

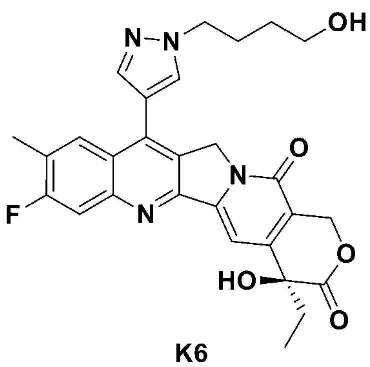

K6

**K6:** $^1H$ NMR (400 MHz, DMSO) δ 8.45 (s, 1H), 8.13 (d, J = 8.3 Hz, 1H), 8.07 (s, 1H), 7.90 (d, J = 10.8 Hz, 1H), 7.32 (s, 1H), 5.41 (s, 2H), 5.34 - 5.16 (m, 2H), 4.39 (t, J = 7.1 Hz, 1H), 3.50 (t, J = 6.1 Hz, 2H), 2.47 (s, 3H), 2.09 - 2.00 (m, 2H), 1.93 - 1.71 (m, 2H), 1.56 - 1.47 (m, 2H), 0.88 (t, *J*= 7.3 Hz, 3H). MS (ESI) m/z =519.2 (M + $H^+$).

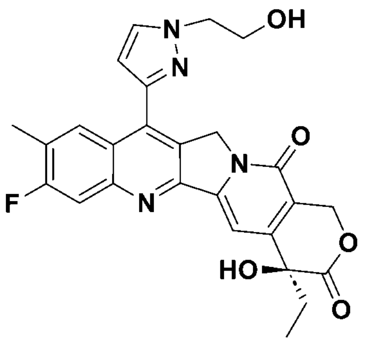

**K7**

**K7:** $^1$H NMR (400 MHz, DMSO) δ 8.33 (d, J = 5.2 Hz, 1H), 8.12 (d, J = 8.2 Hz, 1H), 8.02 (d, J = 5.2 Hz, 1H), 7.91 (d, J = 10.8 Hz, 1H), 7.30 (s, 1H), 5.41 (s, 2H), 5.33 - 5.11 (m, 2H), 4.32 (t, J = 5.4 Hz, 2H), 3.86 (t, J = 5.5 Hz, 2H), 2.47 (s, 3H), 1.98 - 1.72 (m, 2H), 0.89 (t, J = 7.3 Hz, 3H). MS (ESI) m/z =491.2 (M + H$^+$).

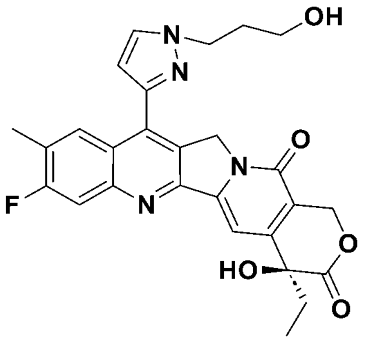

**K8**

**K8:** $^1$H NMR (400 MHz, DMSO) δ 8.31 (d, J = 5.2 Hz, 1H), 8.14 (d, J = 8.2 Hz, 1H), 8.04 (d, J = 5.2 Hz, 1H), 7.93 (d, J = 10.8 Hz, 1H), 7.32 (s, 1H), 5.44 (s, 2H), 5.32 - 5.10 (m, 2H), 4.34 (t, J = 5.4 Hz, 2H), 3.82 (t, J = 5.5 Hz, 2H), 2.45 (s, 3H), 2.14 - 1.96 (m, 2H), 1.98 - 1.72 (m, 2H), 0.89 (t, J = 7.3 Hz, 3H). MS (ESI) m/z =505.2 (M + H$^+$).

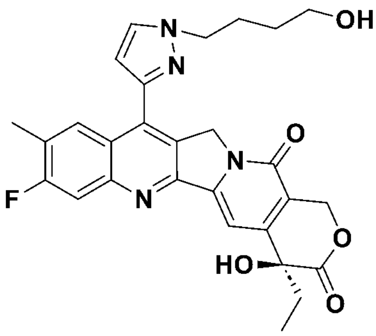

**K9**

**K9:** $^1$H NMR (400 MHz, DMSO) δ 8.36 (d, J = 5.2 Hz, 1H), 8.15 (d, J = 8.2 Hz, 1H), 8.02 (d, J = 5.2 Hz, 1H), 7.91 (d, J = 10.8 Hz, 1H), 7.30 (s, 1H), 5.41 (s, 2H), 5.33 - 5.11 (m, 2H), 4.32 (t, J = 5.4 Hz, 2H), 3.86 (t, J = 5.5 Hz, 2H), 2.47 (s, 3H), 2.17 - 1.99 (m, 2H), 1.98 - 1.72 (m, 2H), 1.56 - 1.47 (m, 2H), 0.89 (t, J = 7.3 Hz, 3H). MS (ESI) m/z =519.2 (M + H$^+$).

Example 3: Preparation of Compounds I-1 and I-2

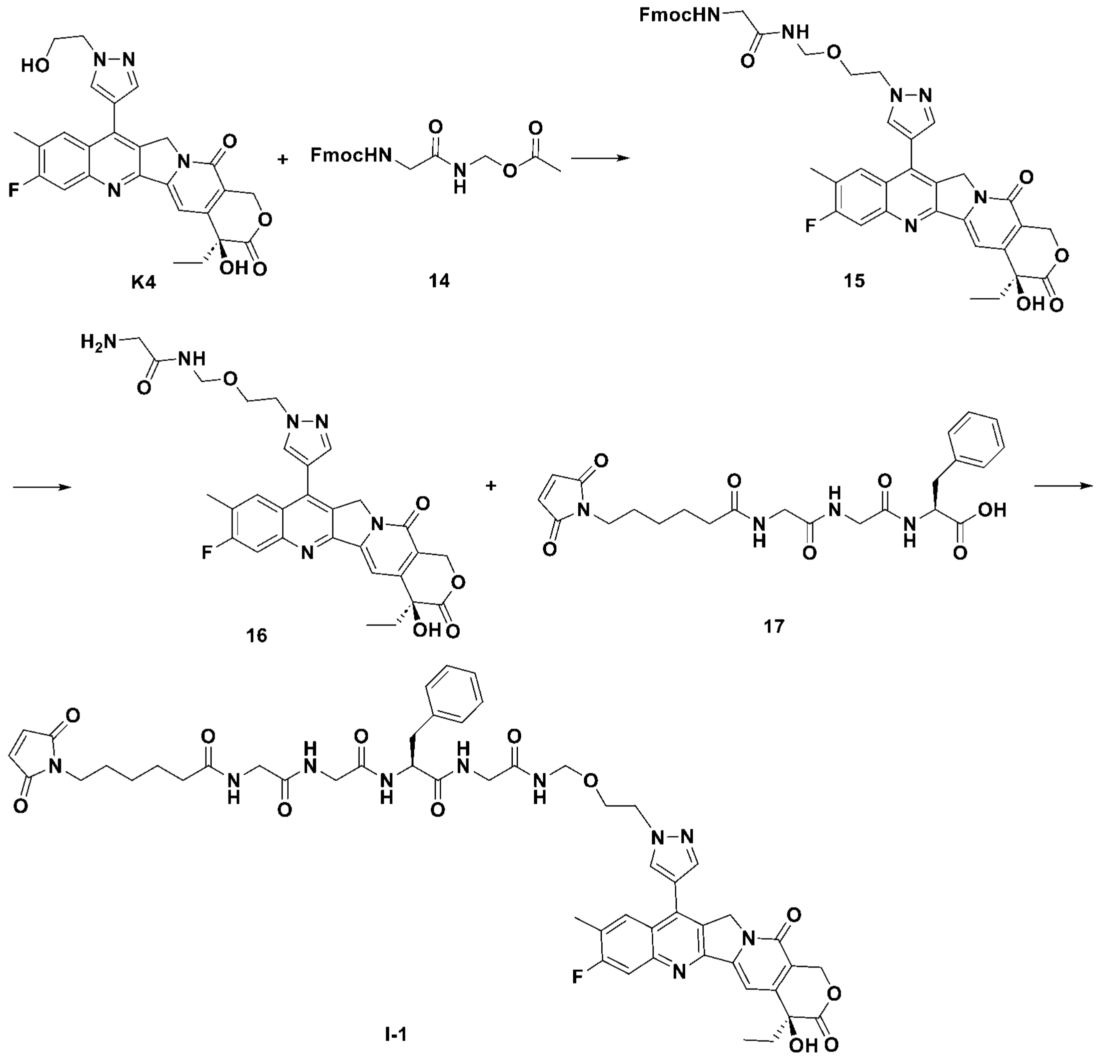

K4 14 15 16 17 I-1

Step 1: Preparation of Compound 15

Under nitrogen, Compound K4 (100 mg) and Compound 14 (150 mg, CAS: 1599440-06-8, purchased from Haijieya (Chengdu) Pharmaceutical Technology Co., Ltd.) were dissolved in 10 mL anhydrous tetrahydrofuran, pyridinium p-toluenesulfonate (25 mg) was added at room temperature, and the mixture was heated to 55°C and reacted overnight. The reaction was quenched with saturated sodium bicarbonate solution, extracted with ethyl acetate, dried over anhydrous sodium sulfate, concentrated under reduced pressure, and the residue was purified by column chromatography to obtain Compound 15 as a white solid (50 mg, yield 30%).

$^1H$ NMR (400 MHz, DMSO) δ 8.76 (t, J = 6.6 Hz, 1H), 8.40 (s, 1H), 8.11 - 8.01 (m, 2H), 7.85 - 7.77 (m, 3H), 7.65 - 7.55 (m, 3H), 7.39 - 7.22 (m, 5H), 6.52 (s, 1H), 5.40 (s, 2H), 5.22 - 5.05 (m, 2H), 4.63 (d, J = 6.6 Hz, 2H), 4.46 (t, J = 5.2 Hz, 2H), 4.25 - 4.18 (m, 2H), 4.17 - 4.10 (m, 1H), 3.90 (t, J = 5.4 Hz, 2H), 3.65 (d, J = 6.0 Hz, 2H), 2.42 (s, 3H), 1.90 - 1.81 (m, 2H), 0.89 (t, J = 7.3 Hz, 3H). MS (ESI) m/z = 799.3 (M + $H^+$).

Step 2: Preparation of Compound 16

Under nitrogen, Compound 15 (50 mg) was dissolved in 5 mL anhydrous tetrahydrofuran, diethylamine (0.5 mL) was added at room temperature, and the mixture was reacted at room temperature for 2 h with a solid precipitated. The reaction was monitored by TLC. After TLC indicated that the starting material had been completely reacted, the solid was filtered and vacuum-dried to obtain Compound 16 as a light yellow solid (30 mg, yield 83%), which was used directly in the next reaction without further purification; MS (ESI) m/z = 577.2 (M + $H^+$).

Step 3: Preparation of Compound I-1

Under nitrogen, Compound 17 (30 mg) was dissolved in 2 mL anhydrous N,N-dimethylformamide, 4-(4,6-dimethoxytriazin-2-yl)-4-methylmorpholinium hydrochloride (18.5 mg) and N,N-diisopropylethylamine (10 μL) were added at room temperature, and the mixture was reacted at room temperature for 2 h. After the reaction solution became clear, Compound 16 (30 mg) was added at room temperature, and the reaction was carried out overnight at room temperature. The reaction was monitored by LC-MS. After starting material Compound 16 had been completely reacted, the reaction solution was directly concentrated under reduced pressure, and the residue was purified by C18 reverse-phase column to obtain Compound I-1 as a light yellow solid (27 mg, yield 50%).

$^1$H NMR (400 MHz, DMSO) δ 8.57 (t, J = 6.6 Hz, 1H), 8.45 (s, 1H), 8.30 (t, J = 5.8 Hz, 1H), 8.17 - 8.07 (m, 3H), 8.04 (t, J = 5.7 Hz, 1H), 7.99 (t, J = 5.6 Hz, 1H), 7.90 (d, J = 10.7 Hz, 1H), 7.33 (s, 1H), 7.26 - 7.10 (m, 5H), 6.98 (s, 2H), 6.51 (s, 1H), 5.42 (s, 2H), 5.35 - 5.18 (m, 2H), 4.62 (d, J = 6.6 Hz, 2H), 4.51 - 4.41 (m, 3H), 3.89 (t, J = 5.4 Hz, 2H), 3.79 - 3.69 (m, 3H), 3.67 - 3.63 (m, 2H), 3.58 (dd, J = 16.7, 5.4 Hz, 1H), 3.35 (t, J = 7.1 Hz, 2H), 3.01 (dd, J = 13.8, 4.5 Hz, 1H), 2.76 (dd, J = 13.7, 9.7 Hz, 1H), 2.47 (s, 3H), 2.09 (t, J = 7.4 Hz, 2H), 1.94 - 1.79 (m, 2H), 1.52 - 1.39 (m, 4H), 1.23 - 1.12 (m, 2H), 0.88 (t, J = 7.3 Hz, 3H). MS (ESI) m/z =1031.4 (M + H$^+$).

Referring to the preparation method of Compound I-1, Compound K4 was replaced with Compound K1, and Compound I-2 was obtained by the same method as described above.

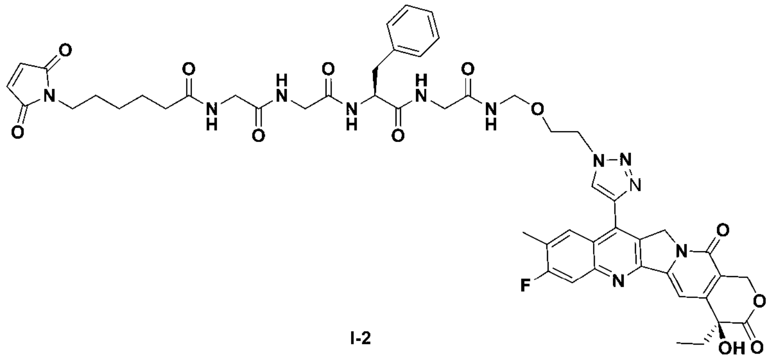

I-2

**I-2:** $^1$H NMR (400 MHz, DMSO) δ 8.70 (t, J = 6.6 Hz, 1H), 8.26 (t, J = 5.6 Hz, 1H), 8.13 - 8.10 (m, 2H), 8.06 (t, J = 5.7 Hz, 1H), 8.02 (s, 1H), 7.92(t, J = 5.7 Hz, 1H), 7.71 (d, J = 7.3 Hz, 1H), 7.36 (s, 1H), 7.31 - 7.25 (m, 2H), 7.25 - 7.16 (m, 3H), 6.92 (s, 2H), 5.44 (s, 2H), 5.24 (s, 2H), 4.65 - 4.59 (m, 2H), 4.56 - 4.34 (m, 3H), 4.23 - 4.18 (m, 2H), 3.88 (t, J = 5.2 Hz, 2H), 3.78 - 3.59 (m, 4H), 3.36 (t, J = 7.1 Hz, 2H), 3.17 - 2.88 (m, 2H), 2.45 (s, 3H), 2.14 (t, J = 7.5 Hz, 2H), 1.91 - 1.75 (m, 2H), 1.64 - 1.42 (m, 4H), 1.32 - 1.05 (m, 2H), 0.89 (t, J = 7.3 Hz, 3H). MS (ESI) m/z =1032.4 (M + H$^+$).

Example 4: Synthesis of Compounds II-1 and II-2

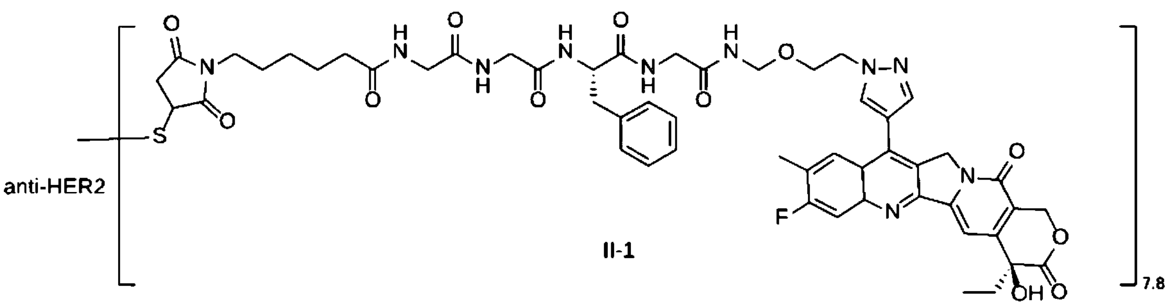

II-1

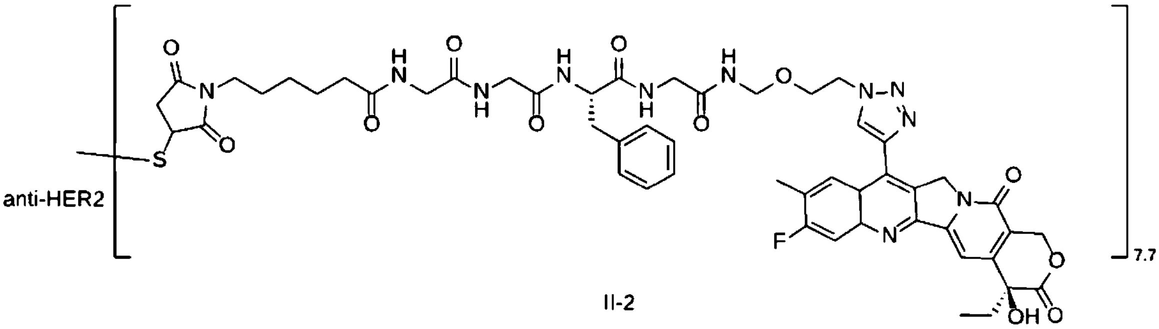

II-2

The HER2 antibody is trastuzumab.

At 37°C, a prepared aqueous solution of tris(2-carboxyethyl) phosphine (TCEP) (10 mM, 37.2 μL, 372 nmol) was added to a PBS buffer aqueous solution (0.05 M PBS buffer aqueous solution, pH = 6.5; 10.0 mg/mL, 1.0 mL, 67.6 nmol) of antibody Trastuzumab (HER2 antibody, CAS: 180288-69-1, purchased from Hangzhou Haoyang Biotechnology Co., Ltd.) . The mixture was placed in a water bath shaker, reacted with shaking at 37°C for 3 h, and then the reaction was stopped. The reaction solution was cooled to 25°C in a water bath. Compound I-1 or I-2 (1014 nmol) was dissolved in 50 μL DMSO, added to the above reaction solution, placed in a water bath shaker, reacted with shaking at 25°C for 3 h, and then the reaction was stopped. The reaction solution was desalted and purified by a Sephadex G25 gel column (mobile phase: 0.05 M PBS buffer aqueous solution, pH = 6.5, containing 0.001 M EDTA) to obtain a PBS buffer solution of conjugates II-1 and II-2 (1.1 mg/mL, 7.5 mL), which was stored at 4°C.

Average drug loading calculated by RP-HPLC: II-1, t = 7.8; II-2, t = 7.7.

Example 5: Preparation of Compound I-3

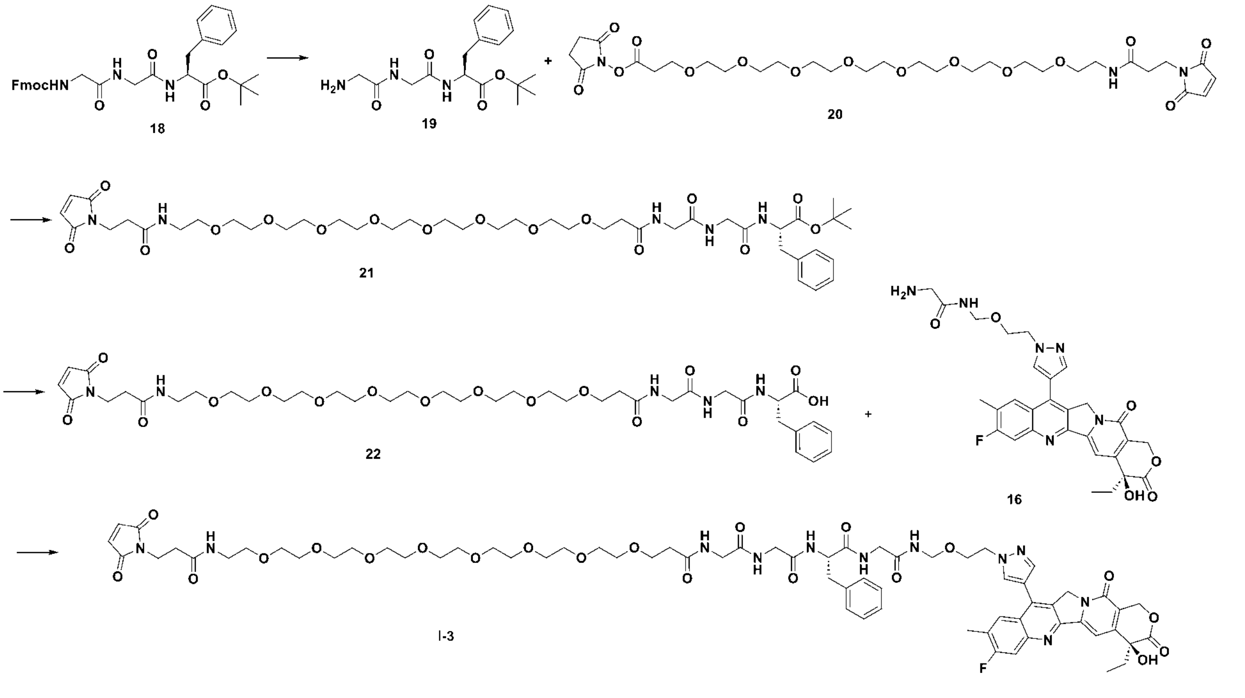

18 19 20 21 22 16 I-3

Step 1: Preparation of Compound 19

Under nitrogen, Compound 18 (1.2 g, CAS: 236426-37-2, purchased from GL Biochem Co., Ltd.) was dissolved in anhydrous tetrahydrofuran (10 mL), diethylamine (3 mL) was added at room temperature, and the mixture was reacted for 2 h. The reaction was monitored by TLC. After TLC indicated that no starting material remained, the reaction was terminated. The reaction solution was directly concentrated to dryness, then dissolved in dichloromethane and concentrated to dryness to obtain crude Compound 19 as a viscous liquid (0.7 g, yield 96.9%). MS (ESI) m/z = 336.2 (M + $H^+$).

Step 2: Preparation of Compound 21

Under nitrogen, Compound 19 (0.7 g) was dissolved in acetonitrile (10 mL), Compound 20 (1.4 g, CAS: 756525-93-6, purchased from Zhejiang Ruiao Biotechnology Co., Ltd.) was added at room temperature, the mixture was heated to 55°C and reacted overnight. Saturated sodium bicarbonate solution, the reaction solution was extracted with ethyl acetate, washed with saturated sodium chloride solution, dried over anhydrous sodium sulfate, concentrated under reduced pressure, and the residue was purified by column chromatography to obtain Compound 21 as a colorless liquid (1.3 g, yield 68.4%); MS (ESI) m/z = 910.5 (M + $H^+$).

Step 3: Preparation of Compound 22

Under nitrogen, Compound 21 (1.3 g) was dissolved in dichloromethane (20 mL), trifluoroacetic acid (5 mL) was added at room temperature, the mixture was heated to 35°C and reacted overnight. The reaction was monitored by TLC. After TLC indicated that no starting material remained, the reaction was terminated. The reaction solution was directly concentrated to dryness, the solvent was co-evaporated with dichloromethane, concentrated under reduced pressure, and purified by column chromatography to obtain Compound 22 as a colorless oil (0.8 g, yield 66.1%).

$^1$H NMR (400 MHz, DMSO) δ 8.17 (t, J = 5.6 Hz, 1H), 8.12 (d, J = 8.0 Hz, 1H), 8.01 (q, J = 5.6 Hz, 2H), 7.31 - 7.25 (m, 2H), 7.22 (dd, J = 7.5, 3.0 Hz, 3H), 7.00 (s, 2H), 5.75 (s, 2H), 4.49 - 4.34 (m, 1H), 3.79 - 3.65 (m, 5H), 3.61 (dt, J = 14.5, 5.6 Hz, 5H), 3.56 - 3.44 (m, 25H), 3.37 (t, J = 5.8 Hz, 2H), 3.17 - 3.12 (m, 2H), 3.06 (dd, J = 13.8, 5.0 Hz, 1H), 2.90 (dd, J = 13.7, 9.0 Hz, 1H), 2.40 (t, J = 6.5 Hz, 2H), 2.34 (t, J = 7.3 Hz, 2H). MS (ESI) m/z =854.4 (M + $H^+$).

Step 4: Preparation of Compound I-3

Under nitrogen, Compound 22 (100 mg) was dissolved in anhydrous tetrahydrofuran (15 mL), 1-hydroxybenzotriazole (HOBT) (20 mg) and 1-(3-dimethylaminopropyl)-3-ethylcarbodiimide hydrochloride (EDCI) (28 mg) were added at room temperature, and the mixture was reacted at room temperature for 2 h. After the reaction solution became clear, Compound 16 (70 mg) was added at room temperature and reacted at room temperature overnight. The reaction was monitored by LC-MS. After starting material Compound 16 had been completely reacted, the reaction solution was concentrated under reduced pressure, and the residue was purified by column chromatography to obtain Compound I-3 as a light yellow solid (35 mg, yield 50%).

$^1$H NMR (400 MHz, DMSO) δ 8.59 (t, J = 6.4 Hz, 1H), 8.46 (s, 1H), 8.32 (d, J = 6.2 Hz, 1H), 8.14 (dd, J = 22.1, 9.0 Hz, 3H), 8.00 (s, 2H), 7.93 (d, J = 10.7 Hz, 1H), 7.35 (s, 1H), 7.19 (dt, J = 17.3, 8.8 Hz, 5H), 7.00 (s, 2H), 6.54 (s, 1H), 5.43 (s, 2H), 5.31 (s, 2H), 4.62 (d, J = 6.6 Hz, 2H), 4.55 - 4.39 (m, 3H), 3.89 (t, J = 5.2 Hz, 2H), 3.83 - 3.66 (m, 4H), 3.59 (t, J = 6.4 Hz, 4H), 3.55 - 3.42 (m, 21H), 3.42 - 3.22 (m, 5H), 3.15 (dd, J = 11.7, 6.0 Hz, 2H), 3.07 - 2.92 (m, 2H), 2.83 - 2.71 (m, 2H), 2.38 (t, J = 6.5 Hz, 2H), 2.33 (t, J = 7.3 Hz, 2H), 2.03 (dd, J = 20.8, 13.2 Hz, 2H), 1.87 (dd, J = 14.6, 7.1 Hz, 3H), 1.28 (d, J = 28.2 Hz, 5H), 0.88 (dd, J = 12.7, 5.4 Hz, 3H). MS (ESI) m/z = 1412.6 (M + $H^+$).

Example 6: Preparation of Compound I-4

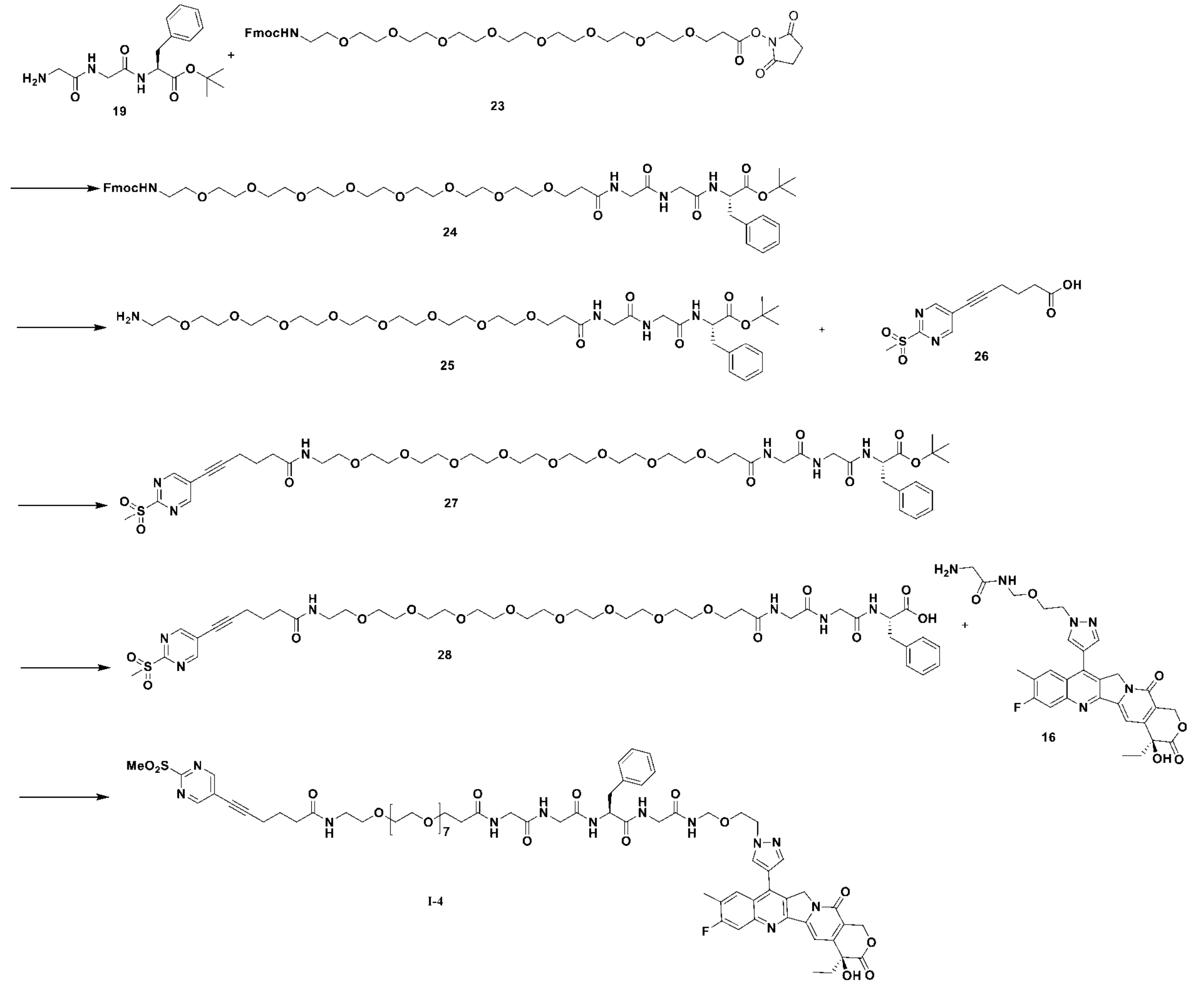


Step 1: Preparation of Compound 24

Under nitrogen, Compound 19 (300 mg) was dissolved in acetonitrile (10 mL), Compound 23 (820 mg, CAS: 1334170-03-4, purchased from Shanghai Bide Pharmaceutical Technology Co., Ltd.) was added at room temperature, the mixture was heated to 45°C and reacted overnight. Saturated sodium bicarbonate solution, the reaction solution was extracted with ethyl acetate, washed with saturated sodium chloride solution, dried over anhydrous sodium sulfate, concentrated under reduced pressure, and the residue was purified by column chromatography to obtain Compound 24 as a colorless oil (600 mg, yield 68.4%); MS (ESI) m/z = 981.5 (M + $H^+$).

Step 2: Preparation of Compound 25

Under nitrogen, Compound 24 (600 mg) was dissolved in anhydrous tetrahydrofuran (10 mL), 1,8-diazabicyclo [5.4.0]undec-7-ene (DBU) (100 mg) was added at room temperature, and the mixture was reacted for 2 h. The reaction was monitored by TLC. After TLC indicated that no starting material remained, the reaction was stopped. The reaction solution was directly concentrated to dryness, and the solvent was co-evaporated with dichloromethane, and concentrated to dryness to obtain crude Compound 25 as a colorless oil (420 mg, yield 91.3%); MS (ESI) m/z = 759.4 (M + $H^+$).

Step 3: Preparation of Compound 27

Under nitrogen, Compound 25 (400 mg) was dissolved in anhydrous tetrahydrofuran (20 mL), 1-hydroxybenzotriazole (HOBT) (100 mg), 1-(3-dimethylaminopropyl)-3-ethylcarbodiimide hydrochloride (EDCI) (140 mg) and Compound 26 (170 mg, CAS: 2356229-58-6, Shanghai Xiyao Pharmaceutical Technology Co., Ltd.) were added at room temperature, the mixture was reacted at room temperature overnight. The reaction was monitored by LC-MS. After starting material Compound 25 had been completely reacted, the reaction solution was concentrated under reduced pressure, and

the residue was purified by column chromatography to obtain Compound 27 as a colorless oil (450 mg, yield 84.9%). MS (ESI) m/z = 1009.5 (M + $H^+$).

Step 4: Preparation of Compound 28

Under nitrogen, Compound 27 (400 mg) was dissolved in dichloromethane (10 mL), trifluoroacetic acid (1.5 mL) was added at room temperature, the mixture was reacted at room temperature overnight. The reaction was monitored by LCMS. After LCMS indicated no starting material remained, the reaction was terminated. The reaction solution was directly concentrated to dryness, and the residue was subsequently triturated with tert-butyl methyl ether and filtered under reduced pressure to obtain Compound 28 as a light yellow solid (350 mg, yield 92.8%).

$^1$H NMR (400 MHz, DMSO) δ 9.11 (s, 2H), 8.17 (t, J = 5.0 Hz, 1H), 8.12 (d, J = 8.0 Hz, 1H), 8.01 (q, J = 5.5 Hz, 1H), 7.94 (t, J = 5.5 Hz, 1H), 7.27 (dd, J = 10.0, 4.6 Hz, 2H), 7.21 (dd, J = 11.8, 6.3 Hz, 3H), 5.75 (s, 2H), 4.45 (ddd, J = 13.5, 12.6, 6.7 Hz, 2H), 3.72 (t, J = 6.3 Hz, 3H), 3.64 - 3.59 (m, 3H), 3.56 - 3.45 (m, 30H), 3.44 - 3.39 (m, 5H), 3.22 (dd, J = 11.5, 5.8 Hz, 2H), 3.10 - 2.98 (m, 1H), 2.97 - 2.84 (m, 1H), 2.39 (dd, J = 13.4, 6.9 Hz, 2H), 2.28 (t, J = 7.3 Hz, 2H). MS (ESI) m/z = 953.4(M + $H^+$).

Step 5: Preparation of Compound I-4

Under nitrogen, Compound 16 (60 mg) was dissolved in anhydrous tetrahydrofuran (10 mL), 1-hydroxybenzotriazole (HOBT) (17 mg) was added at room temperature and the mixture was reacted for 2 h. 1-(3-Dimethylaminopropyl)-3-ethylcarbodiimide hydrochloride (EDCI) (24 mg) and Compound 28 (100 mg) were added at room temperature, and the mixture was reacted at room temperature overnight. The reaction was monitored by LC-MS. After LC-MS indicated that starting material Compound 16 had been completely reacted, the reaction solution was concentrated under reduced pressure, and the residue was purified by column chromatography to obtain Compound I-4 as a light yellow solid (30 mg, yield 19.1%).

$^1$H NMR (400 MHz, DMSO) δ 9.64 (s, 1H), 9.12 (s, 2H), 8.61 (t, J = 6.6 Hz, 1H), 8.46 (s, 1H), 8.34 (t, J = 5.7 Hz, 1H), 8.22 - 8.11 (m, 3H), 8.10 (s, 1H), 8.02 (t, J = 5.5 Hz, 1H), 7.93 (dd, J = 12.1, 8.0 Hz, 2H), 7.34 (s, 1H), 7.26 - 7.09 (m, 5H), 6.54 (s, 1H), 5.43 (s, 2H), 5.29 (s, 2H), 4.63 (d, J = 6.6 Hz, 2H), 4.47 (t, J = 10.6 Hz, 3H), 3.90 (t, J = 5.2 Hz, 2H), 3.82 - 3.65 (m, 5H), 3.64 - 3.54 (m, 4H), 3.52 - 3.44 (m, 25H), 3.33 (s, 4H), 3.28 - 3.16 (m, 3H), 3.02 (dd, J = 13.7, 4.4 Hz, 1H), 2.78 (dd, J = 13.8, 9.8 Hz, 1H), 2.55 (t, J = 7.1 Hz, 2H), 2.48 (s, 3H), 2.39 (t, J = 6.5 Hz, 2H), 2.27 (t, J = 7.3 Hz, 2H), 1.97 - 1.75 (m, 5H), 1.25 (d, J = 11.0 Hz, 1H), 0.89 (t, J = 7.3 Hz, 3H). MS (ESI) m/z = 1511.6 (M + $H^+$).

Example 7: Preparation of Compounds II-3 and II-4

According to Example 4, Compound I-1 was replaced with Compounds I-3 and I-4 respectively, and the conjugates II-3 (t = 7.8) and II-4 (t = 7.7) were prepared.

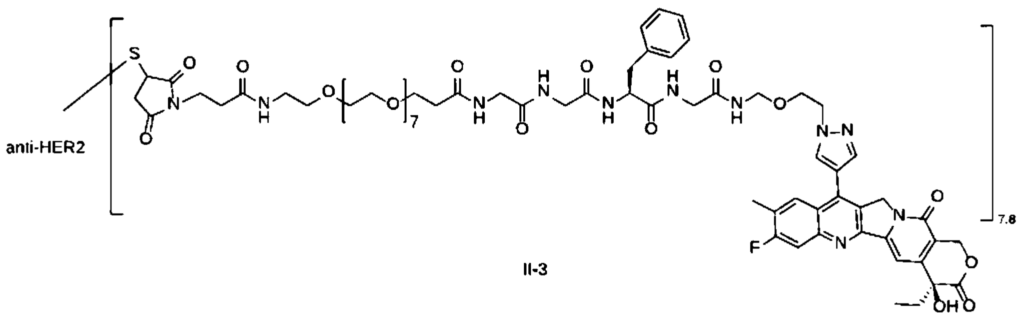

II-3

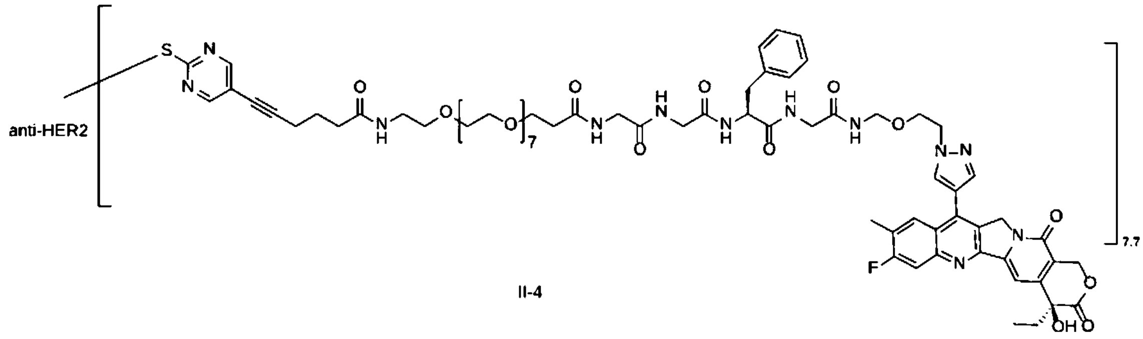

II-4

Effect Example 1: Effect of camptothecin derivatives K1-K9 on the proliferation ability of tumor cells

Test method for the effect of camptothecin on the proliferation ability of tumor cells:
The effect of camptothecin derivatives K1, K2, K3, K4, K5, K6, K7, K8, and K9 on the proliferation ability of tumor cells was tested using the CTG assay. Test cells (see Table 1) were digested, resuspended, and counted. The supernatant was removed by centrifugation, and the cell density was adjusted. Cell counting was performed with a cell counter: 20 μl of the cell suspension was aspirated, mixed with 20 μl of trypan blue, and 20 μl of the mixture was taken for cell counting. Cells were seeded into 96-well cell culture plates respectively, with 180 μl added to each well, and 1000 cells were plated per well. The plate was tapped gently to mix, and then placed in a 37°C cell incubator for incubation for 16 hours. Compounds were diluted with McCoy's 5A medium, and 20 μl was added to each well to achieve a maximum concentration of 100 nM, followed by 3-fold serial dilution with 10 concentration points. DMSO was used as the vehicle control, and 20 μl of McCoy's 5A medium was added to the blank control. The plate was tapped gently to mix, and then placed in a 37°C cell incubator for incubation for 7 days. After 7 days of incubation, the cell plate was taken out of the incubator, the culture medium was aspirated, 20 μl of culture medium was added to each well, and then 20 μl of CTG was added to each well. The plate was shaken on a shaker for 5 min and incubated at room temperature for 10 min. Then, 30 μl of the reaction solution was transferred to a Viewplate-96 white microplate (transparent bottom), and data analysis was performed on an EnVision microplate reader.

Calculation formula: Background value = Control group - Blank group; Inhibition rate = 1 - (Experimental group - Blank group) / (Background value) × 100%.

Table 1. Overview of cell lines used in this screening

| Cell code | Cell line name | Culture medium | Source |
|---|---|---|---|
| NCI-N87 | human gastric cancer cells | 1640+10%FBS | ATCC(CRL-5882) |
| Calu-3 | human lung adenocarcinoma cells | MEM+10%FBS | ATCC(HTB-55) |
| HCT-116 | human colon cancer cells | McCoy's5A+10%FBS | ATCC(CCL-247) |

Table 2. Effect of compounds on the proliferation ability of tumor cells

| Compound | $IC_{50}$ (nM) | | |
|---|---|---|---|
| | NCI-N87 | Calu-3 | HCT-116 |
| K1 | 1.89 | 0.22 | 0.77 |
| K2 | 2.21 | 0.29 | 0.89 |
| K3 | 3.11 | 0.43 | 1.36 |
| K4 | 1.72 | 0.15 | 0.42 |
| K5 | 2.07 | 0.18 | 0.35 |
| K6 | 2.88 | 0.28 | 0.78 |
| K7 | 5.34 | 1.13 | 2.24 |
| K8 | 6.76 | 1.24 | 3.21 |
| K9 | 7.82 | 1.89 | 3.87 |

As shown in Table 2, the compounds of the present invention exhibit excellent in vitro cellular activity.

Effect Example 2: Effect of antibody-drug conjugates on the proliferation ability of tumor cells

Test method for the effect of Compounds II-1, II-2, II-3 and II-4 on the proliferation ability of tumor cells: NCI-N87 cells were digested, resuspended, and counted; the supernatant was removed by centrifugation, and the cell density was adjusted. Cell counting was performed with a cell counter: 20 μL of the cell suspension was aspirated, mixed with 20 μL of VisStain AOPI Staining Solution, and 20 μL of the mixture was taken for cell counting. Cells were seeded into 96-well cell culture plates respectively, with 180 μL added to each well, and 1000 cells or 2000 cells were plated per well. The plate was tapped gently to mix, and then placed in a 37°C cell incubator for incubation for 16 hours. Compounds were diluted with RPMI1640 medium/10%FBS, and 20 μL was added to each well to achieve a maximum concentration of 100 nM, followed by 3-fold serial dilution with 10 concentration points. DMSO was used as the vehicle control, and 20 μL of RPMI1640 medium/10%FBS was added to the blank control. The plate was tapped gently to mix, and then placed in a 37°C cell incubator for incubation for 7 days. After 7 days of incubation, the cell plate was taken out of the incubator, the culture medium was aspirated, 20 μL of culture medium was added to each well, and then 20 μL of CTG was added to each well. The plate was shaken on a shaker for 5 min and incubated at room temperature for 10 min. Then, 30 μL of the reaction solution was transferred to a Viewplate-96 white microplate (transparent bottom), and data analysis was performed on an EnVision microplate reader.

Calculation formula: The inhibition rate (IR) of the tested antibody was calculated using the following formula: IR (%) = (1 - (Antibody - Blank control) / (Vehicle control - Blank control)) × 100%. In Excel, the inhibition rates of the antibody at different concentrations were calculated, and then GraphPad Prism software was used to calculate the relevant parameters, with the results shown in Table 3.

Table 3. Effect of compounds on the proliferation ability of NCI-N87 tumor cells

| **Antibody-drug conjugate** | **$IC_{50}$ (μg/mL)** |
|---|---|
| II-1 | 0.0115 |
| II-2 | 0.0187 |
| II-3 | 0.0261 |
| II-4 | 0.0238 |

Effect Example 3: Bystander effect of antibody-drug conjugate II-2 and T-Dxd on NCI-N87 and MDA-MB-231 cells

In this experiment, II-2 and T-Dxd were co-cultured with NCI-N87/MDA-MB-231 cells for 7 days, and flow cytometry was used to detect the proportion of NCI-N87 or MDA-MB-231 cells in the total particles, which reflects the ability of ADC to kill negative cells via the bystander effect.

NCI-N87 cells were cultured in one T175 flask, digested, resuspended in 8 mL for cell counting, centrifuged to remove the supernatant, and the cell density was adjusted. MDA-MB-231 (ATCC, HTB-26) cells were cultured in one T175 flask, digested, resuspended in 6 mL for cell counting, centrifuged to remove the supernatant, and the cell density was adjusted. Cell counting was performed with a cell counter: 20 μL of the cell suspension was aspirated, mixed with 20 μL of VisStain AOPI Staining Solution, and 20 μL of the mixture was taken for cell counting. The cells were seeded into 96-well cell culture plates (flat-bottomed) with RPMI1640 medium/10%FBS respectively, and 90 μL of each cell was added to each well, i.e., the cell ratio was 10000 cells/well of NCI-N87 + 2000 cells/well of MDA-MB-231 (5:1). The plate was tapped gently to mix, and then placed in a 37°C 5% CO2 cell incubator for incubation for 16 hours. For antibody dilution, the antibodies were first diluted with RPMI1640 medium/10%FBS to a stock concentration of 10 μg/mL, 20 μL was added to each well, and 20 μL of RPMI1640 medium/10%FBS was added to the blank control. The plate was tapped gently to mix, and then placed in a 37°C 5% CO2 cell incubator for incubation for 7 days. After 7 days of incubation, the cell plate was taken out of the incubator, the culture medium was aspirated, 100 μL of trypsin was added, and incubated in a 37°C cell incubator for 15 minutes. 100 μL of RPMI1640 medium/10%FBS was added, and the cells were pipetted up and down to detach from the bottom of the wells, then transferred to a 96-well cell culture plate (V-bottomed). Centrifugation was performed at 350g for 5 minutes to remove the supernatant. Trastuzumab was prepared at a concentration of 1 μg/mL with PBS containing 2% FBS, 100 μL was added to each tube, and incubated at 4°C for 1 hour. Centrifugation was performed at 350g for 5 minutes to remove the supernatant. The secondary antibody PE-labeled human IgG was diluted 1:2000 with PBS containing 2% FBS, 100 μL was added to each well, and incubated at 4°C for 30 minutes. Centrifugation was performed at 350g for 5 minutes to remove the supernatant. The cells were resuspended in 100 μL of PBS containing 2% FBS for analysis, and the results are shown in Table 4 below.

Calculation formula: Percentage of negative cells = Number of negative cell particles / Total number of cell particles × 100%; Percentage of positive cells = Number of positive cell particles / Total number of cell particles × 100%.

Table 4

| NCI-N87: MDA-MB-231 seeding ratio | drug name | Percentage of positive and negative cells (%) | |
|---|---|---|---|
| | | NCI-N87 | MDA-MB-231 |
| 5:1 | blank control | 31.88 | 11.22 |
| | T-Dxd | 9.79 | 4.74 |
| | II-1 | 9.63 | 3.54 |

Compared with the negative control antibody, as shown in the above table, Compound II-1 and T-Dxd induced a significant decrease in the number of both positive and negative cell populations (i.e., NCI-N87 and MDA-MB-231 cells respectively), and the effect of II-1 is superior to that of T-Dxd.

Effect Example 4: Antitumor efficacy test of II-1 and II-2 on the NOG mouse model with subcutaneously implanted NCI-N87 cells

This experiment evaluates the antitumor activity of II-1 and II-2 by subcutaneously inoculating NOG mice with NCI-N87 cells.

NOG mice: Female NOG mice (6 weeks old) were purchased from Beijing Vital River Laboratory Animal Technology Co., Ltd. The mice were acclimatized for 7 days after arrival, followed by the initiation of the study.

Cells: Human gastric cancer NCI-N87 cells (from ATCC, catalog number CRL-5822) were subjected to routine subculture in accordance with the manufacturer's instructions; the cells were resuspended in serum-free medium and the cell density was adjusted, and on Day 0, the cell suspension (inoculum: $1 \times 10^7$) was subcutaneously inoculated into the right axillary region of female NOG mice to establish the NCI-N87 tumor-bearing mouse model.

Administration:

On Day 10 after tumor cell inoculation, the tumor volumes of each mouse were measured. Mice with tumor volumes ranging from 110 $mm^3$ to 250 $mm^3$ were selected and evenly grouped by tumor volume (5 mice per group), and administration was performed on Day 11 after inoculation with the negative control antibody (human IgG Control, source: Hangzhou Haoyang Biotechnology Co., Ltd., catalog number: HSP067-F1), positive control antibody Trastuzumab (HER2 antibody, CAS: 180288-69-1, purchased from Hangzhou Haoyang Biotechnology Co., Ltd.), II-1 and II-2 respectively. During the administration period, changes in tumor volume and body weight of mice in each group were monitored with a frequency of twice a week for 3 consecutive weeks, and body weight and tumor volume were measured before each administration. The tumor growth inhibition rate (TGI%) was calculated on Day 23 after the first administration, with the calculation formula as follows: TGI (%) = $[1 - (Ti - T0)/(Vi - V0)] \times 100$, wherein Ti: mean tumor volume of the administration group, T0: mean tumor volume of the administration group on Day 0, Vi: mean tumor volume of the isotype control group, V0: mean tumor volume of the isotype control group on Day 0. Tumor volume measurement: The long diameter (a) and short diameter (b) of the tumor were measured with a vernier caliper, and the tumor volume was calculated according to the following formula: $TV = 1/2 \times a \times b^2$. Body weight was measured with an electronic balance.

The dosage and administration route are shown in Table 5 below.

Table 5: Administration Experiment Design

| group | dosage | administration frequency | administration route |
|---|---|---|---|
| negative control antibody | 4 mg/kg | single administration | intravenous injection |
| positive control antibody | 4 mg/kg | single administration | intravenous injection |
| II-1 | 4 mg/kg | single administration | intravenous injection |
| II-2 | 4 mg/kg | single administration | intravenous injection |

As shown in Table 6 and Figure 1, on Day 23 after the first administration, II-1 and II-2 significantly inhibited the

growth of tumors at a dosage of 4 mg/kg, with tumor growth inhibition rates (TGI%) of 116.3% and 115.9%, respectively.

Table 6: In vivo tumor growth inhibition ability of antibody-drug conjugates on day 23

| group | observation days | | | | | | | | inhibition rate of tumor volume | P value |
|---|---|---|---|---|---|---|---|---|---|---|
| | 0 | 3 | 7 | 10 | 14 | 17 | 21 | 23 | | |
| | tumor volume (mean ± standard error) | | | | | | | | | |
| negative control antibody | 136.8 ±21.3 | 332.3 ±51.7 | 423.5 ±49.0 | 484.1 ±35.8 | 586.0 ±71.2 | 616.9 ±83.4 | 763.1 ±66.5 | 851.5 ±94.9 | / | / |
| positive control antibody | 139.2 ±22.8 | 301.1 ±62.8 | 322.6±5 9.4 | 380.5±1 14.3 | 487.1±1 00.8 | 579.4±1 76.4 | 714.2±1 29.5 | 752.3±2 30.9 | 14.22% | 0.78 |
| 11-1 | 137.7 ±22.6 | 227.7 ±65.2 | 82.3±25 .8 | 65.7±22 .8 | 40.1±13 .8 | 25.4±9. 4 | 31.8±11. 1 | 20.9±6. 3 | 116.3% | <0.001 |
| 11-2 | 138.2 ±20.6 | 266.9 ±48.3 | 96.5±30 .1 | 72.1±10 .1 | 46.6±5. 4 | 31.2±8. 5 | 39.5±5. 8 | 24.0±3. 6 | 115.9% | <0.001 |

Note: P value is compared with the negative control antibody.

These results indicate that II-1 and 11-2 significantly inhibited tumor growth and had no significant effect on the body weight of NOG mice (Figure 2).

Effect Example 5: Antitumor efficacy test of II-1 and II-2 on the animal model of subcutaneously transplanted JIMT-1 cells in NOG mice

This experiment was conducted to determine the antitumor effects of II-1 and 11-2 by subcutaneously inoculating JIMT-1 cells into NOG mice.

NOG mice: Female NOG mice (6 weeks old) were purchased from Beijing Vital River Laboratory Animal Technology Co., Ltd.The mice were acclimatized for 7 days after arrival, followed by the initiation of the study.

Cells: Human breast cancer JIMT-1 cells (Shanghai Yubo Biotechnology Co., Ltd., catalog number: YBC71) were routinely subcultured according to the manufacturer's instructions. The cells were resuspended in serum-free medium and the cell density was adjusted. On Day 0, the cell suspension (inoculum: $3 \times 10^6$) was subcutaneously inoculated into the right axillary region of female NOG mice to establish the JIMT-1 tumor-bearing mouse model.

Administration:

On Day 10 after tumor cell inoculation, the tumor volume of each mouse was measured. Mice with tumor volumes ranging from 110 $mm^3$ to 250 $mm^3$ were selected and evenly grouped by tumor volume (5 mice per group). Administration was performed once each on Day 11 and Day 18 after inoculation. The group were: the negative control antibody (Human IgG Control, Source: Hangzhou Haoyang Biotechnology Co., Ltd., Catalog Number: HSP067-F1), II-1 and II-2. During the administration period, the tumor volume and body weight changes of mice in each group were monitored. The monitoring frequency was twice a week for 4 consecutive weeks. Body weight and tumor volume were measured before each administration. On Day 28 after the first administration the tumor growth inhibition rate (TGI%) was calculated using the following formula TGI (%) = $[1 - (Ti - T0)/(Vi - V0)] \times 100$, wherein Ti: mean tumor volume of the administration group, T0: mean tumor volume of the administration group on Day 0, Vi: mean tumor volume of the isotype control group, V0: mean tumor volume of the isotype control group on Day 0. Tumor volume measurement: The long diameter (a) and short diameter (b) of tumors were measured using a vernier caliper. Tumor volume was calculated using the following formula: $TV = 1/2 \times a \times b^2$. Body weight was measured using an electronic balance.

Dosage and administration route are as shown in Table 7.

Table 7: Administration Experiment Design

| group | administration dose | administration frequency | administration route |
|---|---|---|---|
| negative control antibody | 6 mg/kg | twice (once on Day 1 and once on Day 8) | intravenous injection |
| II-1 | 6 mg/kg | twice (once on Day 1 and once on Day 8) | intravenous injection |
| II-2 | 6 mg/kg | twice (once on Day 1 and once on Day 8) | intravenous injection |

As shown in Table 8 and Figure 3, on Day 28 after the first administration, II-1 and II-2 significantly inhibited tumor growth at a dosage of 6 mg/kg, with tumor growth inhibition rates of 122.4% and 120.9%, respectively.

Table 8: In vivo tumor growth inhibition ability of ADCs

| group | observation days | | | | | | | | | |
|---|---|---|---|---|---|---|---|---|---|---|
| | 0 | 3 | 8 | 11 | 15 | 18 | 22 | 25 | 29 | |
| | tumor volume (mean ± standard error) | | | | | | | | | P value |
| negative control antibody | 146.4 ±23.0 | 156.6 ±30.2 | 210.8 ±98.9 | 244.9± 123.1 | 285.5± 163.2 | 303.7± 180.1 | 316.7± 172.8 | 347.4± 198.1 | 359.2± 200.2 | / |
| II-1 | 146.5 ±21.3 | 165.1 ±97.3 | 143.8 ±67.5 | 152.5± 47.8 | 139.3± 41.2 | 116.0± 16.5 | 104.2± 16.1 | 105.6± 18.9 | 98.8± 28.1 | <0.001 |
| II-2 | 147.9 ±23.8 | 130.2 ±38.1 | 102.4 ±24.8 | 107.1± 20.4 | 98.5±1 8.2 | 102.9± 18.9 | 101.7± 23.4 | 107.6± 18.17 | 103.5 ±17.5 | <0.001 |
| Note: P value is compared with the negative control antibody. | | | | | | | | | | |

These results indicate that II-1 and II-2 significantly inhibited tumor growth in the animal model of subcutaneously transplanted JIMT-1 cells in NOG mice, and had no significant effect on the body weight of NOG mice (Figure 4).

## Claims

1. A camptothecin compound of formula K or a pharmaceutically acceptable salt thereof:

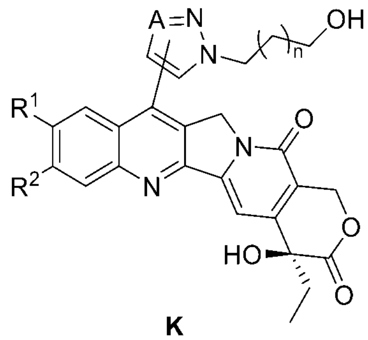

**K**

wherein, $R^1$ is $C_{1-6}$ alkyl;
$R^2$ is halogen;
A is CH or N;
n is 0, 1, 2 or 3.

2. The camptothecin compound of formula K or a pharmaceutically acceptable salt thereof according to claim 1, satisfying one or more of the following conditions:

(1) in $R^1$, the $C_{1-6}$ alkyl is methyl, ethyl, n-propyl, isopropyl, n-butyl, isobutyl, sec-butyl or tert-butyl; for example, methyl;
(2) in $R^2$, the halogen is fluorine, chlorine, bromine or iodine; for example, fluorine;
(3)

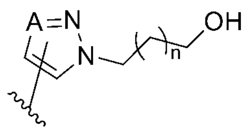

is

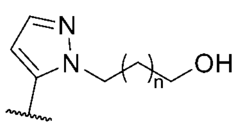

,

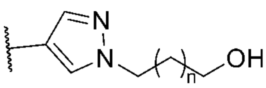

,

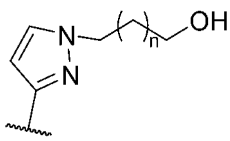

or

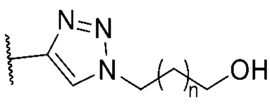

.

3. The camptothecin compound of formula K or a pharmaceutically acceptable salt thereof according to claim 1, satisfying one or more of the following conditions:

(1) $R^1$ is methyl and $R^2$ is fluorine, chlorine, bromine or iodine; for example, fluorine;
(2) n is 0, 1 or 2; preferably,

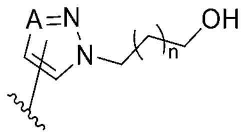

is

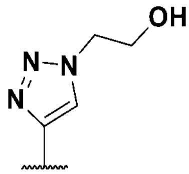 , 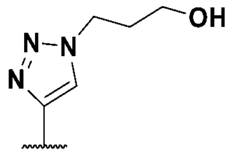 ,

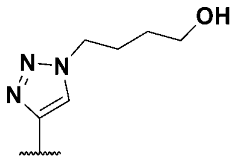 , 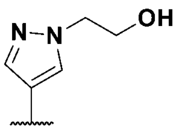 , 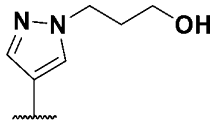 , 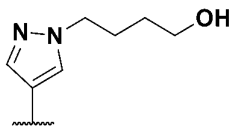 , 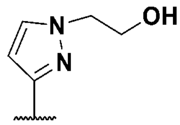 ,

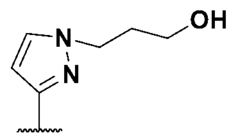 or 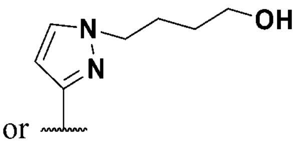 .

**4.** The camptothecin compound of formula K or a pharmaceutically acceptable salt thereof according to claim 1, wherein the camptothecin compound of formula K is a compound of formula K-1 or a compound of formula K-2;

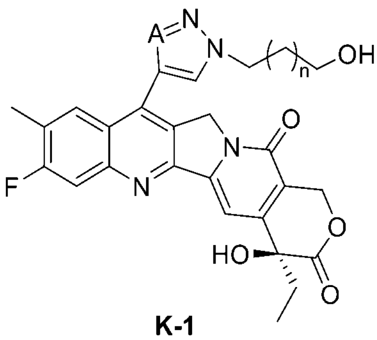

**K-1**

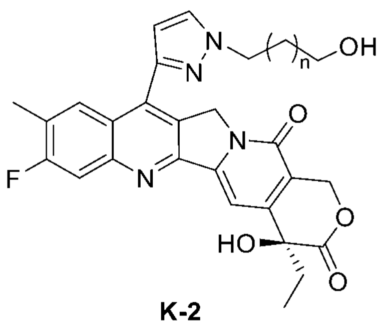

**K-2**

wherein A and n are each independently as described in any one of claims 1-3.

**5.** The camptothecin compound of formula K or a pharmaceutically acceptable salt thereof according to claim 1, wherein the camptothecin compound of formula K is selected from any one of the following structures:

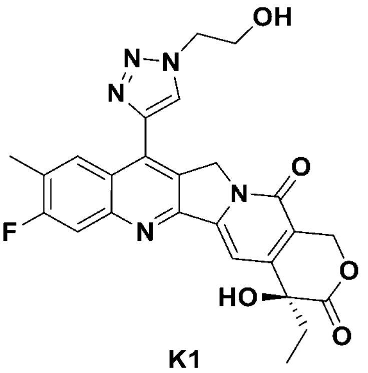
K1,
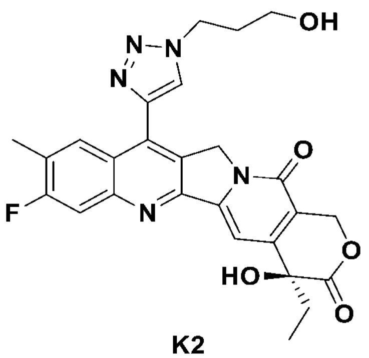
K2,
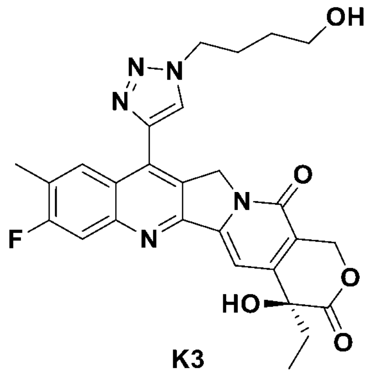
K3,

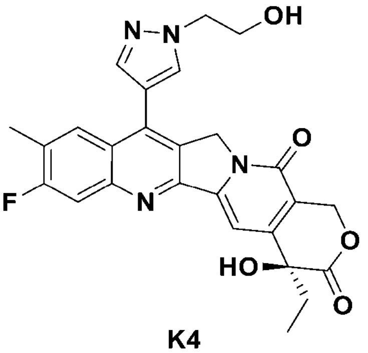
K4,
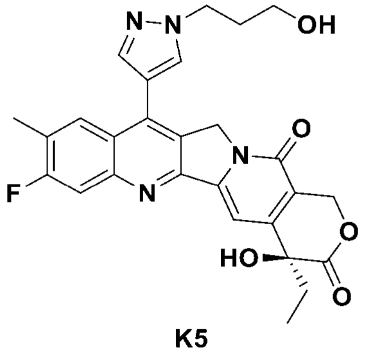
K5,
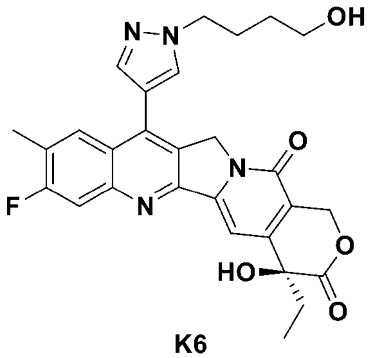
K6,

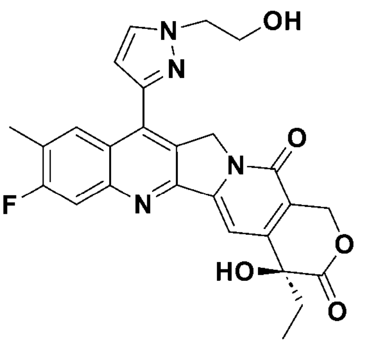
K7,
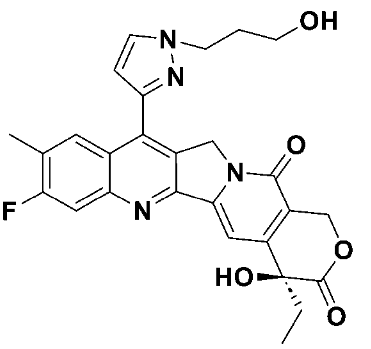
K8,
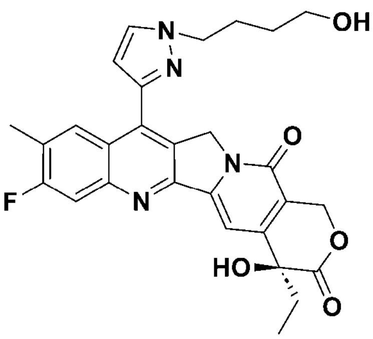
K9.

**6.** A linker-drug conjugate of formula I or a pharmaceutically acceptable salt thereof;

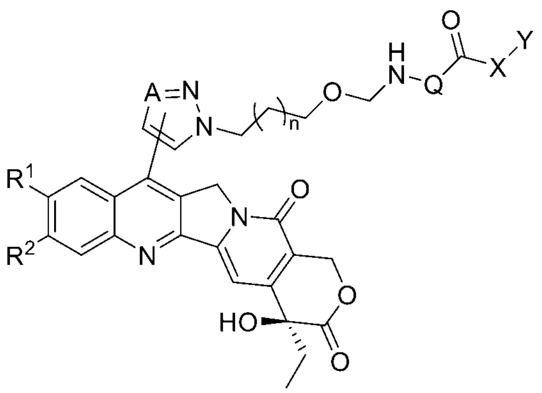

I

wherein, Q is a single amino acid residue, a dipeptide residue, a tripeptide residue, a tetrapeptide residue or a pentapeptide residue;

X is $-(CR^aR^b)_r$-CO-NH-$(CR^aR^b)_q$-$(OCH_2CH_2)_p$-, $-(OCH_2CH_2)_m$-, $-(CR^aR^b)_m$-, -O$(CR^aR^b)_m$-, -NH-$(CR^aR^b)_m$-$CR^cR^d$- or -S$(CR^aR^b)_m$-$CR^cR^d$-;

$R^a$ and $R^b$ are independently H, D, halogen, $C_{1-6}$ alkyl, halogenated $C_{1-6}$ alkyl, deuterated $C_{1-6}$ alkyl, $C_{1-6}$ alkoxy, hydroxyl, amino, cyano, nitro, 3-10-membered cycloalkyl or 3-10-membered heterocycloalkyl;

or, $R^a$ and $R^b$, together with the carbon atom to which they are attached, form a 3-10-membered cycloalkyl or a 3-10-membered heterocycloalkyl;

$R^c$ and $R^d$ are independently H, $C_{1-6}$ alkyl, halogen, halogenated $C_{1-6}$ alkyl, deuterated $C_{1-6}$ alkyl, 3-10-membered cycloalkyl, 3-10-membered heterocycloalkyl, 6-14-membered aryl or 5-10-membered heteroaryl;

or, $R^c$ and $R^d$, together with the carbon atom to which they are attached, form a 3-10-membered cycloalkyl or a 3-10-membered heterocycloalkyl;

the heteroatoms in each of the 3-10-membered heterocycloalkyl and 5-10-membered heteroaryl are independently selected from one, two or three of N, O or S, and the number of heteroatoms is independently 1, 2 or 3;

Y is

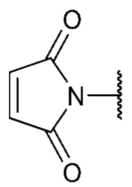

or

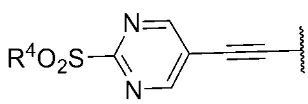

;

$R^4$ is $C_{1-6}$ alkyl or a phenyl group substituted with one or more $C_{1-6}$ alkyl groups;

m, p, q and r are independently an integer from 0 to 14;

the definitions of

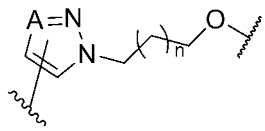

,

$R^1$, $R^2$, A and n are as described in any one of claims 1-5.

**7.** The linker-drug conjugate of formula I or a pharmaceutically acceptable salt thereof according to claim 6, wherein the linker-drug conjugate is any one of the following schemes:

scheme 1:

Q is a single amino acid residue, a dipeptide residue, a tripeptide residue or a tetrapeptide residue;

X is $-(CR^aR^b)_r$-CO-NH-$(CR^aR^b)_q$-$(OCH_2CH_2)_p$-, $-(OCH_2CH_2)_m$-, $-(CR^aR^b)_m$-, -O$(CR^aR^b)_m$-, -NH-$(CR^aR^b)_m$-$CR^cR^d$- or -S$(CR^aR^b)_m$-$CR^cR^d$-;

$R^a$ and $R^b$ are independently H, D, halogen, $C_{1-6}$ alkyl, halogenated $C_{1-6}$ alkyl, deuterated $C_{1-6}$ alkyl, $C_{1-6}$ alkoxy, hydroxy, amino, cyano, nitro, 3-10-membered cycloalkyl or 3-10-membered heterocycloalkyl;

or, $R^a$ and $R^b$, together with the carbon atom to which they are attached, form a 3-10-membered cycloalkyl or a 3-10-membered heterocycloalkyl;

$R^c$ and $R^d$ are independently H, $C_{1-6}$ alkyl, halogen, halogenated $C_{1-6}$ alkyl, deuterated $C_{1-6}$ alkyl, 3-10-membered cycloalkyl, 3-10-membered heterocycloalkyl, 6-14-membered aryl or 5-10-membered heteroaryl;

or, $R^c$ and $R^d$, together with the carbon atom to which they are attached, form a 3-10-membered cycloalkyl or a 3-10-membered heterocycloalkyl;

the heteroatoms in each of the 3-10-membered heterocycloalkyl and 5-10-membered heteroaryl are independently selected from one, two or three of N, O or S, and the number of the heteroatoms is independently 1, 2 or 3;

Y is

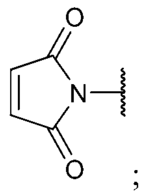

;

m, p, q and r are independently an integer from 0 to 14;
the definitions of $R^1$, $R^2$, A and n are as described in claim 6;

scheme 2:

Q is a tetrapeptide residue;
X is $-(CR^aR^b)_r$-CO-NH-$(CR^aR^b)_q$-$(OCH_2CH_2)_p$-* or $-(CR^aR^b)_m$-*, wherein "-*" terminal is linked to the carbonyl group;
$R^a$ and $R^b$ are H;
Y is

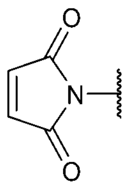 or 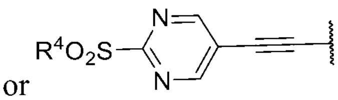 ;

$R^4$ is $C_{1-6}$ alkyl;
m is 5;
P is 8;
q and r are independently 2 or 3;
the definitions of

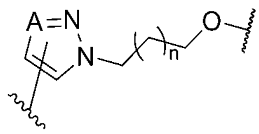

,

$R^1$, $R^2$, A and n are as described in claim 6;

scheme 3:

Q is a tetrapeptide residue;
X is-$(CR^aR^b)_m$-;
$R^a$ and $R^b$ are independently H;
Y is

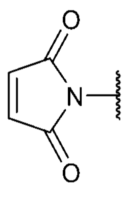

;

m is 5;

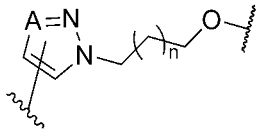

is

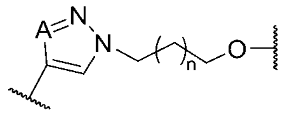

;

the definitions of $R^1$, $R^2$, A and n are as described in claim 6;

scheme 4:

$R^1$ is $C_{1-6}$ alkyl;
$R^2$ is halogen;
A is CH or N;
n is 0, 1, 2 or 3;

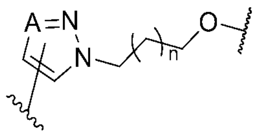

is

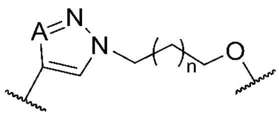

or

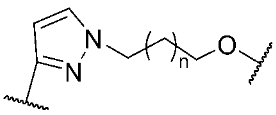

;

Q is a single amino acid residue, a dipeptide residue, a tripeptide residue or a tetrapeptide residue;
X is $-(CR^aR^b)_r$-CO-NH-$(CR^aR^b)_q$-$(OCH_2CH_2)_p$-* or $-(CR^aR^b)_m$-*, wherein "-*" terminal group is linked to the carbonyl group;
$R^a$ and $R^b$ are H;
Y is

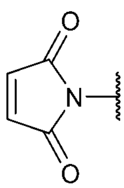

or

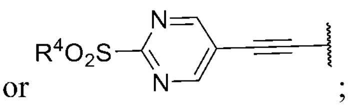

;

$R^4$ is $C_{1-6}$ alkyl;
m is an integer from 0 to 8;
p is an integer from 0 to 10;
q and r are independently an integer from 0 to 8;

scheme 5:

Q is a single amino acid residue, a dipeptide residue, a tripeptide residue, a tetrapeptide residue or a pentapeptide residue;
X is $-(CR^aRb)_r$-CO-NH-$(CR^aR^b)_q$-$(OCH_2CH_2)_p$-*, *-$(OCH_2CH_2)_m$-, $-(CR^aR^b)_m$-, *-O$(CR^aR^b)_m$-, *-NH-$(CR^aR^b)_m$-$CR^cR^d$- or *-S$(CR^aR^b)_m$-$CR^cR^d$-, wherein the * end is linked to the carbonyl group;

$R^a$ and $R^b$ are independently selected from H, D, halogen, C1-6 alkyl, halogenated C1-6 alkyl, deuterated $C_{1-6}$ alkyl, $C_{1-6}$ alkoxy, hydroxyl, amino, cyano, nitro, 3-10-membered cycloalkyl, or 3-10-membered heterocycloalkyl;

or, $R^a$ and $R^b$ together with the carbon atom to which they are attached form a 3-10-membered cycloalkyl or a 3-10-membered heterocycloalkyl;

$R^c$ and $R^d$ are independently H, $C_{1-6}$ alkyl, halogen, halogenated $C_{1-6}$ alkyl, deuterated $C_{1-6}$ alkyl, 3-10-membered cycloalkyl, 3-10-membered heterocycloalkyl, 6-14-membered aryl, or 5-10-membered heteroaryl;

or, $R^c$ and $R^d$ together with the carbon atom to which they are attached form a 3-10-membered cycloalkyl or a 3-10-membered heterocycloalkyl;

the heteroatoms in each 3-10-membered heterocycloalkyl and 5-10-membered heteroaryl are independently selected from one, two or three of N, O or S, and the number of the heteroatoms is independently 1, 2 or 3;

Y is

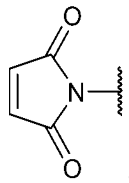 or 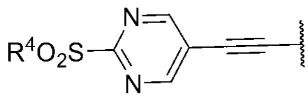 ;

$R^4$ is $C_{1-6}$ alkyl or phenyl substituted with one or more $C_{1-6}$ alkyl groups;

m, p, q and r are independently integers from 0 to 14;

the definitions of

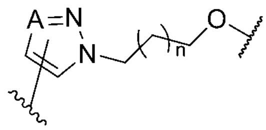 ,

$R^1$, $R^2$, A and n are as described in claim6.

**8.** The linker-drug conjugate of formula I or a pharmaceutically acceptable salt thereof according to claim 6 or 7, wherein the linker-drug conjugate satisfies one or more of the following conditions:

(1) each halogen is independently fluorine, chlorine, bromine or iodine;

(2) each $C_{1-6}$ alkyl is independently methyl, ethyl, n-propyl, isopropyl, n-butyl, isobutyl, sec-butyl or tert-butyl;

(3) each $C_{1-6}$ alkoxy is independently methoxy, ethoxy, n-propoxy, isopropoxy, n-butoxy, isobutoxy, sec-butoxy or tert-butoxy;

(4) each 3-10-membered cycloalkyl is independently cyclopropyl, cyclobutyl, cyclopentyl or cyclohexyl;

(5) each 3-10-membered heterocycloalkyl is independently a 3-6-membered heterocycloalkyl, and the heteroatoms of the 3-6-membered heterocycloalkyl are independently selected from one, two or three of N, O or S, with the number of heteroatoms independently being 1, 2 or 3;

(6) each 6-14-membered aryl is independently phenyl or naphthyl;

(7) each 5-10-membered heteroaryl is independently a 5-6-membered heteroaryl, and the heteroatoms of the 5-6-membered heteroaryl are independently selected from one, two or three of N, O or S, with the number of heteroatoms independently being 1, 2 or 3;

(8) in Q, the single amino acid residue is -Phe-, -Lys-, -Val-, -Ala-, -Cit-, -Leu-, -Ile-, -Arg-, -Trp- or -Gly-;

(9) in Q, the dipeptide residue is $^{C=O}$ -Lys-Phe- $^{NH}$, $^{C=O}$ -Ala-Val- $^{NH}$, $^{C=O}$ -Lys-Val- $^{NH}$, $^{C=O}$ - Lys- Ala- $^{NH}$, $^{C=O}$ -Cit-Val- $^{NH}$, $^{C=O}$ Cit-Phe- $^{NH}$, $^{C=O}$ -Cit-Leu- $^{NH}$, $^{C=O}$ Cit-Ile- $^{NH}$, $^{C=O}$ -Arg-Phe-$^{NH}$, $^{C=O}$ -Cit-Trp- $^{NH}$ or $^{C=O}$ -Val-Gly- $^{NH}$; for example, $^{C=O}$ -Ala-Val- $^{NH}$;

(10) in Q, the tripeptide residue is $^{C=O}$ -Ala-Val-Glu- $^{NH}$, $^{C=O}$ -Cit-Val-Glu- $^{NH}$, $^{C=O}$ - Ala-Val-$\alpha$Glu- $^{NH}$ or $^{C=O}$ -Cit-Val-aGlu- $^{NH}$; and

(11) in Q, the tetrapeptide residue is $^{C=O}$ -Gly-Phe-$(Gly)_2$- $^{NH}$ or $^{C=O}$ -$(Gly)_2$-Phe-Gly- $^{NH}$, for example, $^{C=O}$ -Gly-Phe-$(Gly)_2$- $^{NH}$.

**9.** The linker-drug conjugate of formula I or a pharmaceutically acceptable salt thereof according to claim 6 or 7, wherein the linker-drug conjugate satisfies one or more of the following conditions:

(1) the amino acid residue is independently -Phe-, -Lys-, -Val-, -Ala-, -Cit-, -Leu-, -Glu-, -Ile-, -Arg-, -Trp-, or -Gly-;
(2) Q is a tetrapeptide residue; preferably, the tetrapeptide residue is

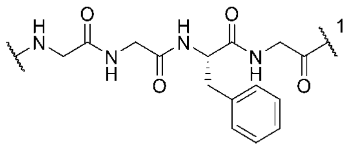

,

wherein the 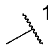 is linked to the -NH- group;
(3) X is $-(CR^aR^b)_r$-CO-NH-$(CR^aR^b)_q$-$(OCH_2CH_2)_p$-*, *-$(OCH_2CH_2)_m$-, $-(CR^aR^b)_m$-, *-O$(CR^aR^b)_m$-, *-NH-$(CR^aR^b)_m$-$CR^cR^d$- or *-S$(CR^aR^b)_m$-$CR^cR^d$-, wherein "*" terminal is linked to the carbonyl group; preferably, X is $-(CR^aR^b)_r$-CO-NH-$(CR^aR^b)_q$-$(OCH_2CH_2)_p$-* or $-(CR^aR^b)_m$-, for example, X is $-(CR^aR^b)_m$-, wherein "*" terminal is linked to the carbonyl group;
(4) m is an integer from 0 to 8, for example, 5, 6, 7, or 8;
(5) p is an integer from 0 to 10, for example, 5, 6, 7, or 8;
(6) q and r are independently integers from 0 to 8;
(4) Y is

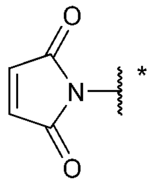

or

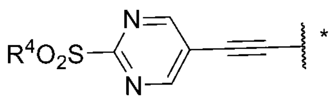

,

wherein "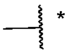"terminal is linked to X;
preferably, Y is

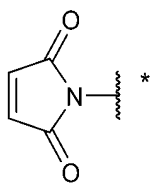

,

wherein "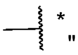" terminal is linked to X;
(6)

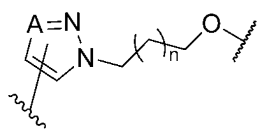

is

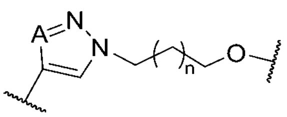

.

**10.** The linker-drug conjugate of formula I or a pharmaceutically acceptable salt thereof according to claim 6 or 7, wherein, the linker-drug conjugate satisfies one or more of the following conditions:

(1) $R^a$ and $R^b$ are H;
(2) m is 1, 2, 3, 4 or 5;
(3) p is 8, 9, 10, 11, 12, 13 or 14;
(4) q is 1, 2, 3, 4 or 5;
(5) r is 1, 2, 3, 4 or 5;
(6) Q is a dipeptide residue or a tetrapeptide residue; for example, $^{C=O}$ -Ala-Val- $^{NH}$ or $^{C=O}$ - Gly-Phe-$(Gly)_2$- $^{NH}$; and
(7) X is $-(CR^aR^b)_r$-CO-NH-$(CR^aR^b)_q$-$(OCH_2CH_2)_p$- or $-(CR^aR^b)_m$-; for example,

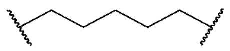
or
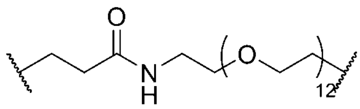
,

further for example,

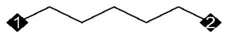

or

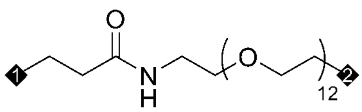
,

wherein the position "1" is linked to Y and the position "2" is linked to

"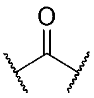".

**11.** The linker-drug conjugate of formula I or a pharmaceutically acceptable salt thereof according to claim 10, wherein the linker-drug conjugate satisfies one or more of the following conditions:

(1) $R^a$ and $R^b$ are independently H;
(2) m is 5;
(3) p is 8;
(4) q is 2;
(5) r is 2;
(6) n is 0 or 1;
(7) the single amino acid residue is -Phe-, -Lys-, -Val-, -Ala-, -Cit-, -Leu-, -Ile-, -Arg- or - Trp-;
(8) the dipeptide residue is $^{C=O}$-Lys-Phe-$^{NH}$, $^{C=O}$-Ala-Val-$^{NH}$, $^{C=O}$-Lys-Val-$^{NH}$, $^{C=O}$-Lys-Ala-$^{NH}$, $^{C=O}$-Cit-Val-$^{NH}$, $^{C=O}$-Cit-Phe-$^{NH}$, $^{C=O}$-Cit-Leu-$^{NH}$, $^{C=O}$-Cit-Ile-$^{NH}$, $^{C=O}$-Arg-Phe-$^{NH}$, $^{C=O}$-Cit-Trp-$^{NH}$ or $^{C=O}$-Val-Gly-$^{NH}$;
(9) the tripeptide residue is $^{C=O}$-Ala-Val-Glu-$^{NH}$, $^{C=O}$-Cit-Val-Glu-$^{NH}$, $^{C=O}$-Ala-Val-$\alpha$Glu-$^{NH}$ or $^{C=O}$-Cit-Val-$\alpha$Glu-$^{NH}$;
(10) the tetrapeptide residue is $^{C=O}$-Gly-Phe-$(Gly)_2$-$^{NH}$ or $^{C=O}$-$(Gly)_2$-Phe-Gly-$^{NH}$.

**12.** The linker-drug conjugate of formula I or a pharmaceutically acceptable salt thereof according to claim 6 or 7, wherein the linker-drug conjugate satisfies one or more of the following conditions:

(1)

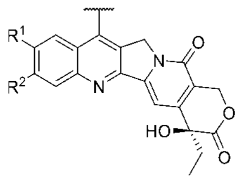

is

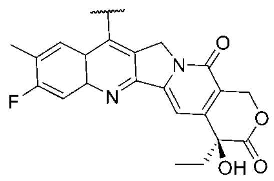

;

(2)

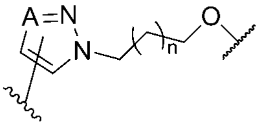

is

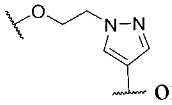

or

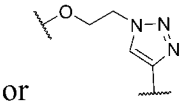

;

(3)

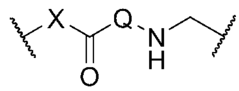

is

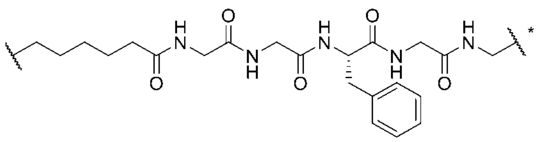

,

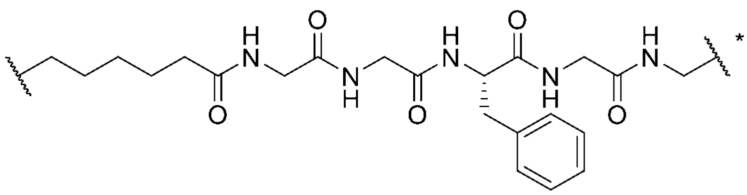

,

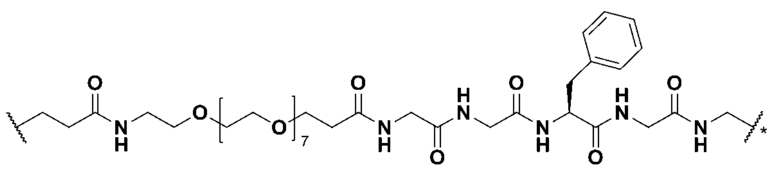

or

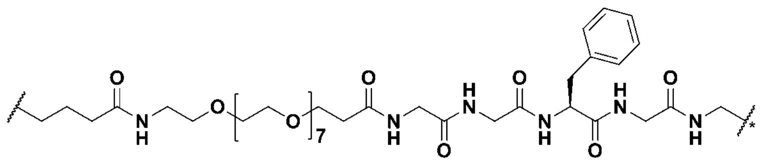,

wherein "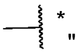" terminal is linked to -O-.

**13.** The linker-drug conjugate of formula I or a pharmaceutically acceptable salt thereof according to claim 6 or 7, wherein the linker-drug conjugate of formula I is any one of the following structures:

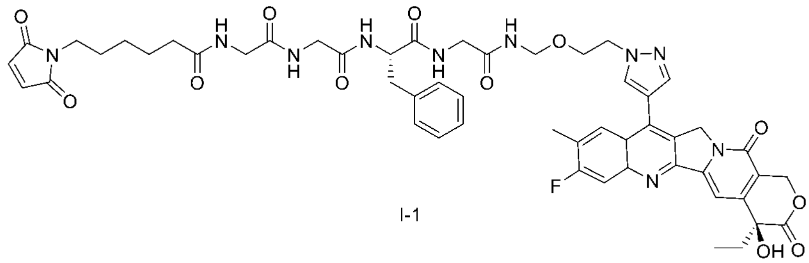

I-1

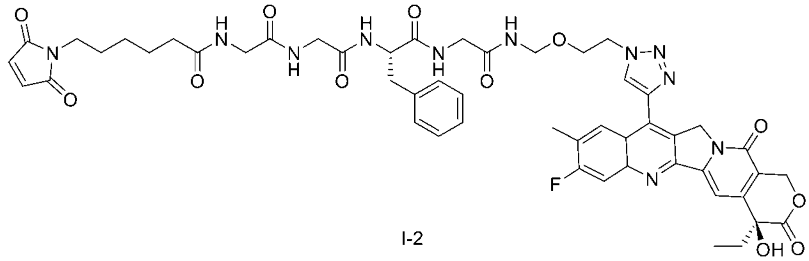

I-2

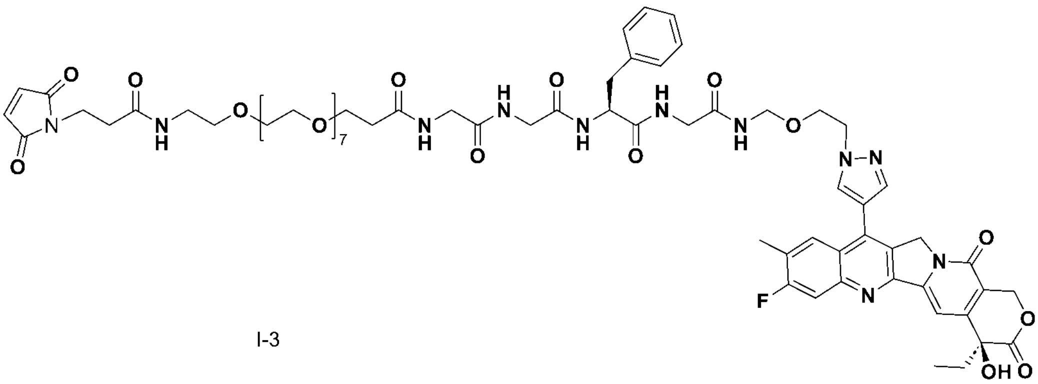

I-3

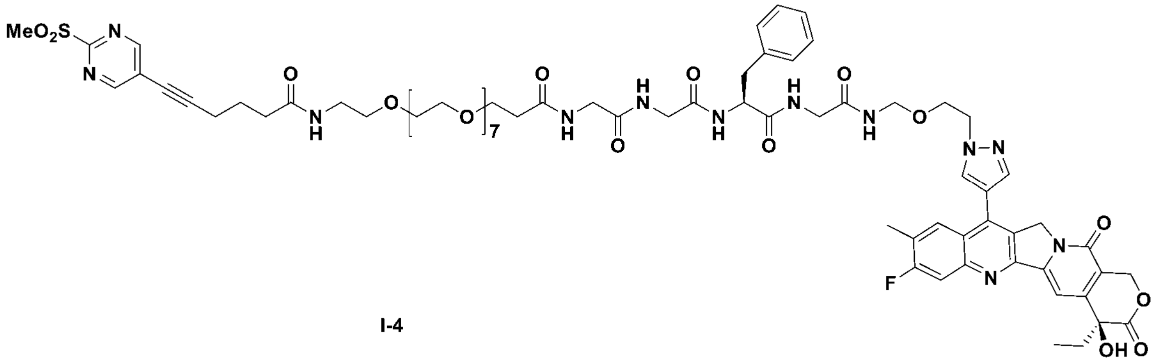

I-4 .

**14.** An antibody-drug conjugate of formula II or a pharmaceutically acceptable salt thereof:

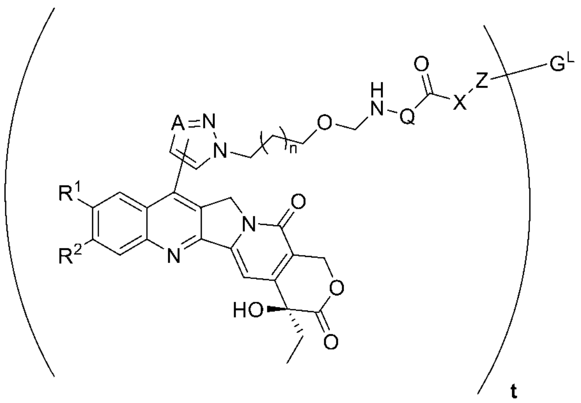

II

wherein, $G^L$ is an antibody;
t is a drug-to-antibody ratio;
Z is

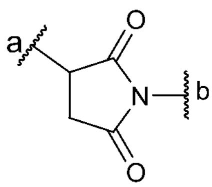 or 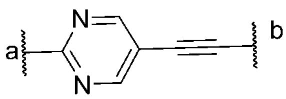 ,

wherein "b" terminal is linked to X and "a" terminal is linked to $G^L$;
the definitions of

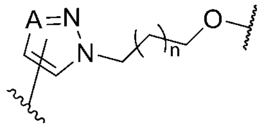 ,

$R^1$, $R^2$, A and n are as described in any one of claims 1-5;
the definitions of Q and X are as described in any one of claims 6-13.

**15.** The antibody-drug conjugate of formula II or a pharmaceutically acceptable salt thereof according to claim 14, wherein the antibody-drug conjugate is any one of the following schemes:

scheme 1:

$G^L$ is an antibody;
t is a drug-to-antibody ratio;
z is

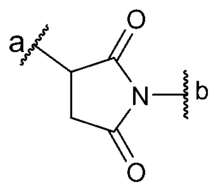

,

wherein the "b" terminal is linked to X and "a" terminal is linked to $G^L$.
the definitions of $R^1$, $R^2$, A and n are as described in any one of claims 1-5;
the definitions of Q and X are as described in any one of claims 6-13;

scheme 2:

$G^L$ is an antibody;
t is a natural number or a decimal number from 1 to 8;
z is

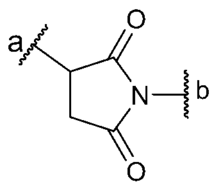

or

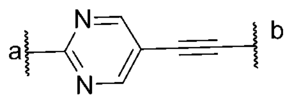

,

wherein the "b" terminal is linked to X and "a" terminal is linked to $G^L$;
the definitions of

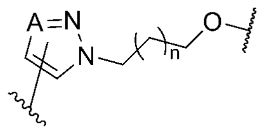

,

$R^1$, $R^2$, A and n are as described in any one of claims 1-5;
the definitions of Q and X are as described in any one of claims 6-13.

**16.** The antibody-drug conjugate of formula II or a pharmaceutically acceptable salt thereof according to claim 14 or 15, wherein the antibody-drug conjugate satisfies one or more of the following conditions:

(1) $G^L$ is an anti-HER2 antibody, for example, trastuzumab;
(2) t is a natural number and/or decimal from 1 to 8; for example, a natural number and/or decimal from 7 to 8; further for example, 7.8; or t is a natural number from 1 to 8; for example, a natural number from 7 to 8, preferably 7, 7.7 or 7.8;
(3) "a" terminal of Z is linked to the $G^L$ via a thioether bond.

**17.** The antibody-drug conjugate of formula II or a pharmaceutically acceptable salt thereof according to claim 14, wherein the antibody-drug conjugate of formula II is any one of the following structures:

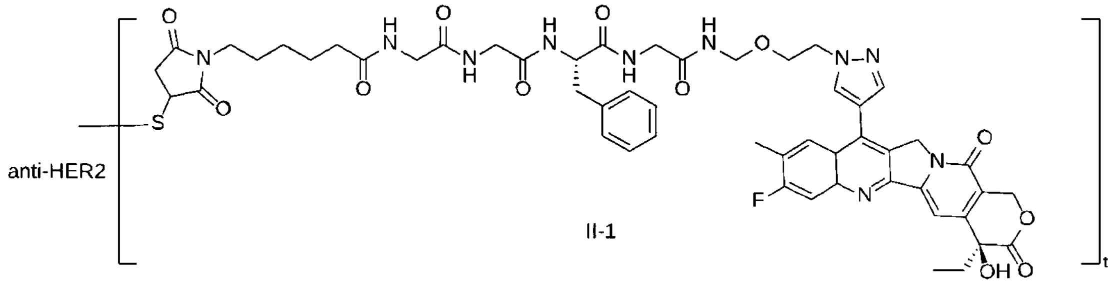

II-1

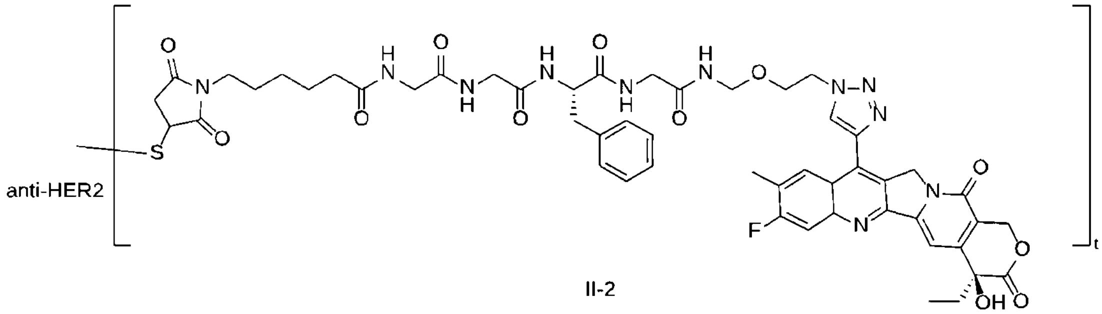

II-2

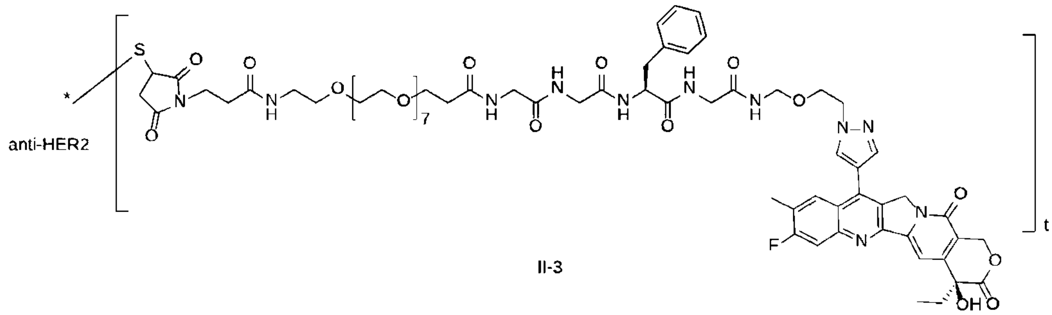

II-3

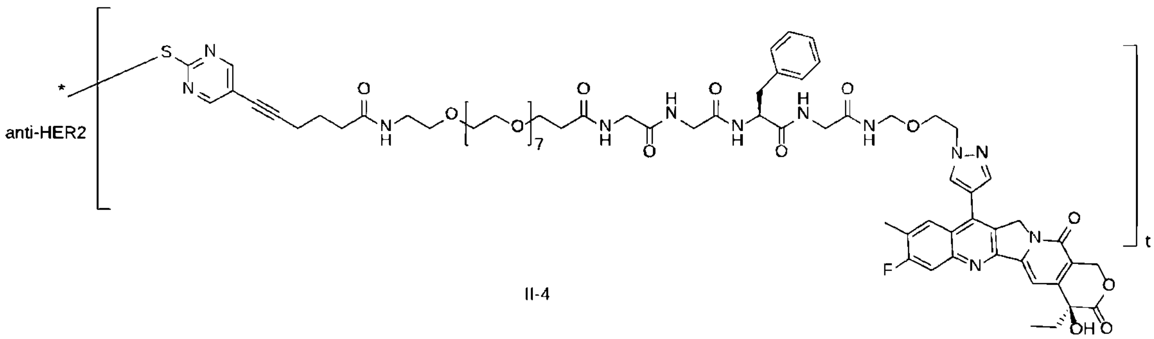

II-4

wherein the anti-HER2 antibody is trastuzumab; t is a drug-to-antibody ratio;
preferably, the antibody-drug conjugate of formula II is any one of the following structures:

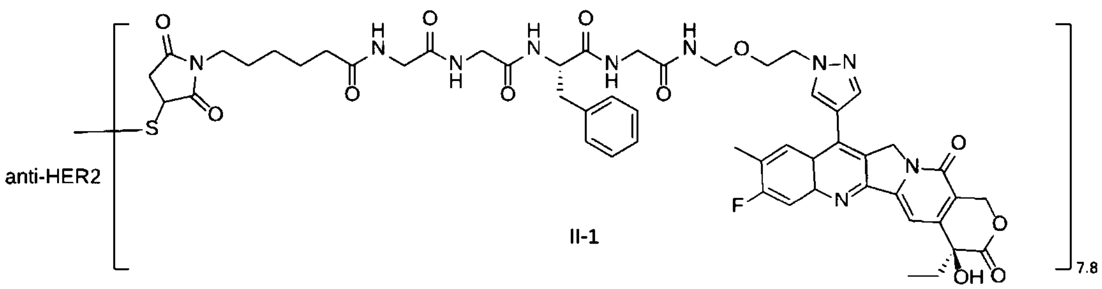

II-1

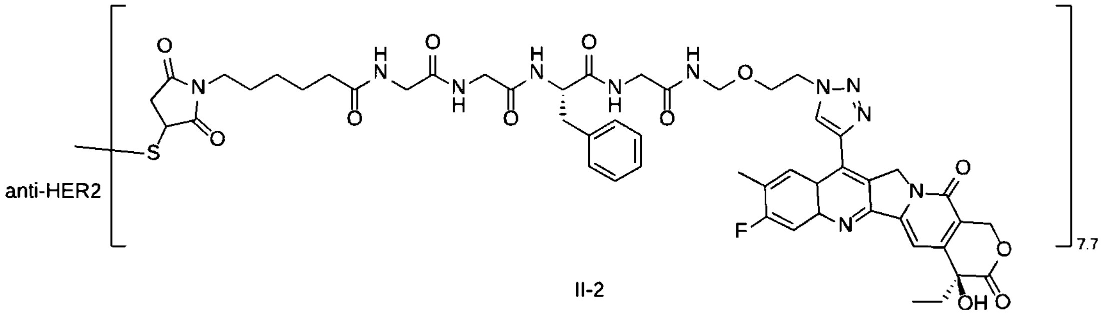

II-2

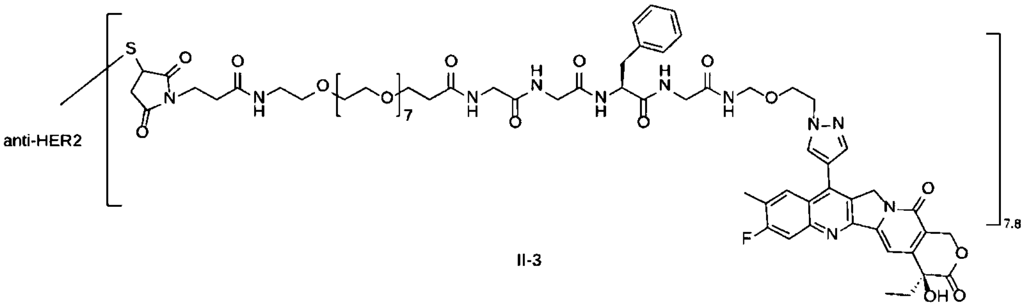

II-3

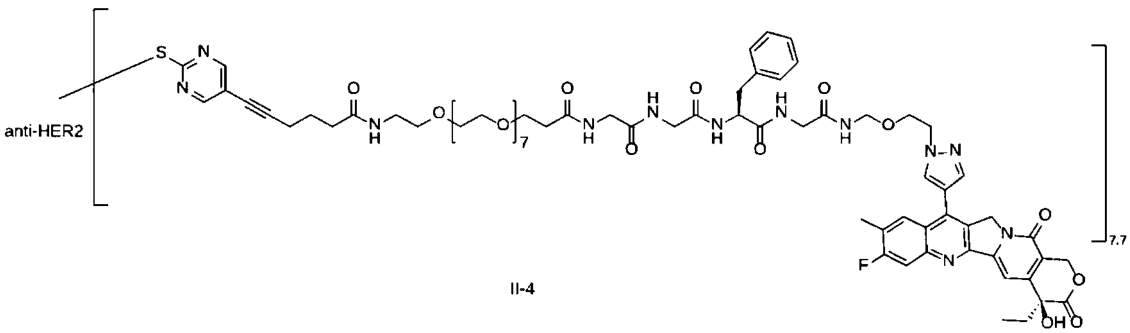

II-4

wherein the anti-HER2 antibody is trastuzumab.

**18.** A pharmaceutical composition comprising the camptothecin compound of formula K or a pharmaceutically acceptable salt thereof according to any one of claims 1-5, the linker-drug conjugate of formula I or a pharmaceutically acceptable salt thereof according to any one of claims 6-13, or the antibody-drug conjugate of formula II or a pharmaceutically acceptable salt thereof according to any one of claims 14-17.

**19.** Use of the camptothecin compound of formula K or a pharmaceutically acceptable salt thereof according to any one of claims 1-5, the linker-drug conjugate of formula I or a pharmaceutically acceptable salt thereof according to any one of claims 6-13, the antibody-drug conjugate of formula II or a pharmaceutically acceptable salt thereof according to any one of claims 14-17, or the pharmaceutical composition according to claim 18 in the manufacture of a medicament for preventing and/or treating cancer;
the cancer is preferably breast cancer, lung cancer, renal cancer, liver cancer, ovarian cancer, urethral cancer, prostate cancer, glioblastoma multiforme, pancreatic cancer, colorectal cancer, gastrointestinal stromal tumor, cervical cancer, squamous cell carcinoma, peritoneal cancer, colon cancer, rectal cancer, uterine cancer, salivary gland cancer, renal cancer, vulvar cancer, thyroid cancer, penile cancer, leukemia, malignant lymphoma, plasmacytoma, myeloma, sarcoma, melanoma, bladder cancer, gastric cancer, or esophageal cancer;preferably breast cancer, or preferably lung adenocarcinoma, gastric cancer, or colon cancer; for example, gastric cancer, lung cancer, colon cancer, or breast cancer.

**20.** A method for preparing the camptothecin compound of formula K according to any one of claims 1-5, the antibody-drug conjugate of formula II according to any one of claims 14-17, or a pharmaceutically acceptable salt thereof, wherein the method is any one of the following schemes:

scheme 1:

scheme 1 comprises the following steps: subjecting a linker-drug conjugate of formula I and an antibody to a conjugation reaction to obtain the antibody-drug conjugate of formula II or a pharmaceutically acceptable salt thereof;

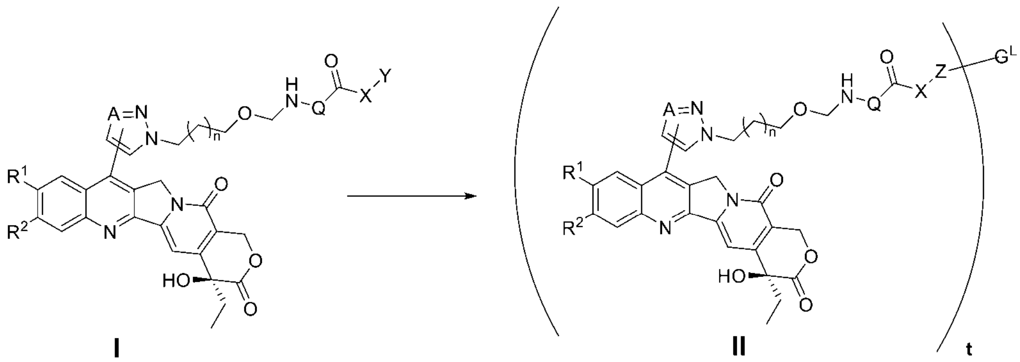

wherein, $R^1$, $R^2$, A, n,

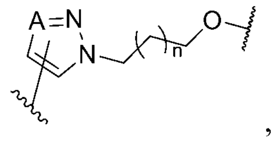

Q, X, $R^a$, $R^b$, Z, $G^L$, t, m, p, q, and r are independently as described in any one of claims 1-17:

scheme 2:

scheme 2 comprises the following steps: in haloalkane solvents, in the presence of a catalyst, subjecting a compound of formula Ka and a azide compound of formula Kb to a cyclization reaction to obtain the camptothecin compound represented by formula K or a pharmaceutically acceptable salt thereof;

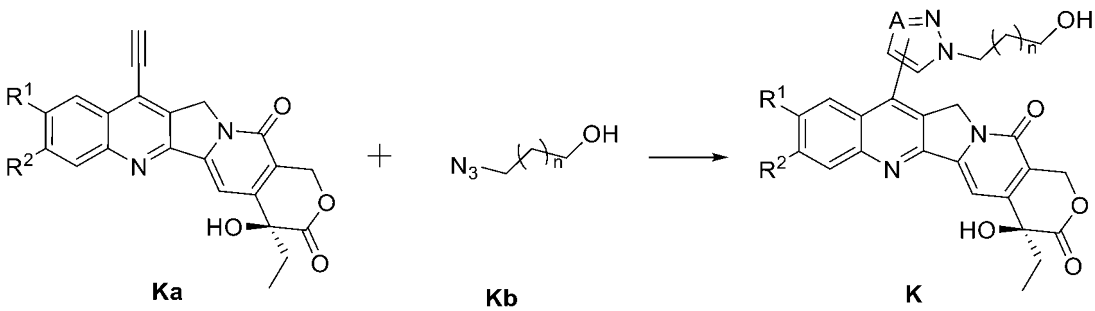

wherein, A is N;

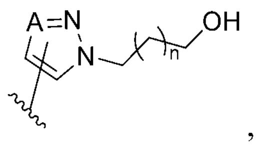

$R^1$, $R^2$, and n are as described in any one of claims 1-5;

scheme 3:

scheme 3 comprises the following steps: in aromatic hydrocarbon solvents, in the presence of an organic

acid, subjecting a compound of formula Kc and a compound of formula Kd to a cyclization reaction to obtain the camptothecin compound represented by formula K or a pharmaceutically acceptable salt thereof;

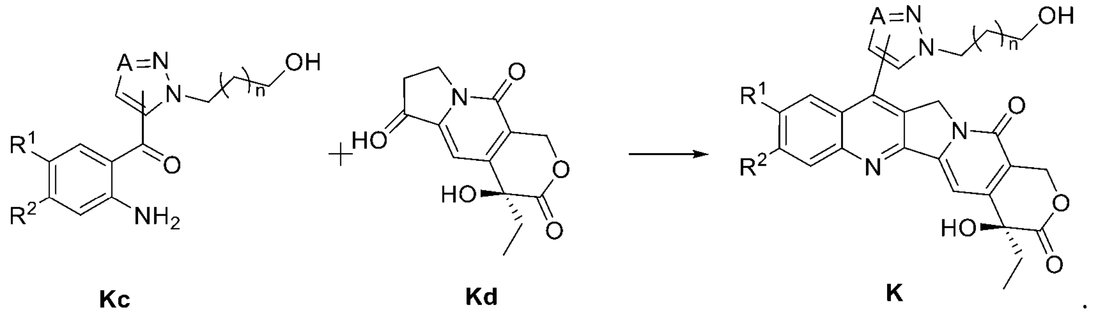

Kc Kd K

;

wherein, A is CH;

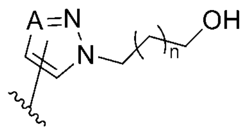

,

$R^1$, $R^2$, and n are as described in any one of claims 1-5.

**21.** A compound or a pharmaceutically acceptable salt thereof, wherein the compound is a compound of formula Ka or a compound of formula Kc;

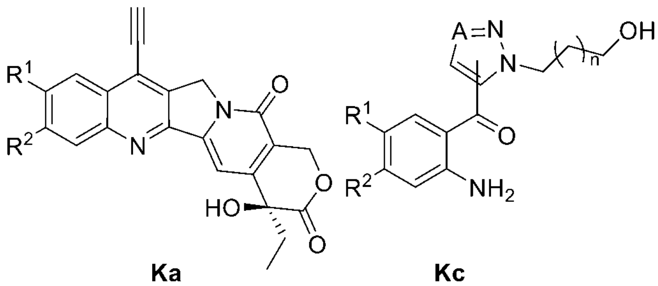

Ka Kc

wherein, A is CH;
$R^1$, n, and $R^2$ are as described in claim 20;
preferably, the compound of formula Ka is

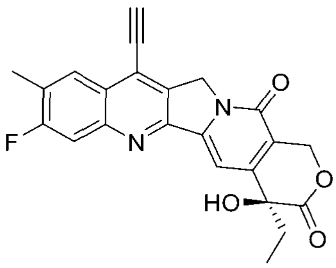

;

preferably, the compound of formula Kc is any one of the following compounds:

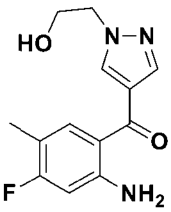, 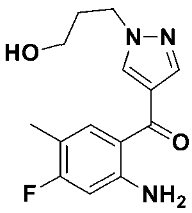, 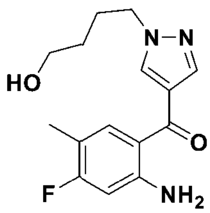, 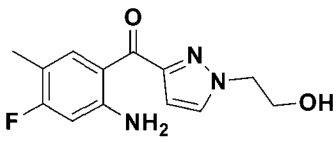,

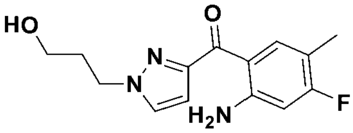 or 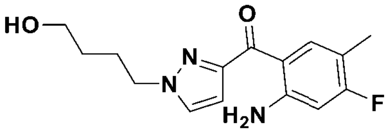.

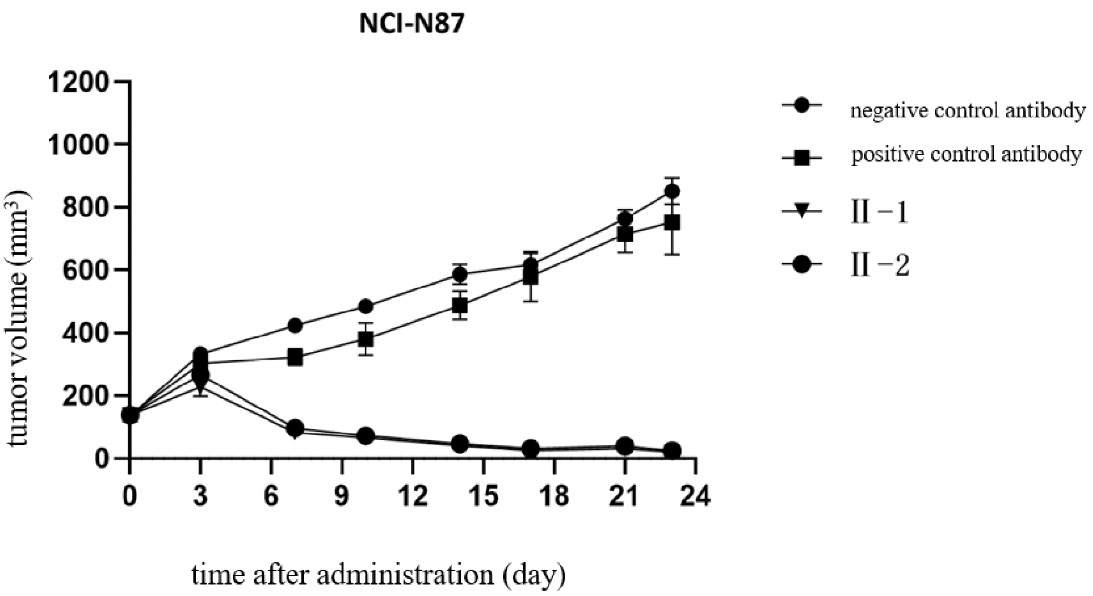


Figure 1

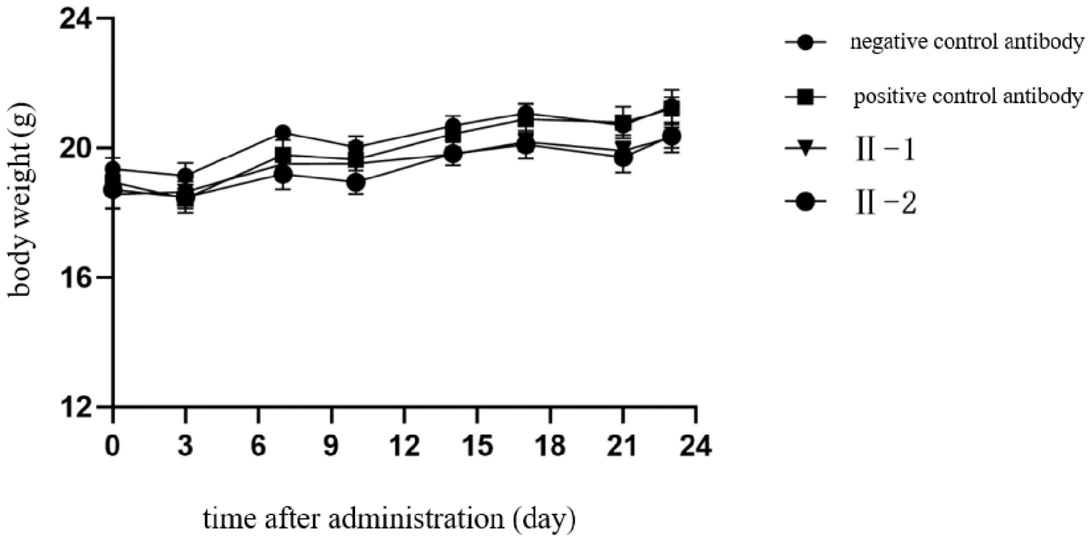


Figure 2

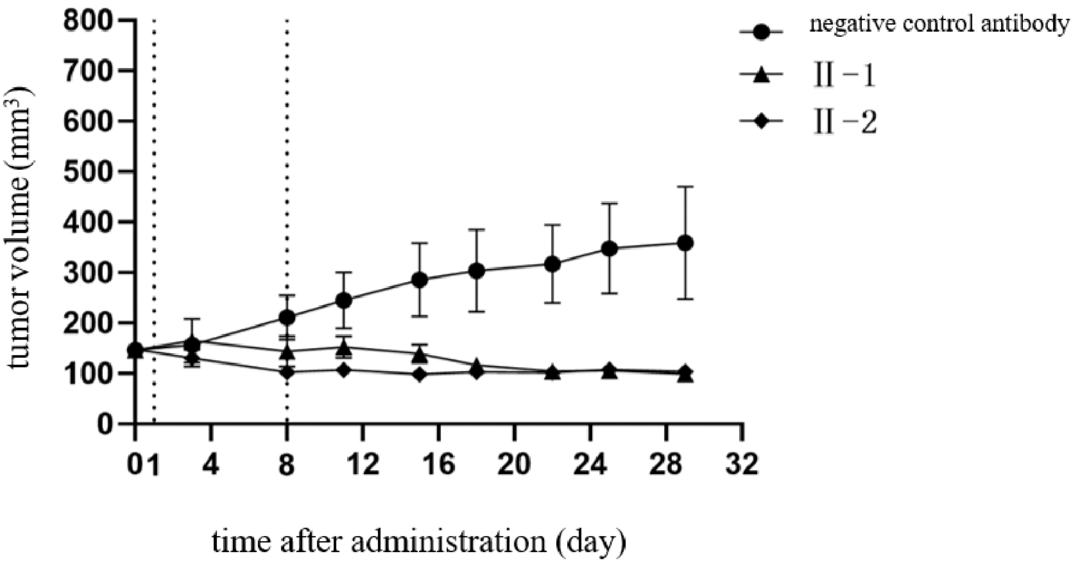


Figure 3

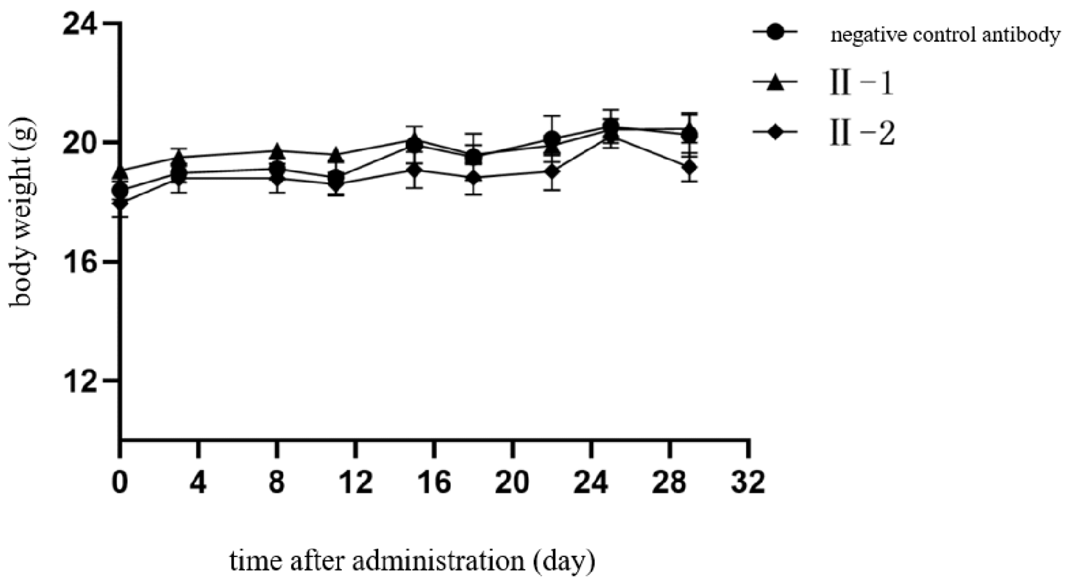


Figure 4

**INTERNATIONAL SEARCH REPORT**

International application No.
**PCT/CN2024/106693**

**A. CLASSIFICATION OF SUBJECT MATTER**

C07D491/22(2006.01)i； C07D498/22(2006.01)i； A61K31/4375(2006.01)i； A61P35/00(2006.01)i

According to International Patent Classification (IPC) or to both national classification and IPC

**B. FIELDS SEARCHED**

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

CNTXT, ENTXT, ENTXTC, DWPI, REGISTRY (STN), CAPLUS (STN), MARPAT (STN), CASREACT (STN), Web of science, 百度学术, BAIDU SCHOLAR, CNKI: 上海医药, 喜树碱, 偶联, 肿瘤, 癌症, camptothecin, CPT, conjug+, ADC, HER2, cancer, tumor, tumour, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| X | WO 2022204184 A1 (NUVATION BIO INC.) 29 September 2022 (2022-09-29)<br>abstract, claims 1-2, 6, and 16-20, and description, page 71 | 1-5, 18-21 |
| Y | WO 2022204184 A1 (NUVATION BIO INC.) 29 September 2022 (2022-09-29)<br>abstract, claims 1-2, 6, and 16-20, and description, page 71 | 6-21 |
| Y | WO 2022170971 A1 (MEDILINK THERAPEUTICS (SUZHOU) CO., LTD.) 18 August 2022 (2022-08-18)<br>abstract, and description, pages 4, 22, 24-27, 38, 41-42, 82-83, 85, 89, 116, and 307 | 6-20 |
| Y | WO 2019238046 A1 (QINGDAO MARINE BIOMEDICAL RESEARCH INSTITUTE CO., LTD.) 19 December 2019 (2019-12-19)<br>abstract, claims 1, 9, and 11-15, and description, pages 10, 14, 25, and 27 | 20, 21 |
| A | WO 2015148415 A2 (CANGET BIOTEKPHARMA, LLC) 01 October 2015 (2015-10-01)<br>abstract, and claims 20-22 and 25-28 | 1-21 |
| A | WO 2023030364 A1 (SHANGHAI BEST-LINK BIOSCIENCE, LLC) 09 March 2023 (2023-03-09)<br>abstract, and claims 1 and 12-16 | 1-21 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 October 2024** | **31 October 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/106693**

**C. DOCUMENTS CONSIDERED TO BE RELEVANT**

| Category* | Citation of document, with indication, where appropriate, of the relevant passages | Relevant to claim No. |
|---|---|---|
| A | WO 2022262516 A1 (BEIJING HOPE PHARMACEUTICAL CO. LTD.) 22 December 2022 (2022-12-22)<br>abstract, and description, pages 2, 59-61, and 71-86 | 1-21 |

Form PCT/ISA/210 (second sheet) (July 2022)

**INTERNATIONAL SEARCH REPORT**
**Information on patent family members**

International application No.
**PCT/CN2024/106693**

| Patent document cited in search report | Publication date (day/month/year) | Patent family member(s) | Publication date (day/month/year) |
|---|---|---|---|
| WO 2022204184 A1 | 29 September 2022 | EP 4313991 A1 | 07 February 2024 |
| | | CA 3214408 A1 | 29 September 2022 |
| | | JP 2024510792 A | 11 March 2024 |
| | | CL 2023002820 A1 | 12 April 2024 |
| | | IL 306010 A | 01 November 2023 |
| | | TW 202304928 A | 01 February 2023 |
| | | MX 2023011241 A | 03 October 2023 |
| | | AR 125209 A1 | 21 June 2023 |
| | | KR 20230160299 A | 23 November 2023 |
| | | CO 2023012534 A2 | 19 October 2023 |
| | | AU 2022246058 A1 | 05 October 2023 |
| | | BR 112023019420 A2 | 24 October 2023 |
| | | US 2022340587 A1 | 27 October 2022 |
| | | US 11834458 B2 | 05 December 2023 |
| WO 2022170971 A1 | 18 August 2022 | CA 3206117 A1 | 18 August 2022 |
| | | MX 2023008895 A | 09 August 2023 |
| | | AU 2022220512 A1 | 13 July 2023 |
| | | KR 20230145038 A | 17 October 2023 |
| | | JP 2024508081 A | 22 February 2024 |
| | | EP 4257154 A1 | 11 October 2023 |
| | | IL 304804 A | 01 September 2023 |
| | | US 2024108745 A1 | 04 April 2024 |
| | | TW 202241521 A | 01 November 2022 |
| WO 2019238046 A1 | 19 December 2019 | US 2022177485 A1 | 09 June 2022 |
| | | EP 3808751 A1 | 21 April 2021 |
| | | EP 3808751 A4 | 23 March 2022 |
| | | JP 2021526560 A | 07 October 2021 |
| | | JP 7485661 B2 | 16 May 2024 |
| WO 2015148415 A2 | 01 October 2015 | US 2018170944 A1 | 21 June 2018 |
| | | US 10344037 B2 | 09 July 2019 |
| | | EP 3122754 A2 | 01 February 2017 |
| | | EP 3122754 A4 | 21 February 2018 |
| | | WO 2015148415 A3 | 10 December 2015 |
| | | US 2019367529 A1 | 05 December 2019 |
| | | US 11084828 B2 | 10 August 2021 |
| WO 2023030364 A1 | 09 March 2023 | None | |
| WO 2022262516 A1 | 22 December 2022 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023109020423
- CN 2023116319073
- WO 2022063278 A


### Non-patent literature cited in the description


- McGraw-Hill Dictionary of Chemical Terms. McGraw-Hill Book Company, 1984
- **ELIEL, E.** ; **WILEN, S.** Stereochemistry of Organic Compounds. John Wiley & Sons, Inc., 1994
- Pharmacopoeia of the People's Republic of China. 2020
- **RAYMOND C ROWE**. Handbook of Pharmaceutical Excipients. 2009
- *Synlett*, 2014, vol. 25 (19), 2802-2805
- *CHEMICAL ABSTRACTS*, 1599440-06-8
- *CHEMICAL ABSTRACTS*, 180288-69-1
- *CHEMICAL ABSTRACTS*, 236426-37-2
- *CHEMICAL ABSTRACTS*, 756525-93-6
- *CHEMICAL ABSTRACTS*, 1334170-03-4
- *CHEMICAL ABSTRACTS*, 2356229-58-6